# EUROPEAN PATENT SPECIFICATION

(11) **EP 1 919 951 B1**
(45) Date of publication and mention of the grant of the patent: **28.01.2015**
(21) Application number: 06774701.4
(22) Date of filing: 27.07.2006
(51) Int. Cl.: C07K 16/00, C12P 21/08, C12Q 1/68, C07K 16/24, C07K 16/46

(54) **ANTI-TNF-ALPHA ANTIBODIES AND METHODS OF USE**
ANTI-TNF-ALPHA-ANTIKÖRPER UND ANWENDUNGSVERFAHREN
ANTICORPS ANTI-TNF-ALPHA ET PROCEDES D'UTILISATION

(30) Priority: 04.08.2005 US 705427 P
(43) Date of publication of application: 14.05.2008
(73) Proprietor: Janssen Biotech, Inc., Horsham, PA 19044 (US)
(72) Inventor: EVANS, Glen A., San Marcos, CA 92069 (US); MCLANE, Katya, Vista, CA 92083 (US); RAGHUNATHAN, Gopalan, San Diego, CA 92129 (US)
(74) Representative: Marshall, Cameron John
(86) International application number: PCT/US2006/029114
(87) International publication number: WO 2007/019064

(56) References cited:
- WO-A2-03/042247
- US-A1- 2004 131 613

### SEQUENCE LISTING

<110> Centocor, Inc.
<120> Anti-TNFα Antibodies and Methods of Use
<130> CEN5105 PCT
<140> To Be Assigned
   <141> 2006-07-27
<150> 60/705,427
   <151> 2005-08-04
<160> 383
<170> FastSEQ for Windows Version 4.0
<210> 1
   <211> 119
   <212> PRT
   <213> Mus musculus
<400> 1
   Glu Val Lys Leu Glu Glu Ser Gly Gly Gly Leu Val Gln Pro Gly Gly
    1               5                  10                  15
   Ser Met Lys Leu Ser Cys Val Ala Ser Gly Phe Ile Phe Ser Asn His
               20                  25                  30
   Trp Met Asn Trp Val Arg Gln Ser Pro Glu Lys Gly Leu Glu Trp Val
           35                  40                  45
   Ala Glu Ile Arg Ser Lys Ser Ile Asn Ser Ala Thr His Tyr Ala Glu
       50                  55                  60
   Ser Val Lys Gly Arg Phe Thr Ile Ser Arg Asp Asp Ser Lys Ser Ala
   65                  70                  75                  80
   Val Tyr Leu Gln Met Asn Ser Leu Arg Thr Glu Asp Thr Gly Val Tyr
                   85                  90                  95
   Tyr Cys Ser Arg Asn Tyr Tyr Gly Ser Thr Tyr Asp Tyr Trp Gly Gln
               100                 105                 110
   Gly Thr Thr Leu Thr Val Ser
           115
<210> 2
   <211> 119
   <212> PRT
   <213> Artificial Sequence
<220>
   <221> unknown
   <222> (5)
   <223> Where Xaa can be V or T
<220>
   <221> unknown
   <222> (23)
   <223> Where Xaa can be A or R
<220>
   <221> unknown
   <222> (26)
   <223> Where Xaa can be G or Q
<220>
   <221> unknown
   <222> (27)
   <223> Where Xaa can be F or S
<220>
   <221> unknown
   <222> (28)
   <223> Where Xaa can be I or T
<220>
   <221> unknown
   <222> (33)
   <223> Where Xaa can be W or Y
<220>
   <221> unknown
   <222> (56)
   <223> Where Xaa can be I or S
<220>
   <221> unknown
   <222> (57)
   <223> Where Xaa can be N or Y
<220>
   <221> unknown
   <222> (58)
   <223> Where Xaa can be S or G
<220>
   <221> unknown
   <222> (61)
   <223> Where Xaa can be H or S
<220>
   <221> unknown
   <222> (76)
   <223> Where Xaa can be D or G
<220>
   <221> unknown
   <222> (78)
   <223> Where Xaa can be K or G
<220>
   <221> unknown
   <222> (79)
   <223> Where Xaa can be N or T
<220>
   <221> unknown
   <222> (80)
   <223> Where Xaa can be S or D
<220>
   <221> unknown
   <222> (82)
   <223> Where Xaa can be Y or T
<220>
   <221> unknown
   <222> (100)
   <223> Where Xaa can be R or Q
<220>
   <221> unknown
   <222> (107)
   <223> Where Xaa can be Y or P
<400> 2
   Glu Val Gln Leu Xaa Glu Ser Gly Gly Gly Leu Val Gln Pro Gly Gly
    1               5                  10                  15
   Ser Leu Arg Leu Ser Cys Xaa Ala Ser Xaa Xaa Xaa Phe Ser Asn His
               20                  25                  30
   Xaa Met Asn Trp Val Arg Gln Ala Pro Gly Lys Gly Leu Glu Trp Ile
           35                  40                  45
   Gly Glu Ile Arg Ser Lys Ser Xaa Xaa Xaa Ala Thr Xaa Tyr Ala Glu
       50                  55                  60
   Ser Val Lys Gly Arg Phe Ile Ile Ser Arg Asp Xaa Asn Xaa Xaa Xaa
   65                  70                  75                  80
   Leu Xaa Leu Glu Met Asn Ser Leu Lys Thr Glu Asp Thr Ala Glu Tyr
                   85                  90                  95
   Tyr Cys Ala Xaa Asn Tyr Tyr Gly Ser Thr Xaa Asp Tyr Trp Gly Gln
               100                 105                 110
   Gly Thr Leu Val Thr Val Ser
           115
<210> 3
   <211> 119
   <212> PRT
   <213> Artificial Sequence
<220>
   <223> Infliximab VH variant
<400> 3
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr His Tyr Ala Glu
       50                  55                  60
   Ser Val Lys Gly Arg Phe Ile Ile Ser Arg Asp Asp Asn Lys Asn Ser
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 4
   <211> 119
   <212> PRT
   <213> Artificial Sequence
<220>
   <223> Infliximab VH variant
<400> 4
   Glu Val Gln Leu Thr Glu Ser Gly Gly Gly Leu Val Gln Pro Gly Gly
   1               5                   10                  15
   Ser Leu Arg Leu Ser Cys Arg Ala Ser Gly Phe Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Tyr Ser Ala Thr Ser Tyr Ala Glu
       50                  55                  60
   Ser Val Lys Gly Arg Phe Ile Ile Ser Arg Asp Asp Asn Lys Thr Ser
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 5
   <211> 119
   <212> PRT
   <213> Artificial Sequence
<220>
   <223> Infliximab VH variant
<400> 5
   Glu Val Gln Leu Thr Glu Ser Gly Gly Gly Leu Val Gln Pro Gly Gly
   1               5                   10                  15
   Ser Leu Arg Leu Ser Cys Ala Ala Ser Gln Phe Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr His Tyr Ala Glu
       50                  55                  60
   Ser Val Lys Gly Arg Phe Ile Ile Ser Arg Asp Asp Asn Lys Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 6
   <211> 119
   <212> PRT
   <213> Artificial Sequence
<220>
   <223> Infliximab VH variant
<400> 6
   Glu Val Gln Leu Val Glu Ser Gly Gly Gly Leu Val Gln Pro Gly Gly
    1               5                   10                  15
   Ser Leu Arg Leu Ser Cys Ala Ala Ser Gln Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Ser Ala Thr His Tyr Ala Glu
       50                  55                  60
   Ser Val Lys Gly Arg Phe Ile Ile Ser Arg Asp Gly Asn Lys Thr Asp
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 7
   <211> 119
   <212> PRT
   <213> Artificial Sequence
<220>
   <223> Infliximab VH variant
<400> 7
   Glu Val Gln Leu Thr Glu Ser Gly Gly Gly Leu Val Gln Pro Gly Gly
    1               5                   10                  15
   Ser Leu Arg Leu Ser Cys Ala Ala Ser Gln Phe Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Asn Ser Ala Thr His Tyr Ala Glu
       50                  55                  60
   Ser Val Lys Gly Arg Phe Ile Ile Ser Arg Asp Asp Asn Lys Thr Asp
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 8
   <211> 119
   <212> PRT
   <213> Artificial Sequence
<220>
   <223> Infliximab VH variant
<400> 8
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gly Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr His Tyr Ala Glu
       50                  55                  60
   Ser Val Lys Gly Arg Phe Ile Ile Ser Arg Asp Gly Asn Gly Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
               115
<210> 9
   <211> 119
   <212> PRT
   <213> Artificial Sequence
<220>
   <223> Infliximab VH variant
<400> 9
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Ser Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr Ser Tyr Ala Glu
       50                  55                  60
   Ser Val Lys Gly Arg Phe Ile Ile Ser Arg Asp Asp Asn Gly Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 10
   <211> 119
   <212> PRT
   <213> Artificial Sequence
<220>
   <223> Infliximab VH variant
<400> 10
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gly Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr Ser Tyr Ala Glu
       50                  55                  60
   Ser Val Lys Gly Arg Phe Ile Ile Ser Arg Asp Asp Asn Lys Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 11
   <211> 119
   <212> PRT
   <213> Artificial Sequence
<220>
   <223> Infliximab VH variant
<400> 11
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gly Phe Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr Ser Tyr Ala Glu
       50                  55                  60
   Ser Val Lys Gly Arg Phe Ile Ile Ser Arg Asp Asp Asn Lys Thr Ser
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 12
   <211> 119
   <212> PRT
   <213> Artificial Sequence
<220>
   <223> Infliximab VH variant
<400> 12
   Glu Val Gln Leu Thr Glu Ser Gly Gly Gly Leu Val Gln Pro Gly Gly
   1 5 10 15
   Ser Leu Arg Leu Ser Cys Arg Ala Ser Gln Ser Ile Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ser Asn Ser Ala Thr Ser Tyr Ala Glu
   50 55 60
   Ser Val Lys Gly Arg Phe Ile Ile Ser Arg Asp Gly Asn Lys Asn Asp
   65 70 75 80
   Leu Thr Leu Glu Met Asn Ser Leu Lys Thr Glu Asp Thr Ala Glu Tyr
   85 90 95
   Tyr Cys Ala Gln Asn Tyr Tyr Gly Ser Thr Pro Asp Tyr Trp Gly Gln
   100 105 110
   Gly Thr Leu Val Thr Val Ser
   115
<210> 13
   <211> 119
   <212> PRT
   <213> Artificial Sequence
<220>
   <223> Infliximab VH variant
<400> 13
   Glu Val Gln Leu Thr Glu Ser Gly Gly Gly Leu Val Gln Pro Gly Gly
   1 5 10 15
   Ser Leu Arg Leu Ser Cys Ala Ala Ser Gly Ser Ile Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Asn Ser Ala Thr His Tyr Ala Glu
   50 55 60
   Ser Val Lys Gly Arg Phe Ile Ile Ser Arg Asp Asp Asn Lys Thr Asp
   65 70 75 80
   Leu Tyr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 14
   <211> 119
   <212> PRT
   <213> Artificial Sequence
<220>
   <223> Infliximab VH variant
<400> 14
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gln Phe Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Tyr Gly Ala Thr His Tyr Ala Glu
       50                  55                  60
   Ser Val Lys Gly Arg Phe Ile Ile Ser Arg Asp Gly Asn Gly Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 15
   <211> 119
   <212> PRT
   <213> Artificial Sequence
<220>
   <223> Infliximab VH variant
<400> 15
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Ser Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Tyr Ser Ala Thr His Tyr Ala Glu
       50                  55                  60
   Ser Val Lys Gly Arg Phe Ile Ile Ser Arg Asp Asp Asn Lys Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 16
   <211> 119
   <212> PRT
   <213> Artificial Sequence
<220>
   <223> Infliximab VH variant
<400> 16
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gln Ser Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Asn Ser Ala Thr His Tyr Ala Glu
       50                  55                  60
   Ser Val Lys Gly Arg Phe Ile Ile Ser Arg Asp Gly Asn Gly Asn Asp
   65                  70                  75                      80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 17
   <211> 119
   <212> PRT
   <213> Artificial Sequence
<220>
   <223> Infliximab VH variant
<400> 17
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gly Phe Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Tyr Ser Ala Thr His Tyr Ala Glu
       50                  55                  60
   Ser Val Lys Gly Arg Phe Ile Ile Ser Arg Asp Asp Asn Lys Thr Ser
   65                  70                  75                      80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 18
   <211> 119
   <212> PRT
   <213> Artificial Sequence
<220>
   <223> Infliximab VH variant
<400> 18
   Glu Val Gln Leu Val Glu Ser Gly Gly Gly Leu Val Gln Pro Gly Gly
   1 5 10 15
   Ser Leu Arg Leu Ser Cys Arg Ala Ser Gln Phe Thr Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Asn Gly Ala Thr Ser Tyr Ala Glu
   50 55 60
   Ser Val Lys Gly Arg Phe Ile Ile Ser Arg Asp Gly Asn Gly Asn Ser
   65 70 75 80
   Leu Thr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 19
   <211> 119
   <212> PRT
   <213> Artificial Sequence
<220>
   <223> Infliximab VH variant
<400> 19
   Glu Val Gln Leu Thr Glu Ser Gly Gly Gly Leu Val Gln Pro Gly Gly
   1 5 10 15
   Ser Leu Arg Leu Ser Cys Arg Ala Ser Gln Phe Thr Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Asn Ser Ala Thr Ser Tyr Ala Glu
   50 55 60
   Ser Val Lys Gly Arg Phe Ile Ile Ser Arg Asp Asp Asn Lys Thr Ser
   65 70 75 80
   Leu Thr Leu Glu Met Asn Ser Leu Lys Thr Glu Asp Thr Ala Glu Tyr
   85 90 95
   Tyr Cys Ala Gln Asn Tyr Tyr Gly Ser Thr Pro Asp Tyr Trp Gly Gln
   100 105 110
   Gly Thr Leu Val Thr Val Ser
   115
<210> 20
   <211> 119
   <212> PRT
   <213> Artificial Sequence
<220>
   <223> Infliximab VH variant
<400> 20
   Glu Val Gln Leu Val Glu Ser Gly Gly Gly Leu Val Gln Pro Gly Gly
   1 5 10 15
   Ser Leu Arg Leu Ser Cys Arg Ala Ser Gly Phe Thr Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Asn Ser Ala Thr Ser Tyr Ala Glu
   50 55 60
   Ser Val Lys Gly Arg Phe Ile Ile Ser Arg Asp Asp Asn Gly Asn Asp
   65 70 75 80
   Leu Thr Leu Glu Met Asn Ser Leu Lys Thr Glu Asp Thr Ala Glu Tyr
   85 90 95
   Tyr Cys Ala Gln Asn Tyr Tyr Gly Ser Thr Pro Asp Tyr Trp Gly Gln
   100 105 110
   Gly Thr Leu Val Thr Val Ser
   115
<210> 21
   <211> 119
   <212> PRT
   <213> Artificial Sequence
<220>
   <223> Infliximab VH variant
<400> 21
   Glu Val Gln Leu Thr Glu Ser Gly Gly Gly Leu Val Gln Pro Gly Gly
   1 5 10 15
   Ser Leu Arg Leu Ser Cys Ala Ala Ser Gln Phe Thr Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Tyr Gly Ala Thr His Tyr Ala Glu
   50 55 60
   Ser Val Lys Gly Arg Phe Ile Ile Ser Arg Asp Asp Asn Gly Asn Asp
   65 70 75 80
   Leu Tyr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 22
   <211> 119
   <212> PRT
   <213> Artificial Sequence
<220>
   <223> Infliximab VH variant
<400> 22
   Glu Val Gln Leu Val Glu Ser Gly Gly Gly Leu Val Gln Pro Gly Gly
   1 5 10 15
   Ser Leu Arg Leu Ser Cys Arg Ala Ser Gly Ser Thr Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ser Asn Ser Ala Thr His Tyr Ala Glu
   50 55 60
   Ser Val Lys Gly Arg Phe Ile Ile Ser Arg Asp Asp Asn Gly Asn Ser
   65 70 75 80
   Leu Thr Leu Glu Met Asn Ser Leu Lys Thr Glu Asp Thr Ala Glu Tyr
   85 90 95
   Tyr Cys Ala Gln Asn Tyr Tyr Gly Ser Thr Tyr Asp Tyr Trp Gly Gln
   100 105 110
   Gly Thr Leu Val Thr Val Ser
   115
<210> 23
   <211> 119
   <212> PRT
   <213> Artificial Sequence
<220>
   <223> Infliximab VH variant
<400> 23
   Glu Val Gln Leu Thr Glu Ser Gly Gly Gly Leu Val Gln Pro Gly Gly
   1 5 10 15
   Ser Leu Arg Leu Ser Cys Ala Ala Ser Gly Ser Ile Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Tyr Gly Ala Thr His Tyr Ala Glu
   50 55 60
   Ser Val Lys Gly Arg Phe Ile Ile Ser Arg Asp Asp Asn Gly Thr Asp
   65 70 75 80
   Leu Tyr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 24
   <211> 119
   <212> PRT
   <213> Artificial Sequence
<220>
   <223> Infliximab VH variant
<400> 24
   Glu Val Gln Leu Thr Glu Ser Gly Gly Gly Leu Val Gln Pro Gly Gly
    1               5                   10                  15
   Ser Leu Arg Leu Ser Cys Ala Ala Ser Gly Ser Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr Ser Tyr Ala Glu
       50                  55                  60
   Ser Val Lys Gly Arg Phe Ile Ile Ser Arg Asp Asp Asn Gly Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 25
   <211> 119
   <212> PRT
   <213> Artificial Sequence
<220>
   <223> Infliximab VH variant
<400> 25
   Glu Val Gln Leu Val Glu Ser Gly Gly Gly Leu Val Gln Pro Gly Gly
    1               5                   10                  15
   Ser Leu Arg Leu Ser Cys Arg Ala Ser Gln Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr His Tyr Ala Glu
       50                  55                  60
   Ser Val Lys Gly Arg Phe Ile Ile Ser Arg Asp Gly Asn Lys Thr Ser
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 26
   <211> 119
   <212> PRT
   <213> Artificial Sequence
<220>
   <223> Infliximab VH variant
<400> 26
   Glu Val Gln Leu Val Glu Ser Gly Gly Gly Leu Val Gln Pro Gly Gly
    1               5                  10                  15
   Ser Leu Arg Leu Ser Cys Arg Ala Ser Gln Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr His Tyr Ala Glu
       50                  55                  60
   Ser Val Lys Gly Arg Phe Ile Ile Ser Arg Asp Asp Asn Lys Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 27
   <211> 119
   <212> PRT
   <213> Artificial Sequence
<220>
   <223> Infliximab VH variant
<400> 27
   Glu Val Gln Leu Val Glu Ser Gly Gly Gly Leu Val Gln Pro Gly Gly
    1               5                  10                  15
   Ser Leu Arg Leu Ser Cys Ala Ala Ser Gln Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Asn Gly Ala Thr His Tyr Ala Glu
       50                  55                  60
   Ser Val Lys Gly Arg Phe Ile Ile Ser Arg Asp Asp Asn Lys Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 28
   <211> 119
   <212> PRT
   <213> Artificial Sequence
<220>
   <223> Infliximab VH variant
<400> 28
   Glu Val Gln Leu Val Glu Ser Gly Gly Gly Leu Val Gln Pro Gly Gly
   1 5 10 15
   Ser Leu Arg Leu Ser Cys Ala Ala Ser Gly Ser Thr Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ser Tyr Gly Ala Thr Ser Tyr Ala Glu
   50 55 60
   Ser Val Lys Gly Arg Phe Ile Ile Ser Arg Asp Gly Asn Gly Asn Ser
   65 70 75 80
   Leu Thr Leu Glu Met Asn Ser Leu Lys Thr Glu Asp Thr Ala Glu Tyr
   85 90 95
   Tyr Cys Ala Gln Asn Tyr Tyr Gly Ser Thr Pro Asp Tyr Trp Gly Gln
   100 105 110
   Gly Thr Leu Val Thr Val Ser
   115
<210> 29
   <211> 119
   <212> PRT
   <213> Artificial Sequence
<220>
   <223> Infliximab VH variant
<400> 29
   Glu Val Gln Leu Thr Glu Ser Gly Gly Gly Leu Val Gln Pro Gly Gly
   1 5 10 15
   Ser Leu Arg Leu Ser Cys Arg Ala Ser Gln Ser Ile Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Asn Ser Ala Thr Ser Tyr Ala Glu
   50 55 60
   Ser Val Lys Gly Arg Phe Ile Ile Ser Arg Asp Asp Asn Lys Thr Ser
   65 70 75 80
   Leu Tyr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 30
   <211> 119
   <212> PRT
   <213> Artificial Sequence
<220>
   <223> Infliximab VH variant
<400> 30
   Glu Val Gln Leu Val Glu Ser Gly Gly Gly Leu Val Gln Pro Gly Gly
    1               5                  10                  15
   Ser Leu Arg Leu Ser Cys Arg Ala Ser Gln Ser Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr Ser Tyr Ala Glu
       50                  55                  60
   Ser Val Lys Gly Arg Phe Ile Ile Ser Arg Asp Gly Asn Lys Thr Ser
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 31
   <211> 119
   <212> PRT
   <213> Artificial Sequence
<220>
   <223> Infliximab VH variant
<400> 31
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gln Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Gly Ala Thr His Tyr Ala Glu
       50                  55                  60
   Ser Val Lys Gly Arg Phe Ile Ile Ser Arg Asp Gly Asn Lys Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 32
   <211> 119
   <212> PRT
   <213> Artificial Sequence
<220>
   <223> Infliximab VH variant
<400> 32
   Glu Val Gln Leu Thr Glu Ser Gly Gly Gly Leu Val Gln Pro Gly Gly
   1 5 10 15
   Ser Leu Arg Leu Ser Cys Arg Ala Ser Gln Phe Thr Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Asn Gly Ala Thr Ser Tyr Ala Glu
   50 55 60
   Ser Val Lys Gly Arg Phe Ile Ile Ser Arg Asp Gly Asn Gly Asn Ser
   65 70 75 80
   Leu Thr Leu Glu Met Asn Ser Leu Lys Thr Glu Asp Thr Ala Glu Tyr
   85 90 95
   Tyr Cys Ala Gln Asn Tyr Tyr Gly Ser Thr Tyr Asp Tyr Trp Gly Gln
   100 105 110
   Gly Thr Leu Val Thr Val Ser
   115
<210> 33
   <211> 119
   <212> PRT
   <213> Artificial Sequence
<220>
   <223> Infliximab VH variant
<400> 33
   Glu Val Gln Leu Val Glu Ser Gly Gly Gly Leu Val Gln Pro Gly Gly
   1 5 10 15
   Ser Leu Arg Leu Ser Cys Arg Ala Ser Gly Ser Ile Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Asn Gly Ala Thr His Tyr Ala Glu
   50 55 60
   Ser Val Lys Gly Arg Phe Ile Ile Ser Arg Asp Asp Asn Lys Asn Ser
   65 70 75 80
   Leu Tyr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 34
   <211> 119
   <212> PRT
   <213> Artificial Sequence
<220>
   <223> Infliximab VH variant
<400> 34
   Glu Val Gln Leu Val Glu Ser Gly Gly Gly Leu Val Gln Pro Gly Gly
    1               5                  10                  15
   Ser Leu Arg Leu Ser Cys Ala Ala Ser Gln Ser Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr Ser Tyr Ala Glu
       50                  55                  60
   Ser Val Lys Gly Arg Phe Ile Ile Ser Arg Asp Gly Asn Gly Thr Ser
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 35
   <211> 119
   <212> PRT
   <213> Artificial Sequence
<220>
   <223> Infliximab VH variant
<400> 35
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr Ser Tyr Ala Glu
       50                  55                  60
   Ser Val Lys Gly Arg Phe Ile Ile Ser Arg Asp Gly Asn Gly Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 36
   <211> 119
   <212> PRT
   <213> Artificial Sequence
<220>
   <223> Infliximab VH variant
<400> 36
   Glu Val Gln Leu Val Glu Ser Gly Gly Gly Leu Val Gln Pro Gly Gly
    1               5                  10                  15
   Ser Leu Arg Leu Ser Cys Ala Ala Ser Gln Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Gly Ala Thr Ser Tyr Ala Glu
       50                  55                  60
   Ser Val Lys Gly Arg Phe Ile Ile Ser Arg Asp Asp Asn Gly Thr Ser
   65                  70                  75                      80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 37
   <211> 119
   <212> PRT
   <213> Artificial Sequence
<220>
   <223> Infliximab VH variant
<400> 37
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Asn Ser Ala Thr Ser Tyr Ala Glu
       50                  55                  60
   Ser Val Lys Gly Arg Phe Ile Ile Ser Arg Asp Gly Asn Gly Asn Asp
   65                  70                  75                      80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 38
   <211> 119
   <212> PRT
   <213> Artificial Sequence
<220>
   <223> Infliximab VH variant
<400> 38
   Glu Val Gln Leu Thr Glu Ser Gly Gly Gly Leu Val Gln Pro Gly Gly
   1 5 10 15
   Ser Leu Arg Leu Ser Cys Arg Ala Ser Gln Ser Thr Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ser Tyr Gly Ala Thr Ser Tyr Ala Glu
   50 55 60
   Ser Val Lys Gly Arg Phe Ile Ile Ser Arg Asp Gly Asn Gly Asn Ser
   65 70 75 80
   Leu Thr Leu Glu Met Asn Ser Leu Lys Thr Glu Asp Thr Ala Glu Tyr
   85 90 95
   Tyr Cys Ala Gln Asn Tyr Tyr Gly Ser Thr Tyr Asp Tyr Trp Gly Gln
   100 105 110
   Gly Thr Leu Val Thr Val Ser
   115
<210> 39
   <211> 119
   <212> PRT
   <213> Artificial Sequence
<220>
   <223> Infliximab VH variant
<400> 39
   Glu Val Gln Leu Val Glu Ser Gly Gly Gly Leu Val Gln Pro Gly Gly
   1 5 10 15
   Ser Leu Arg Leu Ser Cys Arg Ala Ser Gln Ser Thr Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ile Asn Ser Ala Thr His Tyr Ala Glu
   50 55 60
   Ser Val Lys Gly Arg Phe Ile Ile Ser Arg Asp Gly Asn Lys Asn Asp
   65 70 75 80
   Leu Thr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 40
   <211> 119
   <212> PRT
   <213> Artificial Sequence
<220>
   <223> Infliximab VH variant
<400> 40
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr Ser Tyr Ala Glu
       50                  55                  60
   Ser Val Lys Gly Arg Phe Ile Ile Ser Arg Asp Gly Asn Gly Thr Ser
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 41
   <211> 119
   <212> PRT
   <213> Artificial Sequence
<220>
   <223> Infliximab VH variant
<400> 41
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Asn Gly Ala Thr Ser Tyr Ala Glu
       50                  55                  60
   Ser Val Lys Gly Arg Phe Ile Ile Ser Arg Asp Asp Asn Gly Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 42
   <211> 119
   <212> PRT
   <213> Artificial Sequence
<220>
   <223> Infliximab VH variant
<400> 42
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Tyr Ser Ala Thr Ser Tyr Ala Glu
   50 55 60
   Ser Val Lys Gly Arg Phe Ile Ile Ser Arg Asp Asp Asn Lys Asn Ser
   65 70 75 80
   Leu Thr Leu Glu Met Asn Ser Leu Lys Thr Glu Asp Thr Ala Glu Tyr
   85 90 95
   Tyr Cys Ala Arg Asn Tyr Tyr Gly Ser Thr Pro Asp Tyr Trp Gly Gln
   100 105 110
   Gly Thr Leu Val Thr Val Ser
   115
<210> 43
   <211> 119
   <212> PRT
   <213> Artificial Sequence
<220>
   <223> Infliximab VH variant
<400> 43
   Glu Val Gln Leu Val Glu Ser Gly Gly Gly Leu Val Gln Pro Gly Gly
   1 5 10 15
   Ser Leu Arg Leu Ser Cys Arg Ala Ser Gly Ser Ile Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Asn Gly Ala Thr Ser Tyr Ala Glu
   50 55 60
   Ser Val Lys Gly Arg Phe Ile Ile Ser Arg Asp Asp Asn Gly Asn Ser
   65 70 75 80
   Leu Thr Leu Glu Met Asn Ser Leu Lys Thr Glu Asp Thr Ala Glu Tyr
   85 90 95
   Tyr Cys Ala Gln Asn Tyr Tyr Gly Ser Thr Pro Asp Tyr Trp Gly Gln
   100 105 110
   Gly Thr Leu Val Thr Val Ser
   115
<210> 44
   <211> 119
   <212> PRT
   <213> Artificial Sequence
<220>
   <223> Infliximab VH variant
<400> 44
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gly Phe Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Tyr Ser Ala Thr His Tyr Ala Glu
       50                  55                  60
   Ser Val Lys Gly Arg Phe Ile Ile Ser Arg Asp Asp Asn Lys Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 45
   <211> 119
   <212> PRT
   <213> Artificial Sequence
<220>
   <223> Infliximab VH variant
<400> 45
   Glu Val Gln Leu Val Glu Ser Gly Gly Gly Leu Val Gln Pro Gly Gly
    1               5                  10                  15
   Ser Leu Arg Leu Ser Cys Arg Ala Ser Gly Phe Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr His Tyr Ala Glu
       50                  55                  60
   Ser Val Lys Gly Arg Phe Ile Ile Ser Arg Asp Gly Asn Gly Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 46
   <211> 119
   <212> PRT
   <213> Artificial Sequence
<220>
   <223> Infliximab VH variant
<400> 46
   Glu Val Gln Leu Val Glu Ser Gly Gly Gly Leu Val Gln Pro Gly Gly
    1               5                  10                  15
   Ser Leu Arg Leu Ser Cys Arg Ala Ser Gly Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Gly Ala Thr Ser Tyr Ala Glu
       50                  55                  60
   Ser Val Lys Gly Arg Phe Ile Ile Ser Arg Asp Gly Asn Gly Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 47
   <211> 119
   <212> PRT
   <213> Artificial Sequence
<220>
   <223> Infliximab VH variant
<400> 47
   Glu Val Gln Leu Val Glu Ser Gly Gly Gly Leu Val Gln Pro Gly Gly
    1               5                  10                  15
   Ser Leu Arg Leu Ser Cys Arg Ala Ser Gln Ser Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr His Tyr Ala Glu
       50                  55                  60
   Ser Val Lys Gly Arg Phe Ile Ile Ser Arg Asp Asp Asn Gly Thr Ser
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 48
   <211> 119
   <212> PRT
   <213> Artificial Sequence
<220>
   <223> Infliximab VH variant
<400> 48
   Glu Val Gln Leu Thr Glu Ser Gly Gly Gly Leu Val Gln Pro Gly Gly
   1 5 10 15
   Ser Leu Arg Leu Ser Cys Arg Ala Ser Gln Phe Ile Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ile Tyr Gly Ala Thr His Tyr Ala Glu
   50 55 60
   Ser Val Lys Gly Arg Phe Ile Ile Ser Arg Asp Asp Asn Lys Thr Asp
   65 70 75 80
   Leu Tyr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 49
   <211> 119
   <212> PRT
   <213> Artificial Sequence
<220>
   <223> Infliximab VH variant
<400> 49
   Glu Val Gln Leu Thr Glu Ser Gly Gly Gly Leu Val Gln Pro Gly Gly
   1 5 10 15
   Ser Leu Arg Leu Ser Cys Ala Ala Ser Gln Phe Ile Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ser Tyr Gly Ala Thr His Tyr Ala Glu
   50 55 60
   Ser Val Lys Gly Arg Phe Ile Ile Ser Arg Asp Gly Asn Gly Asn Ser
   65 70 75 80
   Leu Tyr Leu Glu Met Asn Ser Leu Lys Thr Glu Asp Thr Ala Glu Tyr
   85 90 95
   Tyr Cys Ala Gln Asn Tyr Tyr Gly Ser Thr Tyr Asp Tyr Trp Gly Gln
   100 105 110
   Gly Thr Leu Val Thr Val Ser
   115
<210> 50
   <211> 119
   <212> PRT
   <213> Artificial Sequence
<220>
   <223> Infliximab VH variant
<400> 50
   Glu Val Gln Leu Thr Glu Ser Gly Gly Gly Leu Val Gln Pro Gly Gly
   1 5 10 15
   Ser Leu Arg Leu Ser Cys Ala Ala Ser Gln Ser Ile Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Tyr Ser Ala Thr Ser Tyr Ala Glu
       50                  55                  60
   Ser Val Lys Gly Arg Phe Ile Ile Ser Arg Asp Asp Asn Gly Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 51
   <211> 119
   <212> PRT
   <213> Artificial Sequence
<220>
   <223> Infliximab VH variant
<400> 51
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gly Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Ser Ala Thr Ser Tyr Ala Glu
       50                  55                  60
   Ser Val Lys Gly Arg Phe Ile Ile Ser Arg Asp Gly Asn Lys Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 52
   <211> 119
   <212> PRT
   <213> Artificial Sequence
<220>
   <223> Infliximab VH variant
<400> 52
   Glu Val Gln Leu Val Glu Ser Gly Gly Gly Leu Val Gln Pro Gly Gly
    1               5                  10                  15
   Ser Leu Arg Leu Ser Cys Arg Ala Ser Gly Ser Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Tyr Ser Ala Thr His Tyr Ala Glu
       50                  55                  60
   Ser Val Lys Gly Arg Phe Ile Ile Ser Arg Asp Gly Asn Gly Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 53
   <211> 119
   <212> PRT
   <213> Artificial Sequence
<220>
   <223> Infliximab VH variant
<400> 53
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Ser Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Tyr Gly Ala Thr His Tyr Ala Glu
       50                  55                  60
   Ser Val Lys Gly Arg Phe Ile Ile Ser Arg Asp Asp Asn Gly Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 54
   <211> 119
   <212> PRT
   <213> Artificial Sequence
<220>
   <223> Infliximab VH variant
<400> 54
   Glu Val Gln Leu Thr Glu Ser Gly Gly Gly Leu Val Gln Pro Gly Gly
   1 5 10 15
   Ser Leu Arg Leu Ser Cys Ala Ala Ser Gly Ser Ile Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ile Tyr Gly Ala Thr Ser Tyr Ala Glu
   50 55 60
   Ser Val Lys Gly Arg Phe Ile Ile Ser Arg Asp Gly Asn Gly Asn Ser
   65 70 75 80
   Leu Tyr Leu Glu Met Asn Ser Leu Lys Thr Glu Asp Thr Ala Glu Tyr
   85 90 95
   Tyr Cys Ala Gln Asn Tyr Tyr Gly Ser Thr Pro Asp Tyr Trp Gly Gln
   100 105 110
   Gly Thr Leu Val Thr Val Ser
   115
<210> 55
   <211> 119
   <212> PRT
   <213> Artificial Sequence
<220>
   <223> Infliximab VH variant
<400> 55
   Glu Val Gln Leu Thr Glu Ser Gly Gly Gly Leu Val Gln Pro Gly Gly
   1 5 10 15
   Ser Leu Arg Leu Ser Cys Arg Ala Ser Gly Ser Ile Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Tyr Gly Ala Thr His Tyr Ala Glu
   50 55 60
   Ser Val Lys Gly Arg Phe Ile Ile Ser Arg Asp Gly Asn Gly Thr Asp
   65 70 75 80
   Leu Thr Leu Glu Met Asn Ser Leu Lys Thr Glu Asp Thr Ala Glu Tyr
   85 90 95
   Tyr Cys Ala Gln Asn Tyr Tyr Gly Ser Thr Tyr Asp Tyr Trp Gly Gln
   100 105 110
   Gly Thr Leu Val Thr Val Ser
   115
<210> 56
   <211> 119
   <212> PRT
   <213> Artificial Sequence
<220>
   <223> Infliximab VH variant
<400> 56
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gly Ser Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Gly Ala Thr His Tyr Ala Glu
       50                  55                  60
   Ser Val Lys Gly Arg Phe Ile Ile Ser Arg Asp Gly Asn Gly Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 57
   <211> 119
   <212> PRT
   <213> Artificial Sequence
<220>
   <223> Infliximab VH variant
<400> 57
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gly Phe Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Tyr Ser Ala Thr His Tyr Ala Glu
       50                  55                  60
   Ser Val Lys Gly Arg Phe Ile Ile Ser Arg Asp Asp Asn Gly Thr Ser
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 58
   <211> 119
   <212> PRT
   <213> Artificial Sequence
<220>
   <223> Infliximab VH variant
<400> 58
   Glu Val Gln Leu Thr Glu Ser Gly Gly Gly Leu Val Gln Pro Gly Gly
   1 5 10 15
   Ser Leu Arg Leu Ser Cys Ala Ala Ser Gln Phe Ile Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ser Asn Ser Ala Thr Ser Tyr Ala Glu
   50 55 60
   Ser Val Lys Gly Arg Phe Ile Ile Ser Arg Asp Asp Asn Gly Asn Asp
   65 70 75 80
   Leu Tyr Leu Glu Met Asn Ser Leu Lys Thr Glu Asp Thr Ala Glu Tyr
   85 90 95
   Tyr Cys Ala Gln Asn Tyr Tyr Gly Ser Thr Tyr Asp Tyr Trp Gly Gln
   100 105 110
   Gly Thr Leu Val Thr Val Ser
   115
<210> 59
   <211> 119
   <212> PRT
   <213> Artificial Sequence
<220>
   <223> Infliximab VH variant
<400> 59
   Glu Val Gln Leu Val Glu Ser Gly Gly Gly Leu Val Gln Pro Gly Gly
   1 5 10 15
   Ser Leu Arg Leu Ser Cys Arg Ala Ser Gln Ser Ile Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Asn Ser Ala Thr Ser Tyr Ala Glu
   50 55 60
   Ser Val Lys Gly Arg Phe Ile Ile Ser Arg Asp Asp Asn Gly Thr Ser
   65 70 75 80
   Leu Tyr Leu Glu Met Asn Ser Leu Lys Thr Glu Asp Thr Ala Glu Tyr
   85 90 95
   Tyr Cys Ala Gln Asn Tyr Tyr Gly Ser Thr Tyr Asp Tyr Trp Gly Gln
   100 105 110
   Gly Thr Leu Val Thr Val Ser
   115
<210> 60
   <211> 119
   <212> PRT
   <213> Artificial Sequence
<220>
   <223> Infliximab VH variant
<400> 60
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Ser Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr Ser Tyr Ala Glu
       50                  55                  60
   Ser Val Lys Gly Arg Phe Ile Ile Ser Arg Asp Asp Asn Gly Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 61
   <211> 119
   <212> PRT
   <213> Artificial Sequence
<220>
   <223> Infliximab VH variant
<400> 61
   Glu Val Gln Leu Val Glu Ser Gly Gly Gly Leu Val Gln Pro Gly Gly
    1               5                  10                  15
   Ser Leu Arg Leu Ser Cys Arg Ala Ser Gly Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Gly Ala Thr His Tyr Ala Glu
       50                  55                  60
   Ser Val Lys Gly Arg Phe Ile Ile Ser Arg Asp Gly Asn Gly Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 62
   <211> 119
   <212> PRT
   <213> Artificial Sequence
<220>
   <223> Infliximab VH variant
<400> 62
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gln Ser Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Asn Gly Ala Thr Ser Tyr Ala Glu
       50                  55                  60
   Ser Val Lys Gly Arg Phe Ile Ile Ser Arg Asp Gly Asn Lys Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 63
   <211> 119
   <212> PRT
   <213> Artificial Sequence
<220>
   <223> Infliximab VH variant
<400> 63
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gly Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Gly Ala Thr Ser Tyr Ala Glu
       50                  55                  60
   Ser Val Lys Gly Arg Phe Ile Ile Ser Arg Asp Gly Asn Gly Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 64
   <211> 119
   <212> PRT
   <213> Artificial Sequence
<220>
   <223> Infliximab VH variant
<400> 64
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gln Phe Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Tyr Gly Ala Thr His Tyr Ala Glu
       50                  55                  60
   Ser Val Lys Gly Arg Phe Ile Ile Ser Arg Asp Asp Asn Lys Thr Ser
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 65
   <211> 119
   <212> PRT
   <213> Artificial Sequence
<220>
   <223> Infliximab VH variant
<400> 65
   Glu Val Gln Leu Val Glu Ser Gly Gly Gly Leu Val Gln Pro Gly Gly
    1               5                  10                  15
   Ser Leu Arg Leu Ser Cys Arg Ala Ser Gly Phe Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Tyr Gly Ala Thr His Tyr Ala Glu
       50                  55                  60
   Ser Val Lys Gly Arg Phe Ile Ile Ser Arg Asp Asp Asn Lys Asn Ser
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 66
   <211> 119
   <212> PRT
   <213> Artificial Sequence
<220>
   <223> Infliximab VH variant
<400> 66
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gly Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr His Tyr Ala Glu
       50                  55                  60
   Ser Val Lys Gly Arg Phe Ile Ile Ser Arg Asp Asp Asn Lys Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 67
   <211> 119
   <212> PRT
   <213> Artificial Sequence
<220>
   <223> Infliximab VH variant
<400> 67
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gly Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr His Tyr Ala Glu
       50                  55                  60
   Ser Val Lys Gly Arg Phe Ile Ile Ser Arg Asp Gly Asn Lys Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 68
   <211> 119
   <212> PRT
   <213> Artificial Sequence
<220>
   <223> Infliximab VH variant
<400> 68
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Phe Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr His Tyr Ala Glu
       50                  55                  60
   Ser Val Lys Gly Arg Phe Ile Ile Ser Arg Asp Asp Asn Lys Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 69
   <211> 119
   <212> PRT
   <213> Artificial Sequence
<220>
   <223> Infliximab VH variant
<400> 69
   Glu Val Gln Leu Val Glu Ser Gly Gly Gly Leu Val Gln Pro Gly Gly
    1               5                  10                  15
   Ser Leu Arg Leu Ser Cys Arg Ala Ser Gln Phe Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Asn Ser Ala Thr His Tyr Ala Glu
       50                  55                  60
   Ser Val Lys Gly Arg Phe Ile Ile Ser Arg Asp Gly Asn Gly Asn Ser
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 70
   <211> 119
   <212> PRT
   <213> Artificial Sequence
<220>
   <223> Infliximab VH variant
<400> 70
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Ser Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr His Tyr Ala Glu
       50                  55                  60
   Ser Val Lys Gly Arg Phe Ile Ile Ser Arg Asp Asp Asn Lys Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 71
   <211> 119
   <212> PRT
   <213> Artificial Sequence
<220>
   <223> Infliximab VH variant
<400> 71
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gly Ser Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Tyr Gly Ala Thr Ser Tyr Ala Glu
       50                  55                  60
   Ser Val Lys Gly Arg Phe Ile Ile Ser Arg Asp Gly Asn Lys Thr Ser
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 72
   <211> 119
   <212> PRT
   <213> Artificial Sequence
<220>
   <223> Infliximab VH variant
<400> 72
   Glu Val Gln Leu Thr Glu Ser Gly Gly Gly Leu Val Gln Pro Gly Gly
   1 5 10 15
   Ser Leu Arg Leu Ser Cys Arg Ala Ser Gly Ser Ile Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ile Tyr Ser Ala Thr His Tyr Ala Glu
   50 55 60
   Ser Val Lys Gly Arg Phe Ile Ile Ser Arg Asp Gly Asn Gly Thr Ser
   65 70 75 80
   Leu Tyr Leu Glu Met Asn Ser Leu Lys Thr Glu Asp Thr Ala Glu Tyr
   85 90 95
   Tyr Cys Ala Gln Asn Tyr Tyr Gly Ser Thr Tyr Asp Tyr Trp Gly Gln
   100 105 110
   Gly Thr Leu Val Thr Val Ser
   115
<210> 73
   <211> 119
   <212> PRT
   <213> Artificial Sequence
<220>
   <223> Infliximab VH variant
<400> 73
   Glu Val Gln Leu Val Glu Ser Gly Gly Gly Leu Val Gln Pro Gly Gly
   1 5 10 15
   Ser Leu Arg Leu Ser Cys Arg Ala Ser Gly Ser Ile Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Tyr Gly Ala Thr Ser Tyr Ala Glu
   50 55 60
   Ser Val Lys Gly Arg Phe Ile Ile Ser Arg Asp Asp Asn Lys Asn Asp
   65 70 75 80
   Leu Thr Leu Glu Met Asn Ser Leu Lys Thr Glu Asp Thr Ala Glu Tyr
   85 90 95
   Tyr Cys Ala Gln Asn Tyr Tyr Gly Ser Thr Pro Asp Tyr Trp Gly Gln
   100 105 110
   Gly Thr Leu Val Thr Val Ser
   115
<210> 74
   <211> 119
   <212> PRT
   <213> Artificial Sequence
<220>
   <223> Infliximab VH variant
<400> 74
   Glu Val Gln Leu Thr Glu Ser Gly Gly Gly Leu Val Gln Pro Gly Gly
    1              5                  10                  15
   Ser Leu Arg Leu Ser Cys Ala Ala Ser Gly Ser Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Tyr Ser Ala Thr Ser Tyr Ala Glu
       50                  55                  60
   Ser Val Lys Gly Arg Phe Ile Ile Ser Arg Asp Asp Asn Lys Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 75
   <211> 119
   <212> PRT
   <213> Artificial Sequence
<220>
   <223> Infliximab VH variant
<400> 75
   Glu Val Gln Leu Val Glu Ser Gly Gly Gly Leu Val Gln Pro Gly Gly
    1              5                  10                  15
   Ser Leu Arg Leu Ser Cys Arg Ala Ser Gly Phe Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr His Tyr Ala Glu
       50                  55                  60
   Ser Val Lys Gly Arg Phe Ile Ile Ser Arg Asp Asp Asn Lys Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 76
   <211> 119
   <212> PRT
   <213> Artificial Sequence
<220>
   <223> Infliximab VH variant
<400> 76
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gly Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr His Tyr Ala Glu
       50                  55                  60
   Ser Val Lys Gly Arg Phe Ile Ile Ser Arg Asp Asp Asn Gly Thr Ser
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 77
   <211> 119
   <212> PRT
   <213> Artificial Sequence
<220>
   <223> Infliximab VH variant
<400> 77
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr Ser Tyr Ala Glu
       50                  55                  60
   Ser Val Lys Gly Arg Phe Ile Ile Ser Arg Asp Asp Asn Gly Asn Ser
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 78
   <211> 119
   <212> PRT
   <213> Artificial Sequence
<220>
   <223> Infliximab VH variant
<400> 78
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Gly Ala Thr Ser Tyr Ala Glu
       50                  55                  60
   Ser Val Lys Gly Arg Phe Ile Ile Ser Arg Asp Asp Asn Gly Thr Ser
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 79
   <211> 119
   <212> PRT
   <213> Artificial Sequence
<220>
   <223> Infliximab VH variant
<400> 79
   Glu Val Gln Leu Val Glu Ser Gly Gly Gly Leu Val Gln Pro Gly Gly
    1               5                  10                  15
   Ser Leu Arg Leu Ser Cys Ala Ala Ser Gln Ser Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Gly Ala Thr His Tyr Ala Glu
       50                  55                  60
   Ser Val Lys Gly Arg Phe Ile Ile Ser Arg Asp Gly Asn Lys Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 80
   <211> 119
   <212> PRT
   <213> Artificial Sequence
<220>
   <223> Infliximab VH variant
<400> 80
   Glu Val Gln Leu Val Glu Ser Gly Gly Gly Leu Val Gln Pro Gly Gly
    1               5                  10                  15
   Ser Leu Arg Leu Ser Cys Arg Ala Ser Gln Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr His Tyr Ala Glu
       50                  55                  60
   Ser Val Lys Gly Arg Phe Ile Ile Ser Arg Asp Gly Asn Gly Thr Asp
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 81
   <211> 119
   <212> PRT
   <213> Artificial Sequence
<220>
   <223> Infliximab VH variant
<400> 81
   Glu Val Gln Leu Val Glu Ser Gly Gly Gly Leu Val Gln Pro Gly Gly
    1               5                  10                  15
   Ser Leu Arg Leu Ser Cys Arg Ala Ser Gly Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr Ser Tyr Ala Glu
       50                  55                  60
   Ser Val Lys Gly Arg Phe Ile Ile Ser Arg Asp Gly Asn Gly Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 82
   <211> 119
   <212> PRT
   <213> Artificial Sequence
<220>
   <223> Infliximab VH variant
<400> 82
   Glu Val Gln Leu Val Glu Ser Gly Gly Gly Leu Val Gln Pro Gly Gly
    1               5                  10                  15
   Ser Leu Arg Leu Ser Cys Arg Ala Ser Gln Ser Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr His Tyr Ala Glu
       50                  55                  60
   Ser Val Lys Gly Arg Phe Ile Ile Ser Arg Asp Gly Asn Gly Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 83
   <211> 119
   <212> PRT
   <213> Artificial Sequence
<220>
   <223> Infliximab VH variant
<400> 83
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gly Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr Ser Tyr Ala Glu
       50                  55                  60
   Ser Val Lys Gly Arg Phe Ile Ile Ser Arg Asp Asp Asn Gly Asn Ser
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 84
   <211> 119
   <212> PRT
   <213> Artificial Sequence
<220>
   <223> Infliximab VH variant
<400> 84
   Glu Val Gln Leu Val Glu Ser Gly Gly Gly Leu Val Gln Pro Gly Gly
    1               5                  10                  15
   Ser Leu Arg Leu Ser Cys Ala Ala Ser Gln Ser Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Tyr Ser Ala Thr His Tyr Ala Glu
       50                  55                  60
   Ser Val Lys Gly Arg Phe Ile Ile Ser Arg Asp Gly Asn Lys Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 85
   <211> 119
   <212> PRT
   <213> Artificial Sequence
<220>
   <223> Infliximab VH variant
<400> 85
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gly Phe Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Asn Gly Ala Thr Ser Tyr Ala Glu
       50                  55                  60
   Ser Val Lys Gly Arg Phe Ile Ile Ser Arg Asp Gly Asn Gly Thr Ser
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 86
   <211> 119
   <212> PRT
   <213> Artificial Sequence
<220>
   <223> Infliximab VH variant
<400> 86
   Glu Val Gln Leu Thr Glu Ser Gly Gly Gly Leu Val Gln Pro Gly Gly
   1               5                   10                  15
   Ser Leu Arg Leu Ser Cys Ala Ala Ser Gln Ser Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Gly Ala Thr His Tyr Ala Glu
       50                  55                  60
   Ser Val Lys Gly Arg Phe Ile Ile Ser Arg Asp Asp Asn Gly Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 87
   <211> 119
   <212> PRT
   <213> Artificial Sequence
<220>
   <223> Infliximab VH variant
<400> 87
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Asn Ser Ala Thr His Tyr Ala Glu
       50                  55                  60
   Ser Val Lys Gly Arg Phe Ile Ile Ser Arg Asp Gly Asn Lys Thr Ser
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 88
   <211> 119
   <212> PRT
   <213> Artificial Sequence
<220>
   <223> Infliximab VH variant
<400> 88
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Asn Ser Ala Thr Ser Tyr Ala Glu
       50                  55                  60
   Ser Val Lys Gly Arg Phe Ile Ile Ser Arg Asp Gly Asn Lys Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 89
   <211> 119
   <212> PRT
   <213> Artificial Sequence
<220>
   <223> Infliximab VH variant
<400> 89
   Glu Val Gln Leu Val Glu Ser Gly Gly Gly Leu Val Gln Pro Gly Gly
    1               5                  10                  15
   Ser Leu Arg Leu Ser Cys Arg Ala Ser Gln Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr Ser Tyr Ala Glu
       50                  55                  60
   Ser Val Lys Gly Arg Phe Ile Ile Ser Arg Asp Asp Asn Gly Thr Asp
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 90
   <211> 119
   <212> PRT
   <213> Artificial Sequence
<220>
   <223> Infliximab VH variant
<400> 90
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr His Tyr Ala Glu
       50                  55                  60
   Ser Val Lys Gly Arg Phe Ile Ile Ser Arg Asp Asp Asn Gly Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 91
   <211> 119
   <212> PRT
   <213> Artificial Sequence
<220>
   <223> Infliximab VH variant
<400> 91
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gln Ser Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr His Tyr Ala Glu
       50                  55                  60
   Ser Val Lys Gly Arg Phe Ile Ile Ser Arg Asp Gly Asn Lys Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 92
   <211> 119
   <212> PRT
   <213> Artificial Sequence
<220>
   <223> Infliximab VH variant
<400> 92
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Ser Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Asn Ser Ala Thr His Tyr Ala Glu
       50                  55                  60
   Ser Val Lys Gly Arg Phe Ile Ile Ser Arg Asp Gly Asn Lys Asn Asp
   65                  70                  75                      80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 93
   <211> 119
   <212> PRT
   <213> Artificial Sequence
<220>
   <223> Infliximab VH variant
<400> 93
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gly Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Gly Ala Thr His Tyr Ala Glu
       50                  55                  60
   Ser Val Lys Gly Arg Phe Ile Ile Ser Arg Asp Asp Asn Gly Thr Asp
   65                  70                  75                      80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 94
   <211> 119
   <212> PRT
   <213> Artificial Sequence
<220>
   <223> Infliximab VH variant
<400> 94
   Glu Val Gln Leu Val Glu Ser Gly Gly Gly Leu Val Gln Pro Gly Gly
   1 5 10 15
   Ser Leu Arg Leu Ser Cys Arg Ala Ser Gly Ser Ile Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Asn Gly Ala Thr His Tyr Ala Glu
   50 55 60
   Ser Val Lys Gly Arg Phe Ile Ile Ser Arg Asp Asp Asn Gly Thr Ser
   65 70 75 80
   Leu Tyr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 95
   <211> 119
   <212> PRT
   <213> Artificial Sequence
<220>
   <223> Infliximab VH variant
<400> 95
   Glu Val Gln Leu Thr Glu Ser Gly Gly Gly Leu Val Gln Pro Gly Gly
   1 5 10 15
   Ser Leu Arg Leu Ser Cys Arg Ala Ser Gly Ser Thr Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Asn Ser Ala Thr Ser Tyr Ala Glu
   50 55 60
   Ser Val Lys Gly Arg Phe Ile Ile Ser Arg Asp Asp Asn Lys Thr Ser
   65 70 75 80
   Leu Tyr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 96
   <211> 119
   <212> PRT
   <213> Artificial Sequence
<220>
   <223> Infliximab VH variant
<400> 96
   Glu Val Gln Leu Val Glu Ser Gly Gly Gly Leu Val Gln Pro Gly Gly
    1               5                  10                  15
   Ser Leu Arg Leu Ser Cys Arg Ala Ser Gln Ser Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr His Tyr Ala Glu
       50                  55                  60
   Ser Val Lys Gly Arg Phe Ile Ile Ser Arg Asp Asp Asn Gly Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 97
   <211> 119
   <212> PRT
   <213> Artificial Sequence
<220>
   <223> Infliximab VH variant
<400> 97
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gly Ser Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Asn Gly Ala Thr Ser Tyr Ala Glu
       50                  55                  60
   Ser Val Lys Gly Arg Phe Ile Ile Ser Arg Asp Asp Asn Gly Thr Asp
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 98
   <211> 119
   <212> PRT
   <213> Artificial Sequence
<220>
   <223> Infliximab VH variant
<400> 98
   Glu Val Gln Leu Thr Glu Ser Gly Gly Gly Leu Val Gln Pro Gly Gly
   1 5 10 15
   Ser Leu Arg Leu Ser Cys Arg Ala Ser Gly Phe Thr Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ser Asn Gly Ala Thr His Tyr Ala Glu
   50 55 60
   Ser Val Lys Gly Arg Phe Ile Ile Ser Arg Asp Gly Asn Lys Thr Asp
   65 70 75 80
   Leu Tyr Leu Glu Met Asn Ser Leu Lys Thr Glu Asp Thr Ala Glu Tyr
   85 90 95
   Tyr Cys Ala Arg Asn Tyr Tyr Gly Ser Thr Pro Asp Tyr Trp Gly Gln
   100 105 110
   Gly Thr Leu Val Thr Val Ser
   115
<210> 99
   <211> 119
   <212> PRT
   <213> Artificial Sequence
<220>
   <223> Infliximab VH variant
<400> 99
   Glu Val Gln Leu Val Glu Ser Gly Gly Gly Leu Val Gln Pro Gly Gly
   1 5 10 15
   Ser Leu Arg Leu Ser Cys Ala Ala Ser Gly Ser Ile Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Asn Gly Ala Thr His Tyr Ala Glu
   50 55 60
   Ser Val Lys Gly Arg Phe Ile Ile Ser Arg Asp Asp Asn Lys Asn Ser
   65 70 75 80
   Leu Tyr Leu Glu Met Asn Ser Leu Lys Thr Glu Asp Thr Ala Glu Tyr
   85 90 95
   Tyr Cys Ala Gln Asn Tyr Tyr Gly Ser Thr Tyr Asp Tyr Trp Gly Gln
   100 105 110
   Gly Thr Leu Val Thr Val Ser
   115
<210> 100
   <211> 119
   <212> PRT
   <213> Artificial Sequence
<220>
   <223> Infliximab VH variant
<400> 100
   Glu Val Gln Leu Thr Glu Ser Gly Gly Gly Leu Val Gln Pro Gly Gly
    1               5                   10                  15
   Ser Leu Arg Leu Ser Cys Arg Ala Ser Gln Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr His Tyr Ala Glu
       50                  55                  60
   Ser Val Lys Gly Arg Phe Ile Ile Ser Arg Asp Asp Asn Lys Thr Asp
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 101
   <211> 119
   <212> PRT
   <213> Artificial Sequence
<220>
   <223> Infliximab VH variant
<400> 101
   Glu Val Gln Leu Val Glu Ser Gly Gly Gly Leu Val Gln Pro Gly Gly
    1               5                   10                  15
   Ser Leu Arg Leu Ser Cys Arg Ala Ser Gly Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Asn Ser Ala Thr Ser Tyr Ala Glu
       50                  55                  60
   Ser Val Lys Gly Arg Phe Ile Ile Ser Arg Asp Asp Asn Gly Asn Ser
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 102
   <211> 119
   <212> PRT
   <213> Artificial Sequence
<220>
   <223> Infliximab VH variant
<400> 102
   Glu Val Gln Leu Val Glu Ser Gly Gly Gly Leu Val Gln Pro Gly Gly
   1 5 10 15
   Ser Leu Arg Leu Ser Cys Arg Ala Ser Gly Phe Ile Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Asn Gly Ala Thr His Tyr Ala Glu
   50 55 60
   Ser Val Lys Gly Arg Phe Ile Ile Ser Arg Asp Asp Asn Lys Thr Ser
   65 70 75 80
   Leu Tyr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 103
   <211> 119
   <212> PRT
   <213> Artificial Sequence
<220>
   <223> Infliximab VH variant
<400> 103
   Glu Val Gln Leu Thr Glu Ser Gly Gly Gly Leu Val Gln Pro Gly Gly
   1 5 10 15
   Ser Leu Arg Leu Ser Cys Ala Ala Ser Gln Ser Thr Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Asn Gly Ala Thr Ser Tyr Ala Glu
   50 55 60
   Ser Val Lys Gly Arg Phe Ile Ile Ser Arg Asp Asp Asn Gly Asn Ser
   65 70 75 80
   Leu Thr Leu Glu Met Asn Ser Leu Lys Thr Glu Asp Thr Ala Glu Tyr
   85 90 95
   Tyr Cys Ala Arg Asn Tyr Tyr Gly Ser Thr Pro Asp Tyr Trp Gly Gln
   100 105 110
   Gly Thr Leu Val Thr Val Ser
   115
<210> 104
   <211> 119
   <212> PRT
   <213> Artificial Sequence
<220>
   <223> Infliximab VH variant
<400> 104
   Glu Val Gln Leu Thr Glu Ser Gly Gly Gly Leu Val Gln Pro Gly Gly
   1               5                   10                  15
   Ser Leu Arg Leu Ser Cys Arg Ala Ser Gln Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr His Tyr Ala Glu
       50                  55                  60
   Ser Val Lys Gly Arg Phe Ile Ile Ser Arg Asp Asp Asn Lys Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 105
   <211> 119
   <212> PRT
   <213> Artificial Sequence
<220>
   <223> Infliximab VH variant
<400> 105
   Glu Val Gln Leu Thr Glu Ser Gly Gly Gly Leu Val Gln Pro Gly Gly
   1               5                   10                  15
   Ser Leu Arg Leu Ser Cys Ala Ala Ser Gly Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Asn Gly Ala Thr Ser Tyr Ala Glu
       50                  55                  60
   Ser Val Lys Gly Arg Phe Ile Ile Ser Arg Asp Asp Asn Gly Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 106
   <211> 119
   <212> PRT
   <213> Artificial Sequence
<220>
   <223> Infliximab VH variant
<400> 106
   Glu Val Gln Leu Val Glu Ser Gly Gly Gly Leu Val Gln Pro Gly Gly
    1               5                  10                  15
   Ser Leu Arg Leu Ser Cys Ala Ala Ser Gln Phe Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Asn Gly Ala Thr Ser Tyr Ala Glu
       50                  55                  60
   Ser Val Lys Gly Arg Phe Ile Ile Ser Arg Asp Gly Asn Lys Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 107
   <211> 119
   <212> PRT
   <213> Artificial Sequence
<220>
   <223> Infliximab VH variant
<400> 107
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr His Tyr Ala Glu
       50                  55                  60
   Ser Val Lys Gly Arg Phe Ile Ile Ser Arg Asp Gly Asn Lys Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 108
   <211> 119
   <212> PRT
   <213> Artificial Sequence
<220>
   <223> Infliximab VH variant
<400> 108
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gln Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr Ser Tyr Ala Glu
       50                  55                  60
   Ser Val Lys Gly Arg Phe Ile Ile Ser Arg Asp Gly Asn Lys Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 109
   <211> 119
   <212> PRT
   <213> Artificial Sequence
<220>
   <223> Infliximab VH variant
<400> 109
   Glu Val Gln Leu Val Glu Ser Gly Gly Gly Leu Val Gln Pro Gly Gly
    1               5                  10                  15
   Ser Leu Arg Leu Ser Cys Arg Ala Ser Gly Ser Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Asn Ser Ala Thr His Tyr Ala Glu
       50                  55                  60
   Ser Val Lys Gly Arg Phe Ile Ile Ser Arg Asp Asp Asn Lys Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 110
   <211> 119
   <212> PRT
   <213> Artificial Sequence
<220>
   <223> Infliximab VH variant
<400> 110
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Ser Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr Ser Tyr Ala Glu
       50                  55                  60
   Ser Val Lys Gly Arg Phe Ile Ile Ser Arg Asp Asp Asn Lys Thr Asp
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 111
   <211> 119
   <212> PRT
   <213> Artificial Sequence
<220>
   <223> Infliximab VH variant
<400> 111
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gly Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr His Tyr Ala Glu
       50                  55                  60
   Ser Val Lys Gly Arg Phe Ile Ile Ser Arg Asp Asp Asn Gly Asn Ser
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 112
   <211> 119
   <212> PRT
   <213> Artificial Sequence
<220>
   <223> Infliximab VH variant
<400> 112
   Glu Val Gln Leu Val Glu Ser Gly Gly Gly Leu Val Gln Pro Gly Gly
   1 5 10 15
   Ser Leu Arg Leu Ser Cys Arg Ala Ser Gly Phe Thr Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Tyr Ser Ala Thr His Tyr Ala Glu
   50 55 60
   Ser Val Lys Gly Arg Phe Ile Ile Ser Arg Asp Asp Asn Lys Thr Asp
   65 70 75 80
   Leu Tyr Leu Glu Met Asn Ser Leu Lys Thr Glu Asp Thr Ala Glu Tyr
   85 90 95
   Tyr Cys Ala Arg Asn Tyr Tyr Gly Ser Thr Pro Asp Tyr Trp Gly Gln
   100 105 110
   Gly Thr Leu Val Thr Val Ser
   115
<210> 113
   <211> 119
   <212> PRT
   <213> Artificial Sequence
<220>
   <223> Infliximab VH variant
<400> 113
   Glu Val Gln Leu Val Glu Ser Gly Gly Gly Leu Val Gln Pro Gly Gly
   1 5 10 15
   Ser Leu Arg Leu Ser Cys Arg Ala Ser Gln Phe Ile Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ile Tyr Gly Ala Thr His Tyr Ala Glu
   50 55 60
   Ser Val Lys Gly Arg Phe Ile Ile Ser Arg Asp Gly Asn Gly Thr Asp
   65 70 75 80
   Leu Tyr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 114
   <211> 119
   <212> PRT
   <213> Artificial Sequence
<220>
   <223> Infliximab VH variant
<400> 114
   Glu Val Gln Leu Thr Glu Ser Gly Gly Gly Leu Val Gln Pro Gly Gly
   1 5 10 15
   Ser Leu Arg Leu Ser Cys Arg Ala Ser Gln Ser Thr Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Asn Ser Ala Thr Ser Tyr Ala Glu
   50 55 60
   Ser Val Lys Gly Arg Phe Ile Ile Ser Arg Asp Gly Asn Lys Asn Ser
   65 70 75 80
   Leu Tyr Leu Glu Met Asn Ser Leu Lys Thr Glu Asp Thr Ala Glu Tyr
   85 90 95
   Tyr Cys Ala Gln Asn Tyr Tyr Gly Ser Thr Pro Asp Tyr Trp Gly Gln
   100 105 110
   Gly Thr Leu Val Thr Val Ser
   115
<210> 115
   <211> 119
   <212> PRT
   <213> Artificial Sequence
<220>
   <223> Infliximab VH variant
<400> 115
   Glu Val Gln Leu Val Glu Ser Gly Gly Gly Leu Val Gln Pro Gly Gly
   1 5 10 15
   Ser Leu Arg Leu Ser Cys Arg Ala Ser Gly Ser Ile Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ile Asn Ser Ala Thr His Tyr Ala Glu
   50 55 60
   Ser Val Lys Gly Arg Phe Ile Ile Ser Arg Asp Gly Asn Gly Thr Ser
   65 70 75 80
   Leu Tyr Leu Glu Met Asn Ser Leu Lys Thr Glu Asp Thr Ala Glu Tyr
   85 90 95
   Tyr Cys Ala Gln Asn Tyr Tyr Gly Ser Thr Tyr Asp Tyr Trp Gly Gln
   100 105 110
   Gly Thr Leu Val Thr Val Ser
   115
<210> 116
   <211> 119
   <212> PRT
   <213> Artificial Sequence
<220>
   <223> Infliximab VH variant
<400> 116
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gln Ser Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr His Tyr Ala Glu
       50                  55                  60
   Ser Val Lys Gly Arg Phe Ile Ile Ser Arg Asp Asp Asn Gly Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 117
   <211> 119
   <212> PRT
   <213> Artificial Sequence
<220>
   <223> Infliximab VH variant
<400> 117
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gln Phe Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Asn Ser Ala Thr His Tyr Ala Glu
       50                  55                  60
   Ser Val Lys Gly Arg Phe Ile Ile Ser Arg Asp Gly Asn Lys Thr Ser
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 118
   <211> 119
   <212> PRT
   <213> Artificial Sequence
<220>
   <223> Infliximab VH variant
<400> 118
   Glu Val Gln Leu Val Glu Ser Gly Gly Gly Leu Val Gln Pro Gly Gly
   1 5 10 15
   Ser Leu Arg Leu Ser Cys Ala Ala Ser Gln Phe Thr Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Asn Ser Ala Thr Ser Tyr Ala Glu
   50 55 60
   Ser Val Lys Gly Arg Phe Ile Ile Ser Arg Asp Asp Asn Lys Asn Ser
   65 70 75 80
   Leu Tyr Leu Glu Met Asn Ser Leu Lys Thr Glu Asp Thr Ala Glu Tyr
   85 90 95
   Tyr Cys Ala Arg Asn Tyr Tyr Gly Ser Thr Pro Asp Tyr Trp Gly Gln
   100 105 110
   Gly Thr Leu Val Thr Val Ser
   115
<210> 119
   <211> 119
   <212> PRT
   <213> Artificial Sequence
<220>
   <223> Infliximab VH variant
<400> 119
   Glu Val Gln Leu Thr Glu Ser Gly Gly Gly Leu Val Gln Pro Gly Gly
   1 5 10 15
   Ser Leu Arg Leu Ser Cys Arg Ala Ser Gln Ser Ile Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Tyr Gly Ala Thr Ser Tyr Ala Glu
   50 55 60
   Ser Val Lys Gly Arg Phe Ile Ile Ser Arg Asp Gly Asn Lys Thr Asp
   65 70 75 80
   Leu Tyr Leu Glu Met Asn Ser Leu Lys Thr Glu Asp Thr Ala Glu Tyr
   85 90 95
   Tyr Cys Ala Gln Asn Tyr Tyr Gly Ser Thr Pro Asp Tyr Trp Gly Gln
   100 105 110
   Gly Thr Leu Val Thr Val Ser
   115
<210> 120
   <211> 119
   <212> PRT
   <213> Artificial Sequence
<220>
   <223> Infliximab VH variant
<400> 120
   Glu Val Gln Leu Thr Glu Ser Gly Gly Gly Leu Val Gln Pro Gly Gly
   1 5 10 15
   Ser Leu Arg Leu Ser Cys Arg Ala Ser Gln Ser Thr Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ser Tyr Ser Ala Thr His Tyr Ala Glu
   50 55 60
   Ser Val Lys Gly Arg Phe Ile Ile Ser Arg Asp Gly Asn Gly Asn Ser
   65 70 75 80
   Leu Tyr Leu Glu Met Asn Ser Leu Lys Thr Glu Asp Thr Ala Glu Tyr
   85 90 95
   Tyr Cys Ala Gln Asn Tyr Tyr Gly Ser Thr Pro Asp Tyr Trp Gly Gln
   100 105 110
   Gly Thr Leu Val Thr Val Ser
   115
<210> 121
   <211> 119
   <212> PRT
   <213> Artificial Sequence
<220>
   <223> Infliximab VH variant
<400> 121
   Glu Val Gln Leu Val Glu Ser Gly Gly Gly Leu Val Gln Pro Gly Gly
   1 5 10 15
   Ser Leu Arg Leu Ser Cys Ala Ala Ser Gln Phe Thr Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ile Asn Ser Ala Thr His Tyr Ala Glu
   50 55 60
   Ser Val Lys Gly Arg Phe Ile Ile Ser Arg Asp Asp Asn Gly Asn Asp
   65 70 75 80
   Leu Tyr Leu Glu Met Asn Ser Leu Lys Thr Glu Asp Thr Ala Glu Tyr
   85 90 95
   Tyr Cys Ala Gln Asn Tyr Tyr Gly Ser Thr Tyr Asp Tyr Trp Gly Gln
   100 105 110
   Gly Thr Leu Val Thr Val Ser
   115
<210> 122
   <211> 119
   <212> PRT
   <213> Artificial Sequence
<220>
   <223> Infliximab VH variant
<400> 122
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr Ser Tyr Ala Glu
       50                  55                  60
   Ser Val Lys Gly Arg Phe Ile Ile Ser Arg Asp Gly Asn Gly Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 123
   <211> 119
   <212> PRT
   <213> Artificial Sequence
<220>
   <223> Infliximab VH variant
<400> 123
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Gly Ala Thr Ser Tyr Ala Glu
       50                  55                  60
   Ser Val Lys Gly Arg Phe Ile Ile Ser Arg Asp Asp Asn Gly Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 124
   <211> 119
   <212> PRT
   <213> Artificial Sequence
<220>
   <223> Infliximab VH variant
<400> 124
   Glu Val Gln Leu Val Glu Ser Gly Gly Gly Leu Val Gln Pro Gly Gly
   1               5                   10                  15
   Ser Leu Arg Leu Ser Cys Ala Ala Ser Gln Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Asn Ser Ala Thr His Tyr Ala Glu
       50                  55                  60
   Ser Val Lys Gly Arg Phe Ile Ile Ser Arg Asp Gly Asn Lys Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 125
   <211> 119
   <212> PRT
   <213> Artificial Sequence
<220>
   <223> Infliximab VH variant
<400> 125
   Glu Val Gln Leu Thr Glu Ser Gly Gly Gly Leu Val Gln Pro Gly Gly
   1               5                   10                  15
   Ser Leu Arg Leu Ser Cys Ala Ala Ser Gln Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr Ser Tyr Ala Glu
       50                  55                  60
   Ser Val Lys Gly Arg Phe Ile Ile Ser Arg Asp Asp Asn Gly Thr Ser
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 126
   <211> 119
   <212> PRT
   <213> Artificial Sequence
<220>
   <223> Infliximab VH variant
<400> 126
   Glu Val Gln Leu Val Glu Ser Gly Gly Gly Leu Val Gln Pro Gly Gly
    1               5                  10                  15
   Ser Leu Arg Leu Ser Cys Arg Ala Ser Gly Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr His Tyr Ala Glu
       50                  55                  60
   Ser Val Lys Gly Arg Phe Ile Ile Ser Arg Asp Gly Asn Lys Asn Ser
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 127
   <211> 119
   <212> PRT
   <213> Artificial Sequence
<220>
   <223> Infliximab VH variant
<400> 127
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gln Phe Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Tyr Gly Ala Thr Ser Tyr Ala Glu
       50                  55                  60
   Ser Val Lys Gly Arg Phe Ile Ile Ser Arg Asp Asp Asn Gly Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 128
   <211> 119
   <212> PRT
   <213> Artificial Sequence
<220>
   <223> Infliximab VH variant
<400> 128
   Glu Val Gln Leu Val Glu Ser Gly Gly Gly Leu Val Gln Pro Gly Gly
   1 5 10 15
   Ser Leu Arg Leu Ser Cys Arg Ala Ser Gln Ser Thr Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Asn Ser Ala Thr Ser Tyr Ala Glu
   50 55 60
   Ser Val Lys Gly Arg Phe Ile Ile Ser Arg Asp Asp Asn Gly Asn Asp
   65 70 75 80
   Leu Tyr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 129
   <211> 119
   <212> PRT
   <213> Artificial Sequence
<220>
   <223> Infliximab VH variant
<400> 129
   Glu Val Gln Leu Val Glu Ser Gly Gly Gly Leu Val Gln Pro Gly Gly
   1 5 10 15
   Ser Leu Arg Leu Ser Cys Ala Ala Ser Gln Ser Thr Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ser Tyr Gly Ala Thr His Tyr Ala Glu
   50 55 60
   Ser Val Lys Gly Arg Phe Ile Ile Ser Arg Asp Gly Asn Gly Thr Ser
   65 70 75 80
   Leu Thr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 130
   <211> 119
   <212> PRT
   <213> Artificial Sequence
<220>
   <223> Infliximab VH variant
<400> 130
   Glu Val Gln Leu Val Glu Ser Gly Gly Gly Leu Val Gln Pro Gly Gly
    1               5                  10                  15
   Ser Leu Arg Leu Ser Cys Arg Ala Ser Gly Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr His Tyr Ala Glu
       50                  55                  60
   Ser Val Lys Gly Arg Phe Ile Ile Ser Arg Asp Asp Asn Gly Asn Ser
   65                  70                  75                      80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 131
   <211> 119
   <212> PRT
   <213> Artificial Sequence
<220>
   <223> Infliximab VH variant
<400> 131
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gln Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Asn Ser Ala Thr Ser Tyr Ala Glu
       50                  55                  60
   Ser Val Lys Gly Arg Phe Ile Ile Ser Arg Asp Gly Asn Gly Thr Ser
   65                  70                  75                      80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 132
   <211> 119
   <212> PRT
   <213> Artificial Sequence
<220>
   <223> Infliximab VH variant
<400> 132
   Glu Val Gln Leu Val Glu Ser Gly Gly Gly Leu Val Gln Pro Gly Gly
    1               5                  10                  15
   Ser Leu Arg Leu Ser Cys Ala Ala Ser Gln Ser Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Gly Ala Thr Ser Tyr Ala Glu
       50                  55                  60
   Ser Val Lys Gly Arg Phe Ile Ile Ser Arg Asp Asp Asn Lys Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 133
   <211> 119
   <212> PRT
   <213> Artificial Sequence
<220>
   <223> Infliximab VH variant
<400> 133
   Glu Val Gln Leu Val Glu Ser Gly Gly Gly Leu Val Gln Pro Gly Gly
    1               5                  10                  15
   Ser Leu Arg Leu Ser Cys Ala Ala Ser Gln Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Gly Ala Thr His Tyr Ala Glu
       50                  55                  60
   Ser Val Lys Gly Arg Phe Ile Ile Ser Arg Asp Asp Asn Lys Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 134
   <211> 119
   <212> PRT
   <213> Artificial Sequence
<220>
   <223> Infliximab VH variant
<400> 134
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gly Ser Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr His Tyr Ala Glu
       50                  55                  60
   Ser Val Lys Gly Arg Phe Ile Ile Ser Arg Asp Gly Asn Gly Thr Ser
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 135
   <211> 119
   <212> PRT
   <213> Artificial Sequence
<220>
   <223> Infliximab VH variant
<400> 135
   Glu Val Gln Leu Val Glu Ser Gly Gly Gly Leu Val Gln Pro Gly Gly
    1               5                  10                  15
   Ser Leu Arg Leu Ser Cys Ala Ala Ser Gln Ser Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Asn Ser Ala Thr Ser Tyr Ala Glu
       50                  55                  60
   Ser Val Lys Gly Arg Phe Ile Ile Ser Arg Asp Gly Asn Lys Thr Asp
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 136
   <211> 119
   <212> PRT
   <213> Artificial Sequence
<220>
   <223> Infliximab VH variant
<400> 136
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gln Ser Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr His Tyr Ala Glu
       50                  55                  60
   Ser Val Lys Gly Arg Phe Ile Ile Ser Arg Asp Asp Asn Gly Thr Ser
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 137
   <211> 119
   <212> PRT
   <213> Artificial Sequence
<220>
   <223> Infliximab VH variant
<400> 137
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Ser Ala Thr Ser Tyr Ala Glu
       50                  55                  60
   Ser Val Lys Gly Arg Phe Ile Ile Ser Arg Asp Gly Asn Gly Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 138
   <211> 119
   <212> PRT
   <213> Artificial Sequence
<220>
   <223> Infliximab VH variant
<400> 138
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gly Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Asn Ser Ala Thr Ser Tyr Ala Glu
       50                  55                  60
   Ser Val Lys Gly Arg Phe Ile Ile Ser Arg Asp Gly Asn Lys Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 139
   <211> 119
   <212> PRT
   <213> Artificial Sequence
<220>
   <223> Infliximab VH variant
<400> 139
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Ser Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Gly Ala Thr Ser Tyr Ala Glu
       50                  55                  60
   Ser Val Lys Gly Arg Phe Ile Ile Ser Arg Asp Asp Asn Lys Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 140
   <211> 119
   <212> PRT
   <213> Artificial Sequence
<220>
   <223> Infliximab VH variant
<400> 140
   Glu Val Gln Leu Thr Glu Ser Gly Gly Gly Leu Val Gln Pro Gly Gly
   1 5 10 15
   Ser Leu Arg Leu Ser Cys Ala Ala Ser Gly Ser Thr Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Asn Ser Ala Thr His Tyr Ala Glu
   50 55 60
   Ser Val Lys Gly Arg Phe Ile Ile Ser Arg Asp Asp Asn Gly Thr Ser
   65 70 75 80
   Leu Tyr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 141
   <211> 119
   <212> PRT
   <213> Artificial Sequence
<220>
   <223> Infliximab VH variant
<400> 141
   Glu Val Gln Leu Val Glu Ser Gly Gly Gly Leu Val Gln Pro Gly Gly
   1 5 10 15
   Ser Leu Arg Leu Ser Cys Ala Ala Ser Gly Ser Thr Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Tyr Gly Ala Thr Ser Tyr Ala Glu
   50 55 60
   Ser Val Lys Gly Arg Phe Ile Ile Ser Arg Asp Gly Asn Lys Asn Ser
   65 70 75 80
   Leu Tyr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 142
   <211> 119
   <212> PRT
   <213> Artificial Sequence
<220>
   <223> Infliximab VH variant
<400> 142
   Glu Val Gln Leu Val Glu Ser Gly Gly Gly Leu Val Gln Pro Gly Gly
   1 5 10 15
   Ser Leu Arg Leu Ser Cys Arg Ala Ser Gln Phe Thr Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ile Tyr Ser Ala Thr Ser Tyr Ala Glu
   50 55 60
   Ser Val Lys Gly Arg Phe Ile Ile Ser Arg Asp Asp Asn Gly Thr Ser
   65 70 75 80
   Leu Thr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 143
   <211> 119
   <212> PRT
   <213> Artificial Sequence
<220>
   <223> Infliximab VH variant
<400> 143
   Glu Val Gln Leu Val Glu Ser Gly Gly Gly Leu Val Gln Pro Gly Gly
   1 5 10 15
   Ser Leu Arg Leu Ser Cys Ala Ala Ser Gln Ser Ile Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Asn Ser Ala Thr Ser Tyr Ala Glu
   50 55 60
   Ser Val Lys Gly Arg Phe Ile Ile Ser Arg Asp Gly Asn Gly Asn Asp
   65 70 75 80
   Leu Tyr Leu Glu Met Asn Ser Leu Lys Thr Glu Asp Thr Ala Glu Tyr
   85 90 95
   Tyr Cys Ala Gln Asn Tyr Tyr Gly Ser Thr Tyr Asp Tyr Trp Gly Gln
   100 105 110
   Gly Thr Leu Val Thr Val Ser
   115
<210> 144
   <211> 119
   <212> PRT
   <213> Artificial Sequence
<220>
   <223> Infliximab VH variant
<400> 144
   Glu Val Gln Leu Val Glu Ser Gly Gly Gly Leu Val Gln Pro Gly Gly
   1 5 10 15
   Ser Leu Arg Leu Ser Cys Arg Ala Ser Gly Ser Ile Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Asn Ser Ala Thr Ser Tyr Ala Glu
   50 55 60
   Ser Val Lys Gly Arg Phe Ile Ile Ser Arg Asp Gly Asn Lys Asn Asp
   65 70 75 80
   Leu Tyr Leu Glu Met Asn Ser Leu Lys Thr Glu Asp Thr Ala Glu Tyr
   85 90 95
   Tyr Cys Ala Gln Asn Tyr Tyr Gly Ser Thr Tyr Asp Tyr Trp Gly Gln
   100 105 110
   Gly Thr Leu Val Thr Val Ser
   115
<210> 145
   <211> 119
   <212> PRT
   <213> Artificial Sequence
<220>
   <223> Infliximab VH variant
<400> 145
   Glu Val Gln Leu Thr Glu Ser Gly Gly Gly Leu Val Gln Pro Gly Gly
   1 5 10 15
   Ser Leu Arg Leu Ser Cys Arg Ala Ser Gln Ser Thr Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Asn Gly Ala Thr Ser Tyr Ala Glu
   50 55 60
   Ser Val Lys Gly Arg Phe Ile Ile Ser Arg Asp Asp Asn Gly Asn Asp
   65 70 75 80
   Leu Tyr Leu Glu Met Asn Ser Leu Lys Thr Glu Asp Thr Ala Glu Tyr
   85 90 95
   Tyr Cys Ala Gln Asn Tyr Tyr Gly Ser Thr Tyr Asp Tyr Trp Gly Gln
   100 105 110
   Gly Thr Leu Val Thr Val Ser
   115
<210> 146
   <211> 119
   <212> PRT
   <213> Artificial Sequence
<220>
   <223> Infliximab VH variant
<400> 146
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr His Tyr Ala Glu
       50                  55                  60
   Ser Val Lys Gly Arg Phe Ile Ile Ser Arg Asp Gly Asn Lys Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 147
   <211> 119
   <212> PRT
   <213> Artificial Sequence
<220>
   <223> Infliximab VH variant
<400> 147
   Glu Val Gln Leu Val Glu Ser Gly Gly Gly Leu Val Gln Pro Gly Gly
   1               5                   10                  15
   Ser Leu Arg Leu Ser Cys Ala Ala Ser Gln Ser Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr His Tyr Ala Glu
       50                  55                  60
   Ser Val Lys Gly Arg Phe Ile Ile Ser Arg Asp Gly Asn Gly Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 148
   <211> 119
   <212> PRT
   <213> Artificial Sequence
<220>
   <223> Infliximab VH variant
<400> 148
   Glu Val Gln Leu Val Glu Ser Gly Gly Gly Leu Val Gln Pro Gly Gly
    1               5                  10                  15
   Ser Leu Arg Leu Ser Cys Arg Ala Ser Gln Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Gly Ala Thr Ser Tyr Ala Glu
       50                  55                  60
   Ser Val Lys Gly Arg Phe Ile Ile Ser Arg Asp Gly Asn Gly Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 149
   <211> 119
   <212> PRT
   <213> Artificial Sequence
<220>
   <223> Infliximab VH variant
<400> 149
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Ser Ala Thr His Tyr Ala Glu
       50                  55                  60
   Ser Val Lys Gly Arg Phe Ile Ile Ser Arg Asp Asp Asn Gly Asn Ser
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 150
   <211> 119
   <212> PRT
   <213> Artificial Sequence
<220>
   <223> Infliximab VH variant
<400> 150
   Glu Val Gln Leu Val Glu Ser Gly Gly Gly Leu Val Gln Pro Gly Gly
   1               5                   10                  15
   Ser Leu Arg Leu Ser Cys Ala Ala Ser Gln Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr Ser Tyr Ala Glu
       50                  55                  60
   Ser Val Lys Gly Arg Phe Ile Ile Ser Arg Asp Asp Asn Gly Thr Asp
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 151
   <211> 119
   <212> PRT
   <213> Artificial Sequence
<220>
   <223> Infliximab VH variant
<400> 151
   Glu Val Gln Leu Val Glu Ser Gly Gly Gly Leu Val Gln Pro Gly Gly
   1               5                   10                  15
   Ser Leu Arg Leu Ser Cys Ala Ala Ser Gln Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Gly Ala Thr His Tyr Ala Glu
       50                  55                  60
   Ser Val Lys Gly Arg Phe Ile Ile Ser Arg Asp Gly Asn Gly Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 152
   <211> 119
   <212> PRT
   <213> Artificial Sequence
<220>
   <223> Infliximab VH variant
<400> 152
   Glu Val Gln Leu Val Glu Ser Gly Gly Gly Leu Val Gln Pro Gly Gly
    1               5                  10                  15
   Ser Leu Arg Leu Ser Cys Ala Ala Ser Gln Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr His Tyr Ala Glu
       50                  55                  60
   Ser Val Lys Gly Arg Phe Ile Ile Ser Arg Asp Asp Asn Gly Thr Ser
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 153
   <211> 119
   <212> PRT
   <213> Artificial Sequence
<220>
   <223> Infliximab VH variant
<400> 153
   Glu Val Gln Leu Val Glu Ser Gly Gly Gly Leu Val Gln Pro Gly Gly
    1               5                  10                  15
   Ser Leu Arg Leu Ser Cys Arg Ala Ser Gln Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Asn Gly Ala Thr Ser Tyr Ala Glu
       50                  55                  60
   Ser Val Lys Gly Arg Phe Ile Ile Ser Arg Asp Gly Asn Gly Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 154
   <211> 119
   <212> PRT
   <213> Artificial Sequence
<220>
   <223> Infliximab VH variant
<400> 154
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Asn Gly Ala Thr Ser Tyr Ala Glu
       50                  55                  60
   Ser Val Lys Gly Arg Phe Ile Ile Ser Arg Asp Asp Asn Gly Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 155
   <211> 119
   <212> PRT
   <213> Artificial Sequence
<220>
   <223> Infliximab VH variant
<400> 155
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gln Ser Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Tyr Ser Ala Thr Ser Tyr Ala Glu
       50                  55                  60
   Ser Val Lys Gly Arg Phe Ile Ile Ser Arg Asp Asp Asn Lys Asn Ser
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 156
   <211> 119
   <212> PRT
   <213> Artificial Sequence
<220>
   <223> Infliximab VH variant
<400> 156
   Glu Val Gln Leu Thr Glu Ser Gly Gly Gly Leu Val Gln Pro Gly Gly
   1 5 10 15
   Ser Leu Arg Leu Ser Cys Ala Ala Ser Gln Ser Thr Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Asn Ser Ala Thr Ser Tyr Ala Glu
   50 55 60
   Ser Val Lys Gly Arg Phe Ile Ile Ser Arg Asp Asp Asn Lys Asn Asp
   65 70 75 80
   Leu Thr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 157
   <211> 119
   <212> PRT
   <213> Artificial Sequence
<220>
   <223> Infliximab VH variant
<400> 157
   Glu Val Gln Leu Thr Glu Ser Gly Gly Gly Leu Val Gln Pro Gly Gly
   1 5 10 15
   Ser Leu Arg Leu Ser Cys Arg Ala Ser Gly Ser Ile Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Tyr Ser Ala Thr Ser Tyr Ala Glu
   50 55 60
   Ser Val Lys Gly Arg Phe Ile Ile Ser Arg Asp Asp Asn Gly Thr Ser
   65 70 75 80
   Leu Thr Leu Glu Met Asn Ser Leu Lys Thr Glu Asp Thr Ala Glu Tyr
   85 90 95
   Tyr Cys Ala Gln Asn Tyr Tyr Gly Ser Thr Pro Asp Tyr Trp Gly Gln
   100 105 110
   Gly Thr Leu Val Thr Val Ser
   115
<210> 158
   <211> 119
   <212> PRT
   <213> Artificial Sequence
<220>
   <223> Infliximab VH variant
<400> 158
   Glu Val Gln Leu Val Glu Ser Gly Gly Gly Leu Val Gln Pro Gly Gly
    1               5                  10                  15
   Ser Leu Arg Leu Ser Cys Arg Ala Ser Gln Phe Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Tyr Gly Ala Thr His Tyr Ala Glu
       50                  55                  60
   Ser Val Lys Gly Arg Phe Ile Ile Ser Arg Asp Asp Asn Lys Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 159
   <211> 119
   <212> PRT
   <213> Artificial Sequence
<220>
   <223> Infliximab VH variant
<400> 159
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr Ser Tyr Ala Glu
       50                  55                  60
   Ser Val Lys Gly Arg Phe Ile Ile Ser Arg Asp Gly Asn Gly Thr Ser
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 160
   <211> 119
   <212> PRT
   <213> Artificial Sequence
<220>
   <223> Infliximab VH variant
<400> 160
   Glu Val Gln Leu Val Glu Ser Gly Gly Gly Leu Val Gln Pro Gly Gly
    1               5                  10                  15
   Ser Leu Arg Leu Ser Cys Arg Ala Ser Gln Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Ser Ala Thr Ser Tyr Ala Glu
       50                  55                  60
   Ser Val Lys Gly Arg Phe Ile Ile Ser Arg Asp Gly Asn Lys Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 161
   <211> 119
   <212> PRT
   <213> Artificial Sequence
<220>
   <223> Infliximab VH variant
<400> 161
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gly Phe Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr His Tyr Ala Glu
       50                  55                  60
   Ser Val Lys Gly Arg Phe Ile Ile Ser Arg Asp Asp Asn Gly Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 162
   <211> 119
   <212> PRT
   <213> Artificial Sequence
<220>
   <223> Infliximab VH variant
<400> 162
   Glu Val Gln Leu Val Glu Ser Gly Gly Gly Leu Val Gln Pro Gly Gly
    1               5                  10                  15
   Ser Leu Arg Leu Ser Cys Ala Ala Ser Gln Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Ser Ala Thr His Tyr Ala Glu
       50                  55                  60
   Ser Val Lys Gly Arg Phe Ile Ile Ser Arg Asp Gly Asn Lys Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 163
   <211> 119
   <212> PRT
   <213> Artificial Sequence
<220>
   <223> Infliximab VH variant
<400> 163
   Glu Val Gln Leu Val Glu Ser Gly Gly Gly Leu Val Gln Pro Gly Gly
    1               5                  10                  15
   Ser Leu Arg Leu Ser Cys Ala Ala Ser Gln Phe Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr His Tyr Ala Glu
       50                  55                  60
   Ser Val Lys Gly Arg Phe Ile Ile Ser Arg Asp Asp Asn Lys Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 164
   <211> 119
   <212> PRT
   <213> Artificial Sequence
<220>
   <223> Infliximab VH variant
<400> 164
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gly Ser Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr His Tyr Ala Glu
       50                  55                  60
   Ser Val Lys Gly Arg Phe Ile Ile Ser Arg Asp Gly Asn Gly Thr Asp
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 165
   <211> 119
   <212> PRT
   <213> Artificial Sequence
<220>
   <223> Infliximab VH variant
<400> 165
   Glu Val Gln Leu Val Glu Ser Gly Gly Gly Leu Val Gln Pro Gly Gly
    1               5                  10                  15
   Ser Leu Arg Leu Ser Cys Ala Ala Ser Gln Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Gly Ala Thr His Tyr Ala Glu
       50                  55                  60
   Ser Val Lys Gly Arg Phe Ile Ile Ser Arg Asp Gly Asn Lys Thr Asp
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 166
   <211> 119
   <212> PRT
   <213> Artificial Sequence
<220>
   <223> Infliximab VH variant
<400> 166
   Glu Val Gln Leu Val Glu Ser Gly Gly Gly Leu Val Gln Pro Gly Gly
    1               5                  10                  15
   Ser Leu Arg Leu Ser Cys Ala Ala Ser Gln Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr Ser Tyr Ala Glu
       50                  55                  60
   Ser Val Lys Gly Arg Phe Ile Ile Ser Arg Asp Gly Asn Lys Thr Asp
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 167
   <211> 119
   <212> PRT
   <213> Artificial Sequence
<220>
   <223> Infliximab VH variant
<400> 167
   Glu Val Gln Leu Val Glu Ser Gly Gly Gly Leu Val Gln Pro Gly Gly
    1               5                  10                  15
   Ser Leu Arg Leu Ser Cys Ala Ala Ser Gln Phe Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Tyr Gly Ala Thr His Tyr Ala Glu
       50                  55                  60
   Ser Val Lys Gly Arg Phe Ile Ile Ser Arg Asp Asp Asn Lys Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 168
   <211> 119
   <212> PRT
   <213> Artificial Sequence
<220>
   <223> Infliximab VH variant
<400> 168
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gln Ser Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr Ser Tyr Ala Glu
       50                  55                  60
   Ser Val Lys Gly Arg Phe Ile Ile Ser Arg Asp Gly Asn Lys Thr Ser
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 169
   <211> 119
   <212> PRT
   <213> Artificial Sequence
<220>
   <223> Infliximab VH variant
<400> 169
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gly Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr Ser Tyr Ala Glu
       50                  55                  60
   Ser Val Lys Gly Arg Phe Ile Ile Ser Arg Asp Asp Asn Gly Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 170
   <211> 119
   <212> PRT
   <213> Artificial Sequence
<220>
   <223> Infliximab VH variant
<400> 170
   Glu Val Gln Leu Val Glu Ser Gly Gly Gly Leu Val Gln Pro Gly Gly
   1 5 10 15
   Ser Leu Arg Leu Ser Cys Ala Ala Ser Gly Ser Thr Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Asn Ser Ala Thr His Tyr Ala Glu
   50 55 60
   Ser Val Lys Gly Arg Phe Ile Ile Ser Arg Asp Asp Asn Gly Thr Ser
   65 70 75 80
   Leu Tyr Leu Glu Met Asn Ser Leu Lys Thr Glu Asp Thr Ala Glu Tyr
   85 90 95
   Tyr Cys Ala Gln Asn Tyr Tyr Gly Ser Thr Tyr Asp Tyr Trp Gly Gln
   100 105 110
   Gly Thr Leu Val Thr Val Ser
   115
<210> 171
   <211> 119
   <212> PRT
   <213> Artificial Sequence
<220>
   <223> Infliximab VH variant
<400> 171
   Glu Val Gln Leu Val Glu Ser Gly Gly Gly Leu Val Gln Pro Gly Gly
   1 5 10 15
   Ser Leu Arg Leu Ser Cys Ala Ala Ser Gln Ser Thr Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ile Tyr Ser Ala Thr Ser Tyr Ala Glu
   50 55 60
   Ser Val Lys Gly Arg Phe Ile Ile Ser Arg Asp Asp Asn Gly Asn Ser
   65 70 75 80
   Leu Tyr Leu Glu Met Asn Ser Leu Lys Thr Glu Asp Thr Ala Glu Tyr
   85 90 95
   Tyr Cys Ala Gln Asn Tyr Tyr Gly Ser Thr Pro Asp Tyr Trp Gly Gln
   100 105 110
   Gly Thr Leu Val Thr Val Ser
   115
<210> 172
   <211> 119
   <212> PRT
   <213> Artificial Sequence
<220>
   <223> Infliximab VH variant
<400> 172
   Glu Val Gln Leu Thr Glu Ser Gly Gly Gly Leu Val Gln Pro Gly Gly
   1 5 10 15
   Ser Leu Arg Leu Ser Cys Ala Ala Ser Gly Phe Thr Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Tyr Gly Ala Thr His Tyr Ala Glu
   50 55 60
   Ser Val Lys Gly Arg Phe Ile Ile Ser Arg Asp Gly Asn Lys Asn Asp
   65 70 75 80
   Leu Thr Leu Glu Met Asn Ser Leu Lys Thr Glu Asp Thr Ala Glu Tyr
   85 90 95
   Tyr Cys Ala Gln Asn Tyr Tyr Gly Ser Thr Pro Asp Tyr Trp Gly Gln
   100 105 110
   Gly Thr Leu Val Thr Val Ser
   115
<210> 173
   <211> 119
   <212> PRT
   <213> Artificial Sequence
<220>
   <223> Infliximab VH variant
<400> 173
   Glu Val Gln Leu Thr Glu Ser Gly Gly Gly Leu Val Gln Pro Gly Gly
   1 5 10 15
   Ser Leu Arg Leu Ser Cys Arg Ala Ser Gln Ser Thr Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ser Asn Ser Ala Thr Ser Tyr Ala Glu
   50 55 60
   Ser Val Lys Gly Arg Phe Ile Ile Ser Arg Asp Gly Asn Lys Thr Asp
   65 70 75 80
   Leu Thr Leu Glu Met Asn Ser Leu Lys Thr Glu Asp Thr Ala Glu Tyr
   85 90 95
   Tyr Cys Ala Arg Asn Tyr Tyr Gly Ser Thr Pro Asp Tyr Trp Gly Gln
   100 105 110
   Gly Thr Leu Val Thr Val Ser
   115
<210> 174
   <211> 119
   <212> PRT
   <213> Artificial Sequence
<220>
   <223> Infliximab VH variant
<400> 174
   Glu Val Gln Leu Val Glu Ser Gly Gly Gly Leu Val Gln Pro Gly Gly
   1               5                   10                  15
   Ser Leu Arg Leu Ser Cys Arg Ala Ser Gly Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Ser Ala Thr Ser Tyr Ala Glu
       50                  55                  60
   Ser Val Lys Gly Arg Phe Ile Ile Ser Arg Asp Asp Asn Lys Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 175
   <211> 119
   <212> PRT
   <213> Artificial Sequence
<220>
   <223> Infliximab VH variant
<400> 175
   Glu Val Gln Leu Val Glu Ser Gly Gly Gly Leu Val Gln Pro Gly Gly
   1               5                   10                  15
   Ser Leu Arg Leu Ser Cys Arg Ala Ser Gly Phe Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Asn Gly Ala Thr His Tyr Ala Glu
       50                  55                  60
   Ser Val Lys Gly Arg Phe Ile Ile Ser Arg Asp Asp Asn Lys Thr Ser
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 176
   <211> 119
   <212> PRT
   <213> Artificial Sequence
<220>
   <223> Infliximab VH variant
<400> 176
   Glu Val Gln Leu Thr Glu Ser Gly Gly Gly Leu Val Gln Pro Gly Gly
    1               5                  10                  15
   Ser Leu Arg Leu Ser Cys Arg Ala Ser Gln Ser Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr His Tyr Ala Glu
       50                  55                  60
   Ser Val Lys Gly Arg Phe Ile Ile Ser Arg Asp Gly Asn Gly Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 177
   <211> 119
   <212> PRT
   <213> Artificial Sequence
<220>
   <223> Infliximab VH variant
<400> 177
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gln Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Asn Gly Ala Thr Ser Tyr Ala Glu
       50                  55                  60
   Ser Val Lys Gly Arg Phe Ile Ile Ser Arg Asp Asp Asn Lys Asn Asp
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 178
   <211> 119
   <212> PRT
   <213> Artificial Sequence
<220>
   <223> Infliximab VH variant
<400> 178
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr His Tyr Ala Glu
       50                  55                  60
   Ser Val Lys Gly Arg Phe Ile Ile Ser Arg Asp Gly Asn Gly Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 179
   <211> 119
   <212> PRT
   <213> Artificial Sequence
<220>
   <223> Infliximab VH variant
<400> 179
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gln Phe Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Asn Gly Ala Thr His Tyr Ala Glu
       50                  55                  60
   Ser Val Lys Gly Arg Phe Ile Ile Ser Arg Asp Asp Asn Gly Thr Ser
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 180
   <211> 119
   <212> PRT
   <213> Artificial Sequence
<220>
   <223> Infliximab VH variant
<400> 180
   Glu Val Gln Leu Val Glu Ser Gly Gly Gly Leu Val Gln Pro Gly Gly
    1               5                  10                  15
   Ser Leu Arg Leu Ser Cys Arg Ala Ser Gly Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Asn Ser Ala Thr His Tyr Ala Glu
       50                  55                  60
   Ser Val Lys Gly Arg Phe Ile Ile Ser Arg Asp Asp Asn Lys Thr Ser
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 181
   <211> 119
   <212> PRT
   <213> Artificial Sequence
<220>
   <223> Infliximab VH variant
<400> 181
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ser Tyr Ser Ala Thr His Tyr Ala Glu
       50                  55                  60
   Ser Val Lys Gly Arg Phe Ile Ile Ser Arg Asp Asp Asn Lys Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 182
   <211> 119
   <212> PRT
   <213> Artificial Sequence
<220>
   <223> Infliximab VH variant
<400> 182
   Glu Val Gln Leu Thr Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr His Tyr Ala Glu
       50                  55                  60
   Ser Val Lys Gly Arg Phe Ile Ile Ser Arg Asp Gly Asn Gly Thr Asp
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Pro Asp Tyr Trp Gly Gln
               100                 105                 110
   Gly Thr Leu Val Thr Val Ser
           115
<210> 183
   <211> 119
   <212> PRT
   <213> Artificial Sequence
<220>
   <223> Infliximab VH variant
<400> 183
   Glu Val Gln Leu Thr Glu Ser Gly Gly Gly Leu Val Gln Pro Gly Gly
   1               5                   10                  15
   Ser Leu Arg Leu Ser Cys Ala Ala Ser Gln Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Gly Ala Thr Ser Tyr Ala Glu
       50                  55                  60
   Ser Val Lys Gly Arg Phe Ile Ile Ser Arg Asp Gly Asn Gly Thr Ser
   65                  70                  75                  80
   Leu Tyr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 184
   <211> 119
   <212> PRT
   <213> Artificial Sequence
<220>
   <223> Infliximab VH variant
<400> 184
   Glu Val Gln Leu Val Glu Ser Gly Gly Gly Leu Val Gln Pro Gly Gly
   1 5 10 15
   Ser Leu Arg Leu Ser Cys Ala Ala Ser Gln Phe Ile Phe Ser Asn His
   20 25 30
   Tyr Met Asn Trp Val Arg Gln Ala Pro Gly Lys Gly Leu Glu Trp Ile
   35 40 45
   Gly Glu Ile Arg Ser Lys Ser Ile Asn Ser Ala Thr His Tyr Ala Glu
   50 55 60
   Ser Val Lys Gly Arg Phe Ile Ile Ser Arg Asp Gly Asn Gly Thr Asp
   65 70 75 80
   Leu Tyr Leu Glu Met Asn Ser Leu Lys Thr Glu Asp Thr Ala Glu Tyr
   85 90 95
   Tyr Cys Ala Arg Asn Tyr Tyr Gly Ser Thr Tyr Asp Tyr Trp Gly Gln
   100 105 110
   Gly Thr Leu Val Thr Val Ser
   115
<210> 185
   <211> 119
   <212> PRT
   <213> Artificial Sequence
<220>
   <223> Infliximab VH variant
<400> 185
   Glu Val Gln Leu Thr Glu Ser Gly Gly Gly Leu Val Gln Pro Gly Gly
   1 5 10 15
   Ser Leu Arg Leu Ser Cys Arg Ala Ser Gln Ser Thr Phe Ser Asn His
   20 25 30
   Trp Met Asn Trp Val Arg Gln Ala Pro Gly Lys Gly Leu Glu Trp Ile
   35 40 45
   Gly Glu Ile Arg Ser Lys Ser Ile Tyr Ser Ala Thr Ser Tyr Ala Glu
   50 55 60
   Ser Val Lys Gly Arg Phe Ile Ile Ser Arg Asp Gly Asn Gly Asn Asp
   65 70 75 80
   Leu Tyr Leu Glu Met Asn Ser Leu Lys Thr Glu Asp Thr Ala Glu Tyr
   85 90 95
   Tyr Cys Ala Gln Asn Tyr Tyr Gly Ser Thr Pro Asp Tyr Trp Gly Gln
   100 105 110
   Gly Thr Leu Val Thr Val Ser
   115
<210> 186
   <211> 119
   <212> PRT
   <213> Artificial Sequence
<220>
   <223> Infliximab VH variant
<400> 186
   Glu Val Gln Leu Thr Glu Ser Gly Gly Gly Leu Val Gln Pro Gly Gly
   1               5                   10                  15
   Ser Leu Arg Leu Ser Cys Ala Ala Ser Gly Ser Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr Ser Tyr Ala Glu
       50                  55                  60
   Ser Val Lys Gly Arg Phe Ile Ile Ser Arg Asp Asp Asn Gly Asn Ser
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 187
   <211> 119
   <212> PRT
   <213> Artificial Sequence
<220>
   <223> Infliximab VH variant
<400> 187
   Glu Val Gln Leu Thr Glu Ser Gly Gly Gly Leu Val Gln Pro Gly Gly
   1               5                   10                  15
   Ser Leu Arg Leu Ser Cys Ala Ala Ser Gly Phe Thr Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ser Tyr Gly Ala Thr Ser Tyr Ala Glu
       50                  55                  60
   Ser Val Lys Gly Arg Phe Ile Ile Ser Arg Asp Gly Asn Gly Thr Asp
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 188
   <211> 119
   <212> PRT
   <213> Artificial Sequence
<220>
   <223> Infliximab VH variant
<400> 188
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Phe Ile Phe Ser Asn His
               20                  25                  30
   Tyr Met Asn Trp Val Arg Gln Ala Pro Gly Lys Gly Leu Glu Trp Ile
           35                  40                  45
   Gly Glu Ile Arg Ser Lys Ser Ile Tyr Gly Ala Thr Ser Tyr Ala Glu
       50                  55                  60
   Ser Val Lys Gly Arg Phe Ile Ile Ser Arg Asp Asp Asn Lys Asn Asp
   65                  70                  75                  80
   Leu Tyr Leu Glu Met Asn Ser Leu Lys Thr Glu Asp Thr Ala Glu Tyr
                   85                  90                  95
   Tyr Cys Ala Gln Asn Tyr Tyr Gly Ser Thr Tyr Asp Tyr Trp Gly Gln
               100                 105                 110
   Gly Thr Leu Val Thr Val Ser
           115
<210> 189
   <211> 119
   <212> PRT
   <213> Artificial Sequence
<220>
   <223> Infliximab VH variant
<400> 189
   Glu Val Gln Leu Thr Glu Ser Gly Gly Gly Leu Val Gln Pro Gly Gly
    1               5                  10                  15
   Ser Leu Arg Leu Ser Cys Ala Ala Ser Gln Phe Thr Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Tyr Ser Ala Thr His Tyr Ala Glu
       50                  55                  60
   Ser Val Lys Gly Arg Phe Ile Ile Ser Arg Asp Asp Asn Gly Thr Ser
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Pro Asp Tyr Trp Gly Gln
               100                 105                 110
   Gly Thr Leu Val Thr Val Ser
           115
<210> 190
   <211> 119
   <212> PRT
   <213> Artificial Sequence
<220>
   <223> Infliximab VH variant
<400> 190
   Glu Val Gln Leu Val Glu Ser Gly Gly Gly Leu Val Gln Pro Gly Gly
   1               5                   10                  15
   Ser Leu Arg Leu Ser Cys Arg Ala Ser Gly Phe Ile Phe Ser Asn His
               20                  25                  30
   Trp Met Asn Trp Val Arg Gln Ala Pro Gly Lys Gly Leu Glu Trp Ile
           35                  40                  45
   Gly Glu Ile Arg Ser Lys Ser Ile Asn Ser Ala Thr Ser Tyr Ala Glu
       50                  55                  60
   Ser Val Lys Gly Arg Phe Ile Ile Ser Arg Asp Gly Asn Lys Thr Asp
   65                  70                  75                  80
   Leu Thr Leu Glu Met Asn Ser Leu Lys Thr Glu Asp Thr Ala Glu Tyr
                   85                  90                  95
   Tyr Cys Ala Arg Asn Tyr Tyr Gly Ser Thr Tyr Asp Tyr Trp Gly Gln
               100                 105                 110
   Gly Thr Leu Val Thr Val Ser
           115
<210> 191
   <211> 357
   <212> DNA
   <213> Artificial Sequence
<220>
   <223> Infliximab VH variant
<400> 191
   gaggtgcagc tggtggagag cggaggagga ctggtgcagc ctggaggaag cctgagactg 60
   agctgcgccg ccagcggctt catcttcagc aaccactgga tgaactgggt gagacaggct 120
   cctggcaagg gcctggagtg gatcggcgag atccggagca agagcatcaa cagcgccacc 180
   cactacgccg agagcgtgaa gggccggttc atcatcagcc gggacgacaa caagaacagc 240
   ctgtacctgg agatgaacag cctgaagacc gaggacaccg ccgagtacta ctgcgcccgg 300
   aactactacg gcagcaccta cgactactgg ggccagggaa ccctggtgac cgtgagc    357
<210> 192
   <211> 357
   <212> DNA
   <213> Artificial Sequence
<220>
   <223> Infliximab VH variant
<400> 192
   gaagttcagc tgaccgaatc tggtggtggt ctggttcagc cgggtggttc tctgcgtctg 60
   tcttgccgtg cgtctggttt catcttctct aaccactgga tgaactgggt tcgtcaggcg 120
   ccgggtaaag gtctggaatg gatcggtgaa atccgttcta aatcttctta ctctgcgacc 180
   tcttacgcgg aatctgttaa aggtcgtttc atcatctctc gtgacgacaa caaaacctct 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 193
   <211> 357
   <212> DNA
   <213> Artificial Sequence
<220>
   <223> Infliximab VH variant
<400> 193
   gaagttcagc tgaccgaatc tggtggtggt ctggttcagc cgggtggttc tctgcgtctg 60
   tcttgcgcgg cgtctcagtt caccttctct aaccactgga tgaactgggt tcgtcaggcg 120
   ccgggtaaag gtctggaatg gatcggtgaa atccgttcta aatctatcaa ctctgcgacc 180
   cactacgcgg aatctgttaa aggtcgtttc atcatctctc gtgacgacaa caaaaactct 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 194
   <211> 357
   <212> DNA
   <213> Artificial Sequence
<220>
   <223> Infliximab VH variant
<400> 194
   gaagttcagt tggttgaatc tggtggtggt ctggttcagc cgggtggttc tctgcgtctg 60
   tcttgcgcgg cgtctcagtt caccttctct aaccactaca tgaactgggt tcgtcaggcg 120
   ccgggtaaag gtctggaatg gatcggtgaa atccgttcta aatcttctta ctctgcgacc 180
   cactacgcgg aatctgttaa aggtcgtttc atcatctctc gtgacggtaa caaaaccgac 240
   ctgtacctgg aaatgaactc tctgaaaacc gaagacaccg cggaatacta ctgcgcgcgt 300
   aactactacg gttctacccc ggactactgg ggtcagggta ccctggttac cgtttct    357
<210> 195
   <211> 357
   <212> DNA
   <213> Artificial Sequence
<220>
   <223> Infliximab VH variant
<400> 195
   gaagttcagc tgaccgaatc tggtggtggt ctggttcagc cgggtggttc tctgcgtctg 60
   tcttgcgcgg cgtctcagtt caccttctct aaccactgga tgaactgggt tcgtcaggcg 120
   ccgggtaaag gtctggaatg gatcggtgaa atccgttcta aatcttctaa ctctgcgacc 180
   cactacgcgg aatctgttaa aggtcgtttc atcatctctc gtgacgacaa caaaaccgac 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 196
   <211> 357
   <212> DNA
   <213> Artificial Sequence
<220>
   <223> Infliximab VH variant
<400> 196
   gaagttcagc tgaccgaatc tggtggtggt ctggttcagc cgggtggttc tctgcgtctg 60
   tcttgccgtg cgtctggttc taccttctct aaccactaca tgaactgggt tcgtcaggcg 120
   ccgggtaaag gtctggaatg gatcggtgaa atccgttcta aatctatcaa cggtgcgacc 180
   cactacgcgg aatctgttaa aggtcgtttc atcatctctc gtgacggtaa cggtaacgac 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 197
   <211> 357
   <212> DNA
   <213> Artificial Sequence
<220>
   <223> Infliximab VH variant
<400> 197
   gaagttcagc tgaccgaatc tggtggtggt ctggttcagc cgggtggttc tctgcgtctg 60
   tcttgccgtg cgtctcagtc tatcttctct aaccactaca tgaactgggt tcgtcaggcg 120
   ccgggtaaag gtctggaatg gatcggtgaa atccgttcta aatctatcaa ctctgcgacc 180
   tcttacgcgg aatctgttaa aggtcgtttc atcatctctc gtgacgacaa cggtaacgac 240
   ctgaccctgg aaatgaactc tctgaaaacc gaagacaccg cggaatacta ctgcgcgcag 300
   aactactacg gttctaccta cgactactgg ggtcagggta ccctggttac cgtttct    357
<210> 198
   <211> 357
   <212> DNA
   <213> Artificial Sequence
<220>
   <223> Infliximab VH variant
<400> 198
   gaagttcagc tgaccgaatc tggtggtggt ctggttcagc cgggtggttc tctgcgtctg 60
   tcttgccgtg cgtctggttt catcttctct aaccactaca tgaactgggt tcgtcaggcg 120
   ccgggtaaag gtctggaatg gatcggtgaa atccgttcta aatctatcaa ctctgcgacc 180
   tcttacgcgg aatctgttaa aggtcgtttc atcatctctc gtgacgacaa caaaaacgac 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 199
   <211> 357
   <212> DNA
   <213> Artificial Sequence
<220>
   <223> Infliximab VH variant
<400> 199
   gaagttcagc tgaccgaatc tggtggtggt ctggttcagc cgggtggttc tctgcgtctg 60
   tcttgcgcgg cgtctggttt catcttctct aaccactgga tgaactgggt tcgtcaggcg 120
   ccgggtaaag gtctggaatg gatcggtgaa atccgttcta aatctatcaa cggtgcgacc 180
   tcttacgcgg aatctgttaa aggtcgtttc atcatctctc gtgacgacaa caaaacctct 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 200
   <211> 357
   <212> DNA
   <213> Artificial Sequence
<220>
   <223> Infliximab VH variant
<400> 200
   gaagttcagc tgaccgaatc tggtggtggt ctggttcagc cgggtggttc tctgcgtctg 60
   tcttgccgtg cgtctcagtc tatcttctct aaccactgga tgaactgggt tcgtcaggcg 120
   ccgggtaaag gtctggaatg gatcggtgaa atccgttcta aatcttctaa ctctgcgacc 180
   tcttacgcgg aatctgttaa aggtcgtttc atcatctctc gtgacggtaa caaaaacgac 240
   ctgaccctgg aaatgaactc tctgaaaacc gaagacaccg cggaatacta ctgcgcgcag 300
   aactactacg gttctacccc ggactactgg ggtcagggta ccctggttac cgtttct    357
<210> 201
   <211> 357
   <212> DNA
   <213> Artificial Sequence
<220>
   <223> Infliximab VH variant
<400> 201
   gaagttcagc tgaccgaatc tggtggtggt ctggttcagc cgggtggttc tctgcgtctg 60
   tcttgcgcgg cgtctggttc tatcttctct aaccactaca tgaactgggt tcgtcaggcg 120
   ccgggtaaag gtctggaatg gatcggtgaa atccgttcta aatcttctaa ctctgcgacc 180
   cactacgcgg aatctgttaa aggtcgtttc atcatctctc gtgacgacaa caaaaccgac 240
   ctgtacctgg aaatgaactc tctgaaaacc gaagacaccg cggaatacta ctgcgcgcgt 300
   aattactacg gttctaccta cgactactgg ggtcagggta ccctggttac cgtttct    357
<210> 202
   <211> 357
   <212> DNA
   <213> Artificial Sequence
<220>
   <223> Infliximab VH variant
<400> 202
   gaagttcagc tgaccgaatc tggtggtggt ctggttcagc cgggtggttc tctgcgtctg 60
   tcttgcgcgg cgtctcagtt caccttctct aaccactgga tgaactgggt tcgtcaggcg 120
   ccgggtaaag gtctggaatg gatcggtgaa atccgttcta aatctatcta cggtgcgacc 180
   cactacgcgg aatctgttaa aggtcgtttc atcatctctc gtgacggtaa cggtaacgac 240
   ctgtacctgg aaatgaactc tctgaaaacc gaagacaccg cggaatacta ctgcgcgcgt 300
   aactactacg gttctacccc ggactactgg ggtcagggta ccctggttac tgtttct 357
<210> 203
   <211> 357
   <212> DNA
   <213> Artificial Sequence
<220>
   <223> Infliximab VH variant
<400> 203
   gaagttcagc tgaccgaatc tggtggtggt ctggttcagc cgggtggttc tctgcgtctg 60
   tcttgccgtg cgtctcagtc taccttctct aaccactgga tgaactgggt tcgtcaggcg 120
   ccgggtaaag gtctggaatg gatcggtgaa atccgttcta aatcttctta ctctgcgacc 180
   cactacgcgg aatctgttaa aggtcgtttc atcatctctc gtgacgacaa caaaaacgac 240
   ctgtacctgg aaatgaactc tctgaaaacc gaagacaccg cggaatacta ctgcgcgcag 300
   aactactacg gttctacccc ggactactgg ggtcagggta ccctggttac cgtttct 357
<210> 204
   <211> 357
   <212> DNA
   <213> Artificial Sequence
<220>
   <223> Infliximab VH variant
<400> 204
   gaagttcagc tgaccgaatc tggtggtggt ctggttcagc cgggtggttc tctgcgtctg 60
   tcttgcgcgg cgtctcagtc tatcttctct aaccactaca tgaactgggt tcgtcaggcg 120
   ccgggtaaag gtctggaatg gatcggtgaa atccgttcta aatcttctaa ctctgcgacc 180
   cactacgcgg aatctgttaa aggtcgtttc atcatctctc gtgacggtaa cggtaacgac 240
   ctgaccctgg aaatgaactc tctgaaaacc gaagacaccg cggaatacta ctgcgcgcag 300
   aactactacg gttctacccc ggactactgg ggtcagggta ccctggttac cgtttct 357
<210> 205
   <211> 357
   <212> DNA
   <213> Artificial Sequence
<220>
   <223> Infliximab VH variant
<400> 205
   gaagttcagc tgaccgaatc tggtggtggt ctggttcagc cgggtggttc tctgcgtctg 60
   tcttgccgtg cgtctggttt caccttctct aaccactgga tgaactgggt tcgtcaggcg 120
   ccgggtaaag gtctggaatg gatcggtgaa atccgttcta aatcttctta ctctgcgacc 180
   cactacgcgg aatctgttaa aggtcgtttc atcatctctc gtgacgacaa caaaacctct 240
   ctgtacctgg aaatgaactc tctgaaaacc gaagacaccg cggaatacta ctgcgcgcgt 300
   aactactacg gttctacccc ggactactgg ggtcagggta ccctggttac cgtttct 357
<210> 206
   <211> 357
   <212> DNA
   <213> Artificial Sequence
<220>
   <223> Infliximab VH variant
<400> 206
   gaagttcagc tggttgaatc tggtggtggt ctggttcagc cgggtggttc tctgcgtctg 60
   tcttgccgtg cgtctcagtt caccttctct aaccactaca tgaactgggt tcgtcaggcg 120
   ccgggtaaag gtctggaatg gatcggtgaa atccgttcta aatcttctaa cggtgcgacc 180
   tcttacgcgg aatctgttaa aggtcgtttc atcatctctc gtgacggtaa cggtaactct 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 207
   <211> 357
   <212> DNA
   <213> Artificial Sequence
<220>
   <223> Infliximab VH variant
<400> 207
   gaagttcagc tgaccgaatc tggtggtggt ctggttcagc cgggtggttc tctgcgtctg 60
   tcttgccgtg cgtctcagtt caccttctct aaccactaca tgaactgggt tcgtcaggcg 120
   ccgggtaaag gtctggaatg gatcggtgaa atccgttcta aatcttctaa ctctgcgacc 180
   tcttacgcgg aatctgttaa aggtcgtttc atcatctctc gtgacgacaa caaaacctct 240
   ctgaccctgg aaatgaactc tctgaaaacc gaagacaccg cggaatacta ctgcgcgcag 300
   aactactacg gttctacccc ggactactgg ggtcagggta ccctggttac cgtttct    357
<210> 208
   <211> 357
   <212> DNA
   <213> Artificial Sequence
<220>
   <223> Infliximab VH variant
<400> 208
   gaagttcagc tggttgaatc tggtggtggt ctggttcagc cgggtggttc tctgcgtctg 60
   tcttgccgtg cgtctggttt caccttctct aaccactaca tgaactgggt tcgtcaggcg 120
   ccgggtaaag gtctggaatg gatcggtgaa atccgttcta aatcttctaa ctctgcgacc 180
   tcttacgcgg aatctgttaa aggtcgtttc atcatctctc gtgacgacaa cggtaacgac 240
   ctgaccctgg aaatgaactc tctgaaaacc gaagacaccg cggaatacta ctgcgcgcag 300
   aactactacg gttctacccc ggactactgg ggtcagggta ccctggttac cgtttct    357
<210> 209
   <211> 357
   <212> DNA
   <213> Artificial Sequence
<220>
   <223> Infliximab VH variant
<400> 209
   gaagttcagc tgaccgaatc tggtggtggt ctggttcagc cgggtggttc tctgcgtctg 60
   tcttgcgcgg cgtctcagtt caccttctct aaccactgga tgaactgggt tcgtcaggcg 120
   ccgggtaaag gtctggaatg gatcggtgaa atccgttcta aatctatcta cggtgcgacc 180
   cactacgcgg aatctgttaa aggtcgtttc atcatctctc gtgacgacaa cggtaacgac 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 210
   <211> 357
   <212> DNA
   <213> Artificial Sequence
<220>
   <223> Infliximab VH variant
<400> 210
   gaagttcagc tggttgaatc tggtggtggt ctggttcagc cgggtggttc tctgcgtctg 60
   tcttgccgtg cgtctggttc taccttctct aaccactgga tgaactgggt tcgtcaggcg 120
   ccgggtaaag gtctggaatg gatcggtgaa atccgttcta aatcttctaa ctctgcgacc 180
   cactacgcgg aatctgttaa aggtcgtttc atcatctctc gtgacgacaa cggtaactct 240
   ctgaccctgg aaatgaactc tctgaaaacc gaagacaccg cggaatacta ctgcgcgcag 300
   aactactacg gttctaccta cgactactgg ggtcagggta ccctggttac cgtttct    357
<210> 211
   <211> 357
   <212> DNA
   <213> Artificial Sequence
<220>
   <223> Infliximab VH variant
<400> 211
   gaagttcagc tgaccgaatc tggtggtggt ctggttcagc cgggtggttc tctgcgtctg 60
   tcttgcgcgg cgtctggttc tatcttctct aaccactgga tgaactgggt tcgtcaggcg 120
   ccgggtaaag gtctggaatg gatcggtgaa atccgttcta aatctatcta cggtgcgacc 180
   cactacgcgg aatctgttaa aggtcgtttc atcatctctc gtgacgacaa cggtaccgac 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 212
   <211> 357
   <212> DNA
   <213> Artificial Sequence
<220>
   <223> Infliximab VH variant
<400> 212
   gaagttcagc tgaccgaatc tggtggtggt ctggttcagc cgggtggttc tctgcgtctg 60
   tcttgcgcgg cgtctggttc tatcttctct aaccactgga tgaactgggt tcgtcaggcg 120
   ccgggtaaag gtctggaatg gatcggtgaa atccgttcta aatctatcaa cggtgcgacc 180
   tcttacgcgg aatctgttaa aggtcgtttc atcatctctc gtgacgacaa cggtaactct 240
   ctgaccctgg aaatgaactc tctgaaaacc gaagacaccg cggaatacta ctgcgcgcag 300
   aactactacg gttctacccc ggactactgg ggtcagggta ccctggttac cgtttct    357
<210> 213
   <211> 357
   <212> DNA
   <213> Artificial Sequence
<220>
   <223> Infliximab VH variant
<400> 213
   gaagttcagc tggttgaatc tggtggtggt ctggttcagc cgggtggttc tctgcgtctg 60
   tcttgccgtg cgtctcagtt catcttctct aaccactaca tgaactgggt tcgtcaggcg 120
   ccgggtaaag gtctggaatg gatcggtgaa atccgttcta aatctatcaa ctctgcgacc 180
   cactacgcgg aatctgttaa aggtcgtttt atcatctctc gtgacggtaa caaaacctct 240
   ctgtacctgg aaatgaactc tctgaaaacc gaagacaccg cggaatacta ctgcgcgcag 300
   aactactacg gttctacccc ggactactgg ggtcagggta ccctggttac cgtttct    357
<210> 214
   <211> 357
   <212> DNA
   <213> Artificial Sequence
<220>
   <223> Infliximab VH variant
<400> 214
   gaagttcagc tggttgaatc tggtggtggt ctggttcagc cgggtggttc tctgcgtctg 60
   tcttgccgtg cgtctcagtt caccttctct aaccactaca tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacgacaa caaaaacgac 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 215
   <211> 357
   <212> DNA
   <213> Artificial Sequence
<220>
   <223> Infliximab VH variant
<400> 215
   gaagttcagc tggttgaatc tggtggtggt ctggttcagc cgggtggttc tctgcgtctg 60
   tcttgcgcgg cgtctcagtt catcttctct aaccactaca tgaactgggt tcgtcaggcg 120
   ccgggtaaag gtctggaatg gatcggtgaa atccgttcta aatcttctaa cggtgcgacc 180
   cactacgcgg aatctgttaa aggtcgtttc atcatctctc gtgacgacaa caaaaacgac 240
   ctgtacctgg aaatgaactc tctgaaaacc gaagacaccg cggaatacta ctgcgcgcag 300
   aactactacg gttctacccc ggactactgg ggtcagggta ccctggttac cgtttct    357
<210> 216
   <211> 357
   <212> DNA
   <213> Artificial Sequence
<220>
   <223> Infliximab VH variant
<400> 216
   gaagttcagt tggtcgaatc tggtggtggt ctggttcagc cgggtggttc tctgcgtctg 60
   tcttgcgcgg cgtctggttc taccttctct aaccactgga tgaactgggt tcgtcaggcg 120
   ccgggtaaag gtctggaatg gatcggtgaa atccgttcta aatcttctta cggtgcgacc 180
   tcttacgcgg aatctgttaa aggtcgtttc atcatctctc gtgacggtaa cggtaactct 240
   ctgaccctgg aaatgaactc tctgaaaacc gaagacaccg cggaatacta ctgcgcgcag 300
   aactactacg gttctacccc ggactactgg ggtcagggta ccctggttac cgtttct    357
<210> 217
   <211> 357
   <212> DNA
   <213> Artificial Sequence
<220>
   <223> Infliximab VH variant
<400> 217
   gaagttcagc tgaccgaatc tggtggtggt ctggttcagc cgggtggttc tctgcgtctg 60
   tcttgccgtg cgtctcagtc tatcttctct aaccactaca tgaactgggt tcgtcaggcg 120
   ccaggtaaag gtctggaatg gatcggtgaa atccgttcta aatcttctaa ctctgcgacc 180
   tcttacgcgg aatctgttaa aggtcgtttc atcatctctc gtgacgacaa caaaacctct 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 218
   <211> 357
   <212> DNA
   <213> Artificial Sequence
<220>
   <223> Infliximab VH variant
<400> 218
   gaagttcagc tggttgaatc tggtggtggt ctggttcagc cgggtggttc tctgcgtctg 60
   tcttgccgtg cgtctcagtc taccttctct aaccactgga tgaactgggt tcgtcaggcg 120
   ccgggtaaag gtctggaatg gatcggtgaa atccgttcta aatctatcta ctctgcgacc 180
   tcttacgcgg aatctgttaa aggtcgtttc atcatctctc gtgacggtaa caaaacctct 240
   ctgaccctgg aaatgaactc tctgaaaacc gaagacaccg cggaatacta ctgcgcgcag 300
   aactactacg gttctacccc ggactactgg ggtcagggta ccctggttac cgtttct    357
<210> 219
   <211> 357
   <212> DNA
   <213> Artificial Sequence
<220>
   <223> Infliximab VH variant
<400> 219
   gaagttcagc tgaccgaatc tggtggtggt ctggttcagc cgggtggttc tctgcgtctg 60
   tcttgcgcgg cgtctcagtt catcttctct aaccactaca tgaactgggt tcgtcaggcg 120
   ccgggtaaag gtctggaatg gatcggtgaa atccgttcta aatcttctta cggtgcgacc 180
   cactacgcgg aatctgttaa aggtcgtttc atcatctctc gtgacggtaa caaaaccgac 240
   ctgaccctgg aaatgaactc tctgaaaacc gaagacaccg cggaatacta ctgcgcgcag 300
   aactactacg gttctaccta cgactactgg ggtcagggta ccctggttac cgtttct    357
<210> 220
   <211> 357
   <212> DNA
   <213> Artificial Sequence
<220>
   <223> Infliximab VH variant
<400> 220
   gaagttcagc tgaccgaatc tggtggtggt ctggttcagc cgggtggttc tctgcgtctg 60
   tcttgccgtg cgtctcagtt caccttctct aaccactaca tgaactgggt tcgtcaggcg 120
   ccgggtaaag gtctggaatg gatcggtgaa atccgttcta aatcttctaa cggtgcgacc 180
   tcttacgcgg aatctgttaa aggtcgtttc atcatctctc gtgacggtaa cggtaactct 240
   ctgaccctgg aaatgaactc tctgaaaacc gaagacaccg cggaatacta ctgcgcgcag 300
   aactactacg gttctaccta cgactactgg ggtcagggta ccctggttac cgtttct    357
<210> 221
   <211> 357
   <212> DNA
   <213> Artificial Sequence
<220>
   <223> Infliximab VH variant
<400> 221
   gaagttcagc tggttgaatc tggtggtggt ctggttcagc cgggtggttc tctgcgtctg 60
   tcttgccgtg cgtctggttc tatcttctct aaccactaca tgaattgggt tcgtcaggcg 120
   ccgggtaaag gtctggaatg gatcggtgaa atccgttcta aatcttctaa cggtgcgacc 180
   cactacgcgg aatctgttaa aggtcgtttc atcatctctc gtgacgacaa caaaaactct 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 222
   <211> 357
   <212> DNA
   <213> Artificial Sequence
<220>
   <223> Infliximab VH variant
<400> 222
   gaagttcagc tggttgaatc tggtggtggt ctggttcagc cgggtggttc tctgcgtctg 60
   tcttgcgcgg cgtctcagtc tatcttctct aaccactgga tgaactgggt tcgtcaggcg 120
   ccgggtaaag gtctggaatg gatcggtgaa atccgttcta aatctatcaa cggtgcgacc 180
   tcttacgcgg aatctgttaa aggtcgtttc atcatctctc gtgacggtaa cggtacctct 240
   ctgaccctgg aaatgaactc tctgaaaacc gaagacaccg cggaatacta ctgcgcgcag 300
   aactactacg gttctaccta cgactactgg ggtcagggta ccctggttac cgtttct    357
<210> 223
   <211> 357
   <212> DNA
   <213> Artificial Sequence
<220>
   <223> Infliximab VH variant
<400> 223
   gaagttcagc tgaccgaatc tggtggtggt ctggttcagc cgggtggttc tctgcgtctg 60
   tcttgccgtg cgtctcagtc taccttctct aaccactaca tgaactgggt tcgtcaggcg 120
   ccgggtaaag gtctggaatg gatcggtgaa atccgttcta aatctatcaa ctctgcgacc 180
   tcttacgcgg aatctgttaa aggtcgtttc atcatctctc gtgacggtaa cggtaacgac 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 224
   <211> 357
   <212> DNA
   <213> Artificial Sequence
<220>
   <223> Infliximab VH variant
<400> 224
   gaagttcagc tggttgaatc tggtggtggt ctggttcagc cgggtggttc tctgcgtctg 60
   tcttgcgcgg cgtctcagtt caccttctct aaccactaca tgaactgggt tcgtcaggcg 120
   ccgggtaaag gtctggaatg gatcggtgaa atccgttcta aatctatcta cggtgcgacc 180
   tcttacgcgg aatctgttaa aggtcgtttc atcatctctc gtgacgacaa cggtacctct 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 225
   <211> 357
   <212> DNA
   <213> Artificial Sequence
<220>
   <223> Infliximab VH variant
<400> 225
   gaagttcagc tgaccgaatc tggtggtggt ctggttcagc cgggtggttc tctgcgtttg 60
   tcttgccgtg cgtctcagtc taccttctct aaccactaca tgaactgggt tcgtcaggcg 120
   ccgggtaaag gtctggaatg gatcggtgaa atccgttcta aatcttctaa ctctgcgacc 180
   tcttacgcgg aatctgttaa aggtcgtttc atcatctctc gtgacggtaa cggtaacgac 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 226
   <211> 357
   <212> DNA
   <213> Artificial Sequence
<220>
   <223> Infliximab VH variant
<400> 226
   gaagttcagc tgaccgaatc tggtggtggt ctggttcagc cgggtggttc tctgcgtctg 60
   tcttgccgtg cgtctcagtc taccttctct aaccactgga tgaactgggt tcgtcaggcg 120
   ccgggtaaag gtctggaatg gatcggtgaa atccgttcta aatcttctta cggtgcgacc 180
   tcttacgcgg aatctgttaa aggtcgtttc atcatctctc gtgacggtaa cggtaactct 240
   ctgaccctgg aaatgaactc tctgaaaacc gaagacaccg cggaatacta ctgcgcgcag 300
   aactactacg gttctaccta cgactactgg ggtcagggta ccctggttac cgtttct    357
<210> 227
   <211> 357
   <212> DNA
   <213> Artificial Sequence
<220>
   <223> Infliximab VH variant
<400> 227
   gaagttcagc tggttgaatc tggtggtggt ctggttcagc cgggtggttc tctgcgtctg 60
   tcttgccgtg cgtctcagtc taccttctct aaccactaca tgaactgggt tcgtcaggcg 120
   ccgggtaaag gtctggaatg gatcggtgaa atccgttcta aatctatcaa ctctgcgacc 180
   cactacgcgg aatctgttaa aggtcgtttc atcatctctc gtgacggtaa caaaaacgac 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 228
   <211> 357
   <212> DNA
   <213> Artificial Sequence
<220>
   <223> Infliximab VH variant
<400> 228
   gaagttcagc tggttgaatc tggtggtggt ctggttcagc cgggtggttc tctgcgtctg 60
   tcttgcgcgg cgtctggttc taccttctct aaccactaca tgaactgggt tcgtcaggcg 120
   ccgggtaaag gtctggaatg gatcggtgaa atccgttcta aatctatcaa ctctgcgacc 180
   tcttacgcgg aatctgttaa aggtcgtttc atcatctctc gtgacggtaa cggtacctct 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 229
   <211> 357
   <212> DNA
   <213> Artificial Sequence
<220>
   <223> Infliximab VH variant
<400> 229
   gaagttcagc tgaccgaatc tggtggtggt ctggttcagc cgggtggttc tctgcgtctg 60
   tcttgccgtg cgtctcagtc taccttctct aaccactaca tgaactgggt tcgtcaggcg 120
   ccgggtaaag gtctggaatg gatcggtgaa atccgttcta aatcttctaa cggtgcgacc 180
   tcttacgcgg aatctgttaa aggtcgtttc atcatctctc gtgacgacaa cggtaccgac 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 230
   <211> 357
   <212> DNA
   <213> Artificial Sequence
<220>
   <223> Infliximab VH variant
<400> 230
   gaagttcagc tggttgaatc tggtggtggt ctggttcagc cgggtggttc tctgcgtctg 60
   tcttgcgcgg cgtctggttt caccttctct aaccactaca tgaactgggt tcgtcaggcg 120
   ccgggtaaag gtctggaatg gatcggtgaa atccgttcta aatcttctta ctctgcgacc 180
   tcttacgcgg aatctgttaa aggtcgtttc atcatctctc gtgacgacaa caaaaactct 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 231
   <211> 357
   <212> DNA
   <213> Artificial Sequence
<220>
   <223> Infliximab VH variant
<400> 231
   gaagttcagc tggttgaatc tggtggtggt ctggttcagc cgggtggttc tctgcgtctg 60
   tcttgccgtg cgtctggttc tatcttctct aaccactgga tgaactgggt tcgtcaggcg 120
   ccgggtaaag gtctggaatg gatcggtgaa atccgttcta aatctatcaa cggtgcgacc 180
   tcttacgcgg aatctgttaa aggtcgtttc atcatctctc gtgacgacaa cggtaactct 240
   ctgaccctgg aaatgaactc tctgaaaacc gaagacaccg cggaatacta ctgcgcgcag 300
   aactactacg gttctacccc ggactactgg ggtcagggta ccctggttac cgtttct    357
<210> 232
   <211> 357
   <212> DNA
   <213> Artificial Sequence
<220>
   <223> Infliximab VH variant
<400> 232
   gaagttcagc tgaccgaatc tggtggtggt ctggttcagc cgggtggttc tctgcgtctg 60
   tcttgcgcgg cgtctggttt caccttctct aaccactgga tgaactgggt tcgtcaggcg 120
   ccgggtaaag gtctggaatg gatcggtgaa atccgttcta aatcttctta ctctgcgacc 180
   cactacgcgg aatctgttaa aggtcgtttc atcatctctc gtgacgacaa caaaaccgac 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 233
   <211> 357
   <212> DNA
   <213> Artificial Sequence
<220>
   <223> Infliximab VH variant
<400> 233
   gaagttcagc tggttgaatc tggtggtggt ctggttcagc cgggtggttc tctgcgtctg 60
   tcttgccgtg cgtcaggttt catcttctct aaccactgga tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacggtaa cggtaacgac 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 234
   <211> 357
   <212> DNA
   <213> Artificial Sequence
<220>
   <223> Infliximab VH variant
<400> 234
   gaagttcagc tggttgaatc tggtggtggt ctggttcagc cgggtggttc tctgcgtctg 60
   tcttgccgtg cgtctggttc taccttctct aaccactaca tgaactgggt tcgtcaggcg 120
   ccgggtaaag gtctggaatg gatcggtgaa atccgttcta aatcttctta cggtgcgacc 180
   tcttacgcgg aatctgttaa aggtcgtttc atcatctctc gtgacggtaa cggtaacgac 240
   ctgtacctgg aaatgaactc tctgaaaacc gaagacaccg cggaatacta ctgcgcgcag 300
   aactactacg gttctacccc ggactactgg ggtcagggta ccctggttac cgtttct    357
<210> 235
   <211> 357
   <212> DNA
   <213> Artificial Sequence
<220>
   <223> Infliximab VH variant
<400> 235
   gaagttcagc tggttgaatc tggtggtggt ctggttcagc cgggtggttc tctgcgtctg 60
   tcttgccgtg cgtctcagtc tatcttctct aaccactgga tgaactgggt tcgtcaggcg 120
   ccgggtaaag gtctggaatg gatcggtgaa atccgttcta aatctatcaa ctctgcgacc 180
   cactacgcgg aatctgttaa aggtcgtttc atcatctctc gtgacgacaa cggtacctct 240
   ctgtacctgg aaatgaactc tctgaaaacc gaagacaccg cggaatacta ctgcgcgcgt 300
   aactactacg gttctacccc ggactactgg ggtcagggta ccctggttac cgtttct    357
<210> 236
   <211> 357
   <212> DNA
   <213> Artificial Sequence
<220>
   <223> Infliximab VH variant
<400> 236
   gaagttcagc tgaccgaatc tggtggtggt ctggttcagc cgggtggttc tctgcgtctg 60
   tcttgccgtg cgtctcagtt catcttctct aaccactaca tgaactgggt tcgtcaggcg 120
   ccgggtaaag gtctggaatg gatcggtgaa atccgttcta aatctatcta cggtgcgacc 180
   cactacgcgg aatctgttaa aggtcgtttc atcatctctc gtgacgacaa caaaaccgac 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 237
   <211> 357
   <212> DNA
   <213> Artificial Sequence
<220>
   <223> Infliximab VH variant
<400> 237
   gaagttcagc tgaccgaatc tggtggtggt ctggttcagc cgggtggttc tctgcgtctg 60
   tcttgcgcgg cgtctcagtt catcttctct aaccactgga tgaactgggt tcgtcaggcg 120
   ccgggtaaag gtctggaatg gatcggtgaa atccgttcta aatcttctta cggtgcgacc 180
   cactacgcgg aatctgttaa aggtcgtttc atcatctctc gtgacggtaa cggtaactct 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 238
   <211> 357
   <212> DNA
   <213> Artificial Sequence
<220>
   <223> Infliximab VH variant
<400> 238
   gaagttcagc tgaccgaatc tggtggtggt ctggttcagc cgggtggttc tctgcgtctg 60
   tcttgcgcgg cgtctcagtc tatcttctct aaccactaca tgaactgggt tcgtcaggcg 120
   ccgggtaaag gtctggaatg gatcggtgaa atccgttcta aatcttctta ctctgcgacc 180
   tcttacgcgg aatctgttaa aggtcgtttc atcatctctc gtgacgacaa cggtaacgac 240
   ctgaccctgg aaatgaactc tctgaaaacc gaagacaccg cggaatacta ctgcgcgcag 300
   aactactacg gttctacccc ggactactgg ggtcagggta ccctggttac cgtttct    357
<210> 239
   <211> 357
   <212> DNA
   <213> Artificial Sequence
<220>
   <223> Infliximab VH variant
<400> 239
   gaagttcagc tgaccgaatc tggtggtggt ctggttcagc cgggtggttc tctgcgtctg 60
   tcttgccgtg cgtctggttt catcttctct aaccactaca tgaactgggt tcgtcaggcg 120
   ccgggtaaag gtctggaatg gatcggtgaa atccgttcta aatcttctta ctctgcgacc 180
   tcttacgcgg aatctgttaa aggtcgtttc atcatctctc gtgacggtaa caaaaacgac 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 240
   <211> 357
   <212> DNA
   <213> Artificial Sequence
<220>
   <223> Infliximab VH variant
<400> 240
   gaagttcagc tggttgaatc tggtggtggt ctggttcagc cgggtggttc tctgcgtctg 60
   tcttgccgtg cgtctggttc tatcttctct aaccactgga tgaactgggt tcgtcaggcg 120
   ccgggtaaag gtctggaatg gatcggtgaa atccgttcta aatcttctta ctctgcgacc 180
   cactacgcgg aatctgttaa aggtcgtttc atcatctctc gtgacggtaa cggtaactct 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 241
   <211> 357
   <212> DNA
   <213> Artificial Sequence
<220>
   <223> Infliximab VH variant
<400> 241
   gaagttcagc tgaccgaatc tggtggtggt ctggttcagc cgggtggttc tctgcgtctg 60
   tcttgccgtg cgtctcagtc taccttctct aaccactgga tgaactgggt tcgtcaggcg 120
   ccgggtaaag gtctggaatg gatcggtgaa atccgttcta aatcttctta cggtgcgacc 180
   cactacgcgg aatctgttaa aggtcgtttc atcatctctc gtgacgacaa cggtaccgac 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 242
   <211> 357
   <212> DNA
   <213> Artificial Sequence
<220>
   <223> Infliximab VH variant
<400> 242
   gaagttcagc tgaccgaatc tggtggtggt ctggttcagc cgggtggttc tctgcgtctg 60
   tcttgcgcgg cgtctggttc tatcttctct aaccactaca tgaattgggt tcgtcaggcg 120
   ccgggtaaag gtctggaatg gatcggtgaa atccgttcta aatctatcta cggtgcgacc 180
   tcttacgcgg aatctgttaa aggtcgtttc atcatctctc gtgacggtaa cggtaactct 240
   ctgtacctgg aaatgaactc tctgaaaacc gaagacaccg cggaatacta ctgcgcgcag 300
   aactactacg gttctacccc ggactactgg ggtcagggta ccctggttac cgtttct    357
<210> 243
   <211> 357
   <212> DNA
   <213> Artificial Sequence
<220>
   <223> Infliximab VH variant
<400> 243
   gaagttcagc tgaccgaatc tggtggtggt ctggttcagc cgggtggttc tctgcgtctg 60
   tcttgccgtg cgtctggttc tatcttctct aaccactaca tgaactgggt tcgtcaggcg 120
   ccgggtaaag gtctggaatg gatcggtgaa atccgttcta aatcttctta cggtgcgacc 180
   cactacgcgg aatctgttaa aggtcgtttc atcatctctc gtgacggtaa cggtaccgac 240
   ctgaccctgg aaatgaactc tctgaaaacc gaagacaccg cggaatacta ctgcgcgcag 300
   aactactacg gttctaccta cgactactgg ggtcagggta ccctggttac cgtttct    357
<210> 244
   <211> 357
   <212> DNA
   <213> Artificial Sequence
<220>
   <223> Infliximab VH variant
<400> 244
   gaagttcagc tggttgaatc tggtggtggt ctggttcagc cgggtggttc tctgcgtctg 60
   tcttgccgtg cgtctcagtc tatcttctct aaccactaca tgaactgggt tcgtcaggcg 120
   ccgggtaaag gtctggaatg gatcggtgaa atccgttcta aatcttctta cggtgcgacc 180
   cactacgcgg aatctgttaa aggtcgtttc atcatctctc gtgacgacaa caaaaactct 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 245
   <211> 357
   <212> DNA
   <213> Artificial Sequence
<220>
   <223> Infliximab VH variant
<400> 245
   gaagttcagc tgaccgaatc tggtggtggt ctggttcagc cgggtggttc tctgcgtctg 60
   tcttgcgcgg cgtctggttt catcttctct aaccactgga tgaactgggt tcgtcaggcg 120
   ccgggtaaag gtctggaatg gatcggtgaa atccgttcta aatcttctta ctctgcgacc 180
   cactacgcgg aatctgttaa aggtcgtttc atcatctctc gtgacgacaa cggtacctct 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 246
   <211> 357
   <212> DNA
   <213> Artificial Sequence
<220>
   <223> Infliximab VH variant
<400> 246
   gaagttcagc tgaccgaatc tggtggtggt ctggttcagc cgggtggttc tctgcgtctg 60
   tcttgcgcgg cgtctcagtt catcttctct aaccactgga tgaactgggt tcgtcaggcg 120
   ccgggtaaag gtctggaatg gatcggtgaa atccgttcta aatcttctaa ctctgcgacc 180
   tcttacgcgg aatctgttaa aggtcgtttc atcatctctc gtgacgacaa cggtaacgac 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 247
   <211> 357
   <212> DNA
   <213> Artificial Sequence
<220>
   <223> Infliximab VH variant
<400> 247
   gaagttcagc tggttgaatc tggtggtggt ctggttcagc cgggtggttc tctgcgtctg 60
   tcttgccgtg cgtctcagtc tatcttctct aaccactgga tgaactgggt tcgtcaggcg 120
   ccgggtaaag gtctggaatg gatcggtgaa atccgttcta aatctatcaa ctctgcgacc 180
   tcttacgcgg aatctgttaa aggtcgtttc atcatctctc gtgacgacaa cggtacctct 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 248
   <211> 357
   <212> DNA
   <213> Artificial Sequence
<220>
   <223> Infliximab VH variant
<400> 248
   gaagttcagc tgaccgaatc tggtggtggt ctggttcagc cgggtggttc tctgcgtctg 60
   tcttgccgtg cgtctcagtc taccttctct aaccactgga tgaactgggt tcgtcaggcg 120
   ccgggtaaag gtctggaatg gatcggtgaa atccgttcta aatctatcaa cggtgcgacc 180
   tcttacgcgg aatctgttaa aggtcgtttc atcatctctc gtgacgacaa cggtaactct 240
   ctgaccctgg aaatgaactc tctgaaaacc gaagacaccg cggaatacta ctgcgcgcag 300
   aactactacg gttctaccta cgactactgg ggtcagggta ccctggttac cgtttct    357
<210> 249
   <211> 357
   <212> DNA
   <213> Artificial Sequence
<220>
   <223> Infliximab VH variant
<400> 249
   gaagttcagc tggttgaatc tggtggtggt ctggttcagc cgggtggttc tctgcgtctg 60
   tcttgccgtg cgtctggttt catcttctct aaccactaca tgaactgggt tcgtcaggcg 120
   ccgggtaaag gtctggaatg gatcggtgaa atccgttcta aatctatcta cggtgcgacc 180
   cactacgcgg aatctgttaa aggtcgtttc atcatctctc gtgacggtaa cggtaacgac 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 250
   <211> 357
   <212> DNA
   <213> Artificial Sequence
<220>
   <223> Infliximab VH variant
<400> 250
   gaagttcagc tgaccgaatc tggtggtggt ctggttcagc cgggtggttc tctgcgtctg 60
   tcttgcgcgg cgtctcagtc taccttctct aaccactgga tgaactgggt tcgtcaggcg 120
   ccgggtaaag gtctggaatg gatcggtgaa atccgttcta aatcttctaa cggtgcgacc 180
   tcttacgcgg aatctgttaa aggtcgtttc atcatctctc gtgacggtaa caaaaactct 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 251
   <211> 357
   <212> DNA
   <213> Artificial Sequence
<220>
   <223> Infliximab VH variant
<400> 251
   gaagttcagc tgaccgaatc tggtggtggt ctggttcagc cgggtggttc tctgcgtctg 60
   tcttgccgtg cgtctggttt caccttctct aaccactaca tgaactgggt tcgtcaggcg 120
   ccgggtaaag gtctggaatg gatcggtgaa atccgttcta aatctatcta cggtgcgacc 180
   tcttacgcgg aatctgttaa aggtcgtttc atcatctctc gtgacggtaa cggtaccgac 240
   ctgaccctgg aaatgaactc tctgaaaacc gaagacaccg cggaatacta ctgcgcgcag 300
   aactactacg gttctaccta cgactactgg ggtcagggta ccctggttac cgtttct    357
<210> 252
   <211> 357
   <212> DNA
   <213> Artificial Sequence
<220>
   <223> Infliximab VH variant
<400> 252
   gaagttcagc tgaccgaatc tggtggtggt ctggttcagc cgggtggttc tctgcgtctg 60
   tcttgcgcgg cgtctcagtt caccttctct aaccactgga tgaactgggt tcgtcaggcg 120
   ccgggtaaag gtctggaatg gatcggtgaa atccgttcta aatctatcta cggtgcgacc 180
   cactacgcgg aatctgttaa aggtcgtttc atcatctctc gtgacgacaa caaaacctct 240
   ctgtacctgg aaatgaactc tctgaaaacc gaagacaccg cggaatacta ctgcgcgcgt 300
   aactactacg gttctacccc ggactactgg ggtcagggta ccctggttac cgtttct    357
<210> 253
   <211> 357
   <212> DNA
   <213> Artificial Sequence
<220>
   <223> Infliximab VH variant
<400> 253
   gaagttcagc tggttgaatc tggtggtggt ctggttcagc cgggtggttc tctgcgtctg 60
   tcttgccgtg cgtctggttt catcttctct aaccactgga tgaactgggt tcgtcaggcg 120
   ccgggtaaag gtctggaatg gatcggtgaa atccgttcta aatctatcta cggtgcgacc 180
   cactacgcgg aatctgttaa aggtcgtttc atcatctctc gtgacgacaa caaaaactct 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 254
   <211> 357
   <212> DNA
   <213> Artificial Sequence
<220>
   <223> Infliximab VH variant
<400> 254
   gaagttcagc tgaccgaatc tggtggtggt ctggttcagc cgggtggttc tctgcgtctg 60
   tcttgccgtg cgtctggttt caccttctct aaccactaca tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacgacaa caaaaccgac 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 255
   <211> 357
   <212> DNA
   <213> Artificial Sequence
<220>
   <223> Infliximab VH variant
<400> 255
   gaagttcagc tgaccgaatc tggtggtggt ctggttcagc cgggtggttc tctgcgtctg 60
   tcttgccgtg cgtctggttt caccttctct aaccactaca tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacggtaa caaaaccgac 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 256
   <211> 357
   <212> DNA
   <213> Artificial Sequence
<220>
   <223> Infliximab VH variant
<400> 256
   gaagttcagc tgaccgaatc tggtggtggt ctggttcagc cgggtggttc tctgcgtctg 60
   tcttgccgtg cgtctcagtt catcttctct aaccactgga tgaactgggt tcgtcaggcg 120
   ccgggtaaag gtctggaatg gatcggtgaa atccgttcta aatctatcaa ctctgcgacc 180
   cactacgcgg aatctgttaa aggtcgtttc atcatctctc gtgacgacaa caaaaacgac 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 257
   <211> 357
   <212> DNA
   <213> Artificial Sequence
<220>
   <223> Infliximab VH variant
<400> 257
   gaagttcagc tggttgaatc tggtggtggt ctggttcagc cgggtggttc tctgcgtctg 60
   tcttgccgtg cgtctcagtt caccttctct aaccactgga tgaactgggt tcgtcaggcg 120
   ccgggtaaag gtctggaatg gatcggtgaa atccgttcta aatcttctaa ctctgcgacg 180
   cactacgcgg aatctgttaa aggtcgtttc atcatctctc gtgacggtaa cggtaactct 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 258
   <211> 357
   <212> DNA
   <213> Artificial Sequence
<220>
   <223> Infliximab VH variant
<400> 258
   gaagttcagc tgaccgaatc tggtggtggt ctggttcagc cgggtggttc tctgcgtctg 60
   tcttgccgtg cgtctcagtc tatcttctct aaccactgga tgaactgggt tcgtcaggcg 120
   ccgggtaaag gtctggaatg gatcggtgaa atccgttcta aatctatcaa cggtgcgacc 180
   cactacgcgg aatctgttaa aggtcgtttc atcatctctc gtgacgacaa caaaaacgac 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 259
   <211> 357
   <212> DNA
   <213> Artificial Sequence
<220>
   <223> Infliximab VH variant
<400> 259
   gaagttcagc tgaccgaatc tggtggtggt ctggttcagc cgggtggttc tctgcgtctg 60
   tcttgccgtg cgtctggttc taccttctct aaccactgga tgaactgggt tcgtcaggcg 120
   ccgggtaaag gtctggaatg gatcggtgaa atccgttcta aatctatcta cggtgcgacc 180
   tcttacgcgg aatctgttaa aggtcgtttc atcatctctc gtgacggtaa caaaacctct 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 260
   <211> 357
   <212> DNA
   <213> Artificial Sequence
<220>
   <223> Infliximab VH variant
<400> 260
   gaagttcagc tgaccgaatc tggtggtggt ctggttcagc cgggtggttc tctgcgtctg 60
   tcttgccgtg cgtctggttc tatcttctct aaccactaca tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacggtaa cggtacctct 240
   ctgtacctgg aaatgaactc tttgaaaacc gaagacaccg cggaatacta ctgcgcgcag 300
   aactactacg gttctaccta cgactactgg ggtcagggta ccctggttac cgtttct    357
<210> 261
   <211> 357
   <212> DNA
   <213> Artificial Sequence
<220>
   <223> Infliximab VH variant
<400> 261
   gaagttcagc tggttgaatc tggtggtggt ctggttcagc cgggtggttc tctgcgtctg 60
   tcttgccgtg cgtctggttc tatcttctct aaccactaca tgaactgggt tcgtcaggcg 120
   ccgggtaaag gtctggaatg gatcggtgaa atccgttcta aatcttctta cggtgcgacc 180
   tcttacgcgg aatctgttaa aggtcgtttc atcatctctc gtgacgacaa caaaaacgac 240
   ctgaccctgg aaatgaactc tctgaaaacc gaagacaccg cggaatacta ctgcgcgcag 300
   aactactacg gttctacccc ggactactgg ggtcagggta ccctggttac cgtttct    357
<210> 262
   <211> 357
   <212> DNA
   <213> Artificial Sequence
<220>
   <223> Infliximab VH variant
<400> 262
   gaagttcagc tgaccgaatc tggtggtggt ctggttcagc cgggtggttc tctgcgtctg 60
   tcttgcgcgg cgtctggttc taccttctct aaccactgga tgaactgggt tcgtcaggcg 120
   ccgggtaaag gtctggaatg gatcggtgaa atccgttcta aatcttctta ctctgcgacc 180
   tcttacgcgg aatctgttaa aggtcgtttc atcatctctc gtgacgacaa caaaaactct 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 263
   <211> 357
   <212> DNA
   <213> Artificial Sequence
<220>
   <223> Infliximab VH variant
<400> 263
   gaagttcagc tggttgaatc tggtggtggt ctggttcagc cgggtggttc tctgcgtctg 60
   tcttgccgtg cgtctggttt caccttctct aaccactgga tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacgacaa caaaaactct 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 264
   <211> 357
   <212> DNA
   <213> Artificial Sequence
<220>
   <223> Infliximab VH variant
<400> 264
   gaagttcagc tgaccgaatc tggtggtggt ctggttcagc cgggtggttc tctgcgtctg 60
   tcttgccgtg cgtctggttt catcttctct aaccactaca tgaactgggt tcgtcaggcg 120
   ccgggtaaag gtctggaatg gatcggtgaa atccgttcta aatctatcaa cggtgcgacc 180
   cactacgcgg aatctgttaa aggtcgtttc atcatctctc gtgacgacaa cggtacctct 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 265
   <211> 357
   <212> DNA
   <213> Artificial Sequence
<220>
   <223> Infliximab VH variant
<400> 265
   gaagttcagc tggttgaatc tggtggtggt ctggttcagc cgggtggttc tctgcgtctg 60
   tcttgcgcgg cgtctggttc taccttctct aaccactaca tgaactgggt tcgtcaggcg 120
   ccgggtaaag gtctggaatg gatcggtgaa atccgttcta aatctatcta ctctgcgacc 180
   tcttacgcgg aatctgttaa aggtcgtttt atcatctctc gtgacgacaa cggtaactct 240
   ctgtacctgg aaatgaactc tctgaaaacc gaagacaccg cggaatacta ctgcgcgcag 300
   aattactacg gttctaccta cgactactgg ggtcagggta ccctggttac cgtttct    357
<210> 266
   <211> 357
   <212> DNA
   <213> Artificial Sequence
<220>
   <223> Infliximab VH variant
<400> 266
   gaagttcagc tgaccgaatc tggtggtggt ctggttcagc cgggtggttc tctgcgtctg 60
   tcttgccgtg cgtctcagtc taccttctct aaccactaca tgaactgggt tcgtcaggcg 120
   ccgggtaaag gtctggaatg gatcggtgaa atccgttcta aatcttctta cggtgcgacc 180
   tcttacgcgg aatctgttaa aggtcgtttc atcatctctc gtgacgacaa cggtacctct 240
   ctgtacctgg aaatgaactc tctgaaaacc gaagacaccg cggaatacta ctgcgcgcag 300
   aactactacg gttctacccc ggactactgg ggtcagggta ccctggttac cgtttct    357
<210> 267
   <211> 357
   <212> DNA
   <213> Artificial Sequence
<220>
   <223> Infliximab VH variant
<400> 267
   gaagttcagc tggttgaatc tggtggtggt ctggttcagc cgggtggttc tctgcgtcta 60
   tcttgcgcgg cgtctcagtc tatcttctct aaccactaca tgaactgggt tcgtcaggcg 120
   ccgggtaaag gtctggaatg gatcggtgaa atccgttcta aatctatcta cggtgcgacc 180
   cactacgcgg aatctgttaa aggtcgtttc atcatctctc gtgacggtaa caaaaacgac 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 268
   <211> 357
   <212> DNA
   <213> Artificial Sequence
<220>
   <223> Infliximab VH variant
<400> 268
   gaagttcagc tggttgaatc tggtggtggt ctggttcagc cgggtggttc tctgcgtctg 60
   tcttgccgtg cgtctcagtt catcttctct aaccactaca tgaactgggt tcgtcaggcg 120
   ccgggtaaag gtctggaatg gatcggtgaa atccgttcta aatccatcaa cggtgcgacc 180
   cactacgcgg aatctgttaa aggtcgtttc atcatctctc gtgacggtaa cggtaccgac 240
   ctgtacctgg aaatgaactc tctgaaaacc gaagacaccg cggaatacta ctgcgcgcgt 300
   aactactacg gttctacccc ggactactgg ggtcagggta ccctggttac cgtttct    357
<210> 269
   <211> 357
   <212> DNA
   <213> Artificial Sequence
<220>
   <223> Infliximab VH variant
<400> 269
   gaagttcagc tggttgaatc tggtggtggt ctggttcagc cgggtggttc tctgcgtctg 60
   tcttgccgtg cgtctggttc taccttctct aaccactaca tgaactgggt tcgtcaggcg 120
   ccgggtaaag gtctggaatg gatcggtgaa atccgttcta aatctatcaa ctctgcgacc 180
   tcttacgcgg aatctgttaa aggtcgtttc atcatctctc gtgacggtaa cggtaccgac 240
   ctgaccctgg aaatgaactc tctgaaaacc gaagacaccg cggaatacta ctgcgcgcag 300
   aactactacg gttctaccta cgactactgg ggtcagggta ccctggttac cgtttct    357
<210> 270
   <211> 357
   <212> DNA
   <213> Artificial Sequence
<220>
   <223> Infliximab VH variant
<400> 270
   gaagttcagc tggttgaatc tggtggtggt ctggttcagc cgggtggttc tctgcgtctg 60
   tcttgccgtg cgtctcagtc tatcttctct aaccactaca tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacggtaa cggtaacgac 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 271
   <211> 357
   <212> DNA
   <213> Artificial Sequence
<220>
   <223> Infliximab VH variant
<400> 271
   gaagttcagc tgaccgaatc tggtggtggt ctggttcagc cgggtggttc tctgcgtctg 60
   tcttgccgtg cgtctggttt catcttctct aaccactaca tgaactgggt tcgtcaggcg 120
   ccgggtaaag gtctggaatg gatcggtgaa atccgttcta aatctatcaa cggtgcgacc 180
   tcttacgcgg aatctgttaa aggtcgtttc atcatctctc gtgacgacaa cggtaactct 240
   ctgtacctgg aaatgaactc tctgaaaacc gaagacaccg cggaatacta ctgcgcgcgt 300
   aactactacg gttctacccc ggactactgg ggtcagggta ccctggttac cgtttct    357
<210> 272
   <211> 357
   <212> DNA
   <213> Artificial Sequence
<220>
   <223> Infliximab VH variant
<400> 272
   gaagttcagc tggttgaatc tggtggtggt ctggttcagc cgggtggttc tctgcgtctg 60
   tcttgcgcgg cgtcacagtc taccttctct aaccactgga tgaactgggt tcgtcaggcg 120
   ccgggtaaag gtctggaatg gatcggtgaa atccgttcta aatcttctta ctctgcgacc 180
   cactacgcgg aatctgttaa aggtcgtttc atcatctctc gtgacggtaa caaaaacgac 240
   ctgtacctgg aaatgaactc tctgaaaacc gaagacaccg cggaatacta ctgcgcgcgt 300
   aactactacg gttctactta cgactactgg ggtcagggta ccctggttac cgtttct    357
<210> 273
   <211> 357
   <212> DNA
   <213> Artificial Sequence
<220>
   <223> Infliximab VH variant
<400> 273
   gaagttcagc tgaccgaatc tggtggtggt ctggttcagc cgggtggttc tctgcgtctg 60
   tcttgccgtg cgtctggttt catcttctct aaccactgga tgaactgggt tcgtcaggcg 120
   ccgggtaaag gtctggaatg gatcggtgaa atccgttcta aatcttctaa cggtgcgacc 180
   tcttacgcgg aatctgttaa aggtcgtttc atcatctctc gtgacggtaa cggtacctct 240
   ctgaccctgg aaatgaactc tctgaaaacc gaagacaccg cggaatacta ctgcgcgcag 300
   aactactacg gttctaccta cgactactgg ggtcagggta ccctggttac cgtttct    357
<210> 274
   <211> 357
   <212> DNA
   <213> Artificial Sequence
<220>
   <223> Infliximab VH variant
<400> 274
   gaagttcagc tgaccgaatc tggtggtggt ctggttcagc cgggtggttc tctgcgtctg 60
   tcttgcgcgg cgtctcagtc tatcttctct aaccactaca tgaactgggt tcgtcaggcg 120
   ccgggtaaag gtctggaatg gatcggtgaa atccgttcta aatctatcta cggtgcgacc 180
   cactacgcgg aatctgttaa aggtcgtttc atcatctctc gtgacgacaa cggtaccgac 240
   ctgaccctgg aaatgaactc tctgaaaacc gaagacaccg cggaatacta ctgcgcgcag 300
   aactactacg gttctacccc ggactactgg ggtcagggta ccctggttac cgtttct    357
<210> 275
   <211> 357
   <212> DNA
   <213> Artificial Sequence
<220>
   <223> Infliximab VH variant
<400> 275
   gaagttcagc tggttgaatc tggtggtggt ctggttcagc cgggtggttc tctgcgtctg 60
   tcttgcgcgg cgtctggttt caccttctct aaccactgga tgaactgggt tcgtcaggcg 120
   ccgggtaaag gtctggaatg gatcggtgaa atccgttcta aatcttctaa ctctgcgacc 180
   cactacgcgg aatctgttaa aggtcgtttc atcatctctc gtgacggtaa caaaacctct 240
   ctgtacctgg aaatgaactc tctgaaaacc gaagacaccg cggaatacta ctgcgcgcgt 300
   aactattacg gttctaccta cgactactgg ggtcagggta ccctggttac cgtttct    357
<210> 276
   <211> 357
   <212> DNA
   <213> Artificial Sequence
<220>
   <223> Infliximab VH variant
<400> 276
   gaagttcagc tggttgaatc tggtggtggt ctggttcagc cgggtggttc tctgcgtctg 60
   tcttgcgcgg cgtctggttt caccttctct aaccactgga tgaactgggt tcgtcaggcg 120
   ccgggtaaag gtctggaatg gatcggtgaa atccgttcta aatcttctaa ctctgcgacc 180
   tcttacgcgg aatctgttaa aggtcgtttc atcatctctc gtgacggtaa caaaaacgac 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 277
   <211> 357
   <212> DNA
   <213> Artificial Sequence
<220>
   <223> Infliximab VH variant
<400> 277
   gaagttcagc tggttgaatc tggtggtggt ctggttcagc cgggtggttc tctgcgtctg 60
   tcttgccgtg cgtctcagtt caccttctct aaccactaca tgaactgggt tcgtcaggcg 120
   ccgggtaaag gtctggaatg gatcggtgaa atccgttcta aatctatcaa cggtgcgacc 180
   tcttacgcgg aatctgttaa aggtcgtttc atcatctctc gtgacgacaa cggtaccgac 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 278
   <211> 357
   <212> DNA
   <213> Artificial Sequence
<220>
   <223> Infliximab VH variant
<400> 278
   gaagttcagc tgaccgaatc tggtggtggt ctggttcagc cgggtggttc tctgcgtctg 60
   tcttgccgtg cgtctcagtt catcttctct aaccactaca tgaactgggt tcgtcaggcg 120
   ccgggtaaag gtctggaatg gatcggtgaa atccgttcta aatctatcaa cggtgcgacc 180
   cactacgcgg aatctgttaa aggtcgtttc atcatctctc gtgacgacaa cggtaccgac 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 279
   <211> 357
   <212> DNA
   <213> Artificial Sequence
<220>
   <223> Infliximab VH variant
<400> 279
   gaagttcagc tgaccgaatc tggtggtggt ctggttcagc cgggtggttc tctgcgtctg 60
   tcttgcgcgg cgtctcagtc tatcttctct aaccactaca tgaactgggt tcgtcaggcg 120
   ccgggtaaag gtctggaatg gatcggtgaa atccgttcta aatctatcaa ctctgcgacc 180
   cactacgcgg aatctgttaa aggtcgtttc atcatctctc gtgacggtaa caaaaacgac 240
   ctgtacctgg aaatgaactc tctgaaaacc gaagacaccg cggaatacta ctgcgcgcgt 300
   aactactacg gttctacccc ggactactgg ggtcagggta ccctggttac cgtttct    357
<210> 280
   <211> 357
   <212> DNA
   <213> Artificial Sequence
<220>
   <223> Infliximab VH variant
<400> 280
   gaagttcagc tggttgaatc tggtggtggt ctggttcagc cgggtggttc tctgcgtctg 60
   tcttgcgcgg cgtctggttc tatcttctct aaccactgga tgaactgggt tcgtcaggcg 120
   ccgggtaaag gtctggaatg gatcggtgaa atccgttcta aatcttctaa ctctgcgacc 180
   cactacgcgg aatctgttaa aggtcgtttc atcatctctc gtgacggtaa caaaaacgac 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 281
   <211> 357
   <212> DNA
   <213> Artificial Sequence
<220>
   <223> Infliximab VH variant
<400> 281
   gaagttcagc tgaccgaatc tggtggtggt ctggtacagc cgggtggttc tctgcgtctg 60
   tcttgtcgtg cgtctggttt caccttctct aaccactaca tgaactgggt tcgtcaggcg 120
   ccgggtaaag gtctggaatg gatcggtgaa atccgttcta aatcttctta cggtgcgacc 180
   cactacgcgg aatctgttaa aggtcgtttc atcatctctc gtgacgacaa cggtaccgac 240
   ctgtacctgg aaatgaactc tctgaaaacc gaagacaccg cggaatacta ctgcgcgcag 300
   aactactacg gttctacccc ggactactgg ggtcagggta ccctggttac cgtttct    357
<210> 282
   <211> 357
   <212> DNA
   <213> Artificial Sequence
<220>
   <223> Infliximab VH variant
<400> 282
   gaagttcagc tggttgaatc tggtggtggt ctggttcagc cgggtggttc tctgcgtcta 60
   tcttgccgtg cgtctggttc tatcttctct aaccactgga tgaactgggt tcgtcaggcg 120
   ccgggtaaag gtctggaatg gatcggtgaa atccgttcta aatctatcaa cggtgcgacc 180
   cactacgcgg aatctgttaa aggtcgtttc atcatctctc gtgacgacaa cggtacctct 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 283
   <211> 357
   <212> DNA
   <213> Artificial Sequence
<220>
   <223> Infliximab VH variant
<400> 283
   gaagttcagc tgaccgaatc tggtggtggt ctggttcagc cgggtggttc tctgcgtctg 60
   tcttgccgtg cgtctggttc taccttctct aaccactaca tgaactgggt tcgtcaggcg 120
   ccgggtaaag gtctggaatg gatcggtgaa atccgttcta aatcttctaa ctctgcgacc 180
   tcttacgcgg aatctgttaa aggtcgtttc atcatctctc gtgacgacaa caaaacctct 240
   ctgtacctgg aaatgaactc tctgaaaacc gaagacaccg cggaatacta ctgcgcgcgt 300
   aattactacg gttctaccta cgactactgg ggtcagggta ccctggttac cgtttct    357
<210> 284
   <211> 357
   <212> DNA
   <213> Artificial Sequence
<220>
   <223> Infliximab VH variant
<400> 284
   gaagttcagc tggttgaatc tggtggtggt ctggttcagc cgggtggttc tctgcgtctg 60
   tcttgccgtg cgtctcagtc tatcttctct aaccactaca tgaactgggt tcgtcaggcg 120
   ccgggtaaag gtctggaatg gatcggtgaa atccgttcta aatctatcaa ctctgcgacc 180
   cactacgcgg aatctgttaa aggtcgtttc atcatctctc gtgacgacaa cggtaactct 240
   ctgaccctgg aaatgaactc tctaaaaacc gaagacaccg cggaatacta ctgcgcgcag 300
   aactactacg gttctaccta cgactactgg ggtcagggta ccctggttac cgtttct    357
<210> 285
   <211> 357
   <212> DNA
   <213> Artificial Sequence
<220>
   <223> Infliximab VH variant
<400> 285
   gaagttcagc tgaccgaatc tggtggtggt ctggttcagc cgggtggttc tctgcgtctg 60
   tcttgccgtg cgtctggttc taccttctct aaccactgga tgaactgggt tcgtcaggcg 120
   ccgggtaaag gtctggaatg gatcggtgaa atccgttcta aatcttctaa cggtgcgacc 180
   tcttacgcgg aatctgttaa aggtcgtttc atcatctctc gtgacgacaa cggtaccgac 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 286
   <211> 357
   <212> DNA
   <213> Artificial Sequence
<220>
   <223> Infliximab VH variant
<400> 286
   gaagttcagc tgaccgaatc tggtggtggt ctggttcagc cgggtggttc tctgcgtctg 60
   tcttgccgtg cgtctggttt caccttctct aaccactgga tgaactgggt tcgtcaggcg 120
   ccgggtaaag gtctggaatg gatcggtgaa atccgttcta aatcttctaa cggtgcgacc 180
   cactacgcgg aatctgttaa aggtcgtttc atcatctctc gtgacggtaa caaaaccgac 240
   ctgtacctgg aaatgaactc tctgaaaacc gaagacaccg cggaatacta ctgcgcgcgt 300
   aactactacg gttctacccc ggactactgg ggtcagggta ccctggttac cgtttct    357
<210> 287
   <211> 357
   <212> DNA
   <213> Artificial Sequence
<220>
   <223> Infliximab VH variant
<400> 287
   gaagttcagc tggttgaatc tggtggtggt ctggttcagc cgggtggttc tctgcgtctg 60
   tcttgcgcgg cgtctggttc tatcttctct aaccactaca tgaactgggt tcgtcaggcg 120
   ccgggtaaag gtctggaatg gatcggtgaa atccgttcta aatcttctaa cggtgcgacc 180
   cactacgcgg aatctgttaa aggtcgtttc atcatctctc gtgacgacaa caaaaactct 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 288
   <211> 357
   <212> DNA
   <213> Artificial Sequence
<220>
   <223> Infliximab VH variant
<400> 288
   gaagttcagc tgaccgaatc tggtggtggt ctggttcagc cgggtggttc tctgcgtctg 60
   tcttgccgtg cgtctcagtt catcttctct aaccactaca tgaactgggt tcgtcaggcg 120
   ccgggtaaag gtctggaatg gatcggtgaa atccgttcta aatctatcaa cggtgcgacc 180
   cactacgcgg aatctgttaa aggtcgtttc atcatctctc gtgacgacaa caaaaccgac 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 289
   <211> 357
   <212> DNA
   <213> Artificial Sequence
<220>
   <223> Infliximab VH variant
<400> 289
   gaagttcagc tggttgaatc tggtggtggt ctggttcagc cgggtggttc tctgcgtctg 60
   tcttgccgtg cgtctggttt caccttctct aaccactaca tgaactgggt tcgtcaggcg 120
   ccgggtaaag gtctggaatg gatcggtgaa atccgttcta aatcttctaa ctctgcgacc 180
   tcttacgcgg aatctgttaa aggtcgtttc atcatctctc gtgacgacaa cggtaactct 240
   ctgtacctgg aaatgaactc tctgaaaacc gaagacaccg cggaatacta ctgcgcgcag 300
   aactactacg gttctacccc ggactactgg ggtcagggta ccctggttac cgtttct    357
<210> 290
   <211> 357
   <212> DNA
   <213> Artificial Sequence
<220>
   <223> Infliximab VH variant
<400> 290
   gaagttcagc tggttgaatc tggtggtggt ctggttcagc cgggtggttc tctgcgtctg 60
   tcttgccgtg cgtctggttt catcttctct aaccactgga tgaactgggt tcgtcaggcg 120
   ccgggtaaag gtctggaatg gatcggtgaa atccgttcta aatctatcaa cggtgcgacc 180
   cactacgcgg aatctgttaa aggtcgtttc atcatctctc gtgacgacaa caaaacctct 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 291
   <211> 357
   <212> DNA
   <213> Artificial Sequence
<220>
   <223> Infliximab VH variant
<400> 291
   gaagttcagc tgaccgaatc tggtggtggt ctggttcagc cgggtggttc tctgcgtctg 60
   tcttgcgcgg cgtctcagtc taccttctct aaccactgga tgaactgggt tcgtcaggcg 120
   ccgggtaaag gtctggaatg gatcggtgaa atccgttcta aatctatcaa cggtgcgacc 180
   tcttacgcgg aatctgttaa aggtcgtttc atcatctctc gtgacgacaa cggtaactct 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 292
   <211> 357
   <212> DNA
   <213> Artificial Sequence
<220>
   <223> Infliximab VH variant
<400> 292
   gaagttcagc tgaccgaatc tggtggtggt ctggttcagc cgggtggttc tctgcgtctg 60
   tcttgccgtg cgtctcagtt catcttctct aaccactaca tgaactgggt tcgtcaggcg 120
   ccgggtaaag gtctggaatg gatcggtgaa atccgttcta aatctatcaa ctctgcgacc 180
   cactacgcgg aatctgttaa aggtcgtttc atcatctctc gtgacgacaa caaaaacgac 240
   ctgtacctgg aaatgaactc tctgaaaacc gaagacaccg cggaatacta ctgcgcgcag 300
   aactactacg gttctacccc ggactactgg ggtcagggta ccctggttac cgtttct    357
<210> 293
   <211> 357
   <212> DNA
   <213> Artificial Sequence
<220>
   <223> Infliximab VH variant
<400> 293
   gaagttcagc tgaccgaatc tggtggtggt ctggttcagc cgggtggttc tctgcgtctg 60
   tcttgcgcgg cgtctggttt catcttctct aaccactaca tgaactgggt tcgtcaggcg 120
   ccgggtaaag gtctggaatg gatcggtgaa atccgttcta aatcttctaa cggtgcgacc 180
   tcttacgcgg aatctgttaa aggtcgtttc atcatctctc gtgacgacaa cggtaactct 240
   ctgaccctgg aaatgaactc tctgaaaacc gaagacaccg cggaatacta ctgcgcgcag 300
   aactactacg gttctacccc ggactactgg ggtcagggta ccctggttac cgtttct    357
<210> 294
   <211> 357
   <212> DNA
   <213> Artificial Sequence
<220>
   <223> Infliximab VH variant
<400> 294
   gaagttcagc tggttgaatc tggtggtggt ctggttcagc cgggtggttc tctgcgtctg 60
   tcttgcgcgg cgtctcagtt catcttctct aaccactgga tgaactgggt tcgtcaggcg 120
   ccgggtaaag gtctggaatg gatcggtgaa atccgttcta aatcttctaa cggtgcgacc 180
   tcttacgcgg aatctgttaa aggtcgtttc atcatctctc gtgacggtaa caaaaccgac 240
   ctgaccctgg aaatgaactc tctgaaaacc gaagacaccg cggaatacta ctgcgcgcag 300
   aactactacg gttctaccta cgactactgg ggtcagggta ccctggttac cgtttct    357
<210> 295
   <211> 357
   <212> DNA
   <213> Artificial Sequence
<220>
   <223> Infliximab VH variant
<400> 295
   gaagttcagc tgaccgaatc tggtggtggt ctggttcagc cgggtggttc tctgcgtctg 60
   tcttgccgtg cgtctcagtc taccttctct aaccactaca tgaactgggt tcgtcaggcg 120
   ccgggtaaag gtctggaatg gatcggtgaa atccgttcta aatctatcaa ctctgcgacc 180
   cactacgcgg aatctgttaa aggtcgtttc atcatctctc gtgacggtaa caaaaccgac 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 296
   <211> 357
   <212> DNA
   <213> Artificial Sequence
<220>
   <223> Infliximab VH variant
<400> 296
   gaagttcagc tgaccgaatc tggtggtggt ctggttcagc cgggtggttc tctgcgtctg 60
   tcttgcgcgg cgtctcagtt caccttctct aaccactaca tgaactgggt tcgtcaggcg 120
   ccgggtaaag gtctggaatg gatcggtgaa atccgttcta aatctatcta ctctgcgacc 180
   tcttacgcgg aatctgttaa aggtcgtttc atcatctctc gtgacggtaa caaaaactct 240
   ctgaccctgg aaatgaactc tctgaaaacc gaagacaccg cggaatacta ctgcgcgcag 300
   aactactatg gttctaccta cgactactgg ggtcagggta ccctggttac cgtttct    357
<210> 297
   <211> 357
   <212> DNA
   <213> Artificial Sequence
<220>
   <223> Infliximab VH variant
<400> 297
   gaagttcagc tggttgaatc tggtggtggt ctggttcagc cgggtggttc tctgcgtctg 60
   tcttgccgtg cgtctggttc tatcttctct aaccactgga tgaactgggt tcgtcaggcg 120
   ccgggtaaag gtctggaatg gatcggtgaa atccgttcta aatcttctaa ctctgcgacc 180
   cactacgcgg aatctgttaa aggtcgtttc atcatctctc gtgacgacaa caaaaacgac 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 298
   <211> 357
   <212> DNA
   <213> Artificial Sequence
<220>
   <223> Infliximab VH variant
<400> 298
   gaagttcagc tggttgaatc tggtggtggt ctggttcagc cgggtggttc tctgcgtctg 60
   tcttgcgcgg cgtctggttc tatcttctct aaccactgga tgaactgggt tcgtcaggcg 120
   ccgggtaaag gtctggaatg gatcggtgaa atccgttcta aatctatcaa ctctgcgacc 180
   tcttacgcgg aatctgttaa aggtcgtttc atcatctctc gtgacgacaa caaaaccgac 240
   ctgtacctgg aaatgaactc tctgaaaacc gaagacaccg cggaatacta ctgcgcgcag 300
   aactactacg gttctacccc ggactactgg ggtcagggta ccctggttac cgtttct    357
<210> 299
   <211> 357
   <212> DNA
   <213> Artificial Sequence
<220>
   <223> Infliximab VH variant
<400> 299
   gaagttcagc tgaccgaatc tggtggtggt ctggttcagc cgggtggttc tctgcgtctg 60
   tcttgccgtg cgtctggttt catcttctct aaccactaca tgaactgggt tcgtcaggcg 120
   ccgggtaaag gtctggaatg gatcggtgaa atccgttcta aatctatcaa ctctgcgacc 180
   cactacgcgg aatctgttaa aggtcgtttc atcatctctc gtgacgacaa cggtaactct 240
   ctgtacctgg aaatgaactc tctgaaaacc gaagacaccg cggaatacta ctgcgcgcgt 300
   aactactacg gttctacccc ggactactgg ggtcagggta ccctggttac cgtttct    357
<210> 300
   <211> 357
   <212> DNA
   <213> Artificial Sequence
<220>
   <223> Infliximab VH variant
<400> 300
   gaagttcagc tggttgaatc tggtggtggt ctggttcagc cgggtggttc tctgcgtctg 60
   tcttgtcgtg cgtctggttt caccttctct aaccactgga tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacgacaa caaaaccgac 240
   ctgtacctgg aaatgaactc tctgaaaacc gaagacaccg cggaatacta ctgcgcgcgt 300
   aactactacg gttctacccc ggactactgg ggtcagggta ccctggttac cgtttct    357
<210> 301
   <211> 357
   <212> DNA
   <213> Artificial Sequence
<220>
   <223> Infliximab VH variant
<400> 301
   gaagttcagc tggttgaatc tggtggtggt ctggttcagc cgggtggttc tctgcgtctg 60
   tcttgccgtg cgtctcagtt catcttctct aaccactaca tgaactgggt tcgtcaggcg 120
   ccgggtaaag gtctggaatg gatcggtgaa atccgttcta aatctatcta cggtgcgacc 180
   cactacgcgg aatctgttaa aggtcgtttc atcatctctc gtgacggtaa cggtaccgac 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 302
   <211> 357
   <212> DNA
   <213> Artificial Sequence
<220>
   <223> Infliximab VH variant
<400> 302
   gaagttcagc tgaccgaatc tggtggtggt ctggttcagc cgggtggttc tctgcgtctg 60
   tcttgccgtg cgtctcagtc taccttctct aaccactaca tgaactgggt tcgtcaggcg 120
   ccgggtaaag gtctggaatg gatcggtgaa atccgttcta aatcttctaa ctctgcgacc 180
   tcttacgcgg aatctgttaa aggtcgtttc atcatctctc gtgacggtaa caaaaactct 240
   ctgtacctgg aaatgaactc tctgaaaacc gaagacaccg cggaatacta ctgcgcgcag 300
   aactactacg gttctacccc ggactactgg ggtcagggta ccctggttac cgtttct    357
<210> 303
   <211> 357
   <212> DNA
   <213> Artificial Sequence
<220>
   <223> Infliximab VH variant
<400> 303
   gaagttcagc tggttgaatc tggtggtggt ctggttcagc cgggtggttc tctgcgtctg 60
   tcttgccgtg cgtctggttc tatcttctct aaccactaca tgaactgggt tcgtcaggcg 120
   ccgggtaaag gtctggaatg gatcggtgaa atccgttcta aatctatcaa ctctgcgacc 180
   cactacgcgg aatctgttaa aggtcgtttc atcatctctc gtgacggtaa cggtacctct 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 304
   <211> 357
   <212> DNA
   <213> Artificial Sequence
<220>
   <223> Infliximab VH variant
<400> 304
   gaagttcagc tgaccgaatc tggtggtggt ctggttcagc cgggtggttc tctgcgtctg 60
   tcttgcgcgg cgtctcagtc taccttctct aaccactgga tgaactgggt tcgtcaggcg 120
   ccgggtaaag gtctggaatg gatcggtgaa atccgttcta aatctatcaa cggtgcgacc 180
   cactacgcgg aatctgttaa aggtcgtttc atcatctctc gtgacgacaa cggtaacgac 240
   ctgaccctgg aaatgaactc tctgaaaacc gaagacaccg cggaatacta ctgcgcgcag 300
   aactactacg gttctacccc ggactactgg ggtcagggta ccctggttac cgtttct    357
<210> 305
   <211> 357
   <212> DNA
   <213> Artificial Sequence
<220>
   <223> Infliximab VH variant
<400> 305
   gaagttcagc tgaccgaatc tggtggtggt ctggttcagc cgggtggttc tctgcgtctg 60
   tcttgcgcgg cgtctcagtt caccttctct aaccactgga tgaactgggt tcgtcaggcg 120
   ccgggtaaag gtctggaatg gatcggtgaa atccgttcta aatcttctaa ctctgcgacc 180
   cactacgcgg aatctgttaa aggtcgtttc atcatctctc gtgacggtaa caaaacctct 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 306
   <211> 357
   <212> DNA
   <213> Artificial Sequence
<220>
   <223> Infliximab VH variant
<400> 306
   gaagttcagc tggttgaatc tggtggtggt ctggttcagc cgggtggttc tctgcgtctg 60
   tcttgcgcgg cgtctcagtt caccttctct aaccactgga tgaactgggt tcgtcaggcg 120
   ccgggtaaag gtctggaatg gatcggtgaa atccgttcta aatctatcaa ctctgcgacc 180
   tcttacgcgg aatctgttaa aggtcgtttc atcatctctc gtgacgacaa caaaaactct 240
   ctgtacctgg aaatgaactc tctgaaaacc gaagacaccg cggaatacta ctgcgcgcgt 300
   aactactacg gttctactcc ggactactgg ggtcagggta ccctggttac cgtttct    357
<210> 307
   <211> 357
   <212> DNA
   <213> Artificial Sequence
<220>
   <223> Infliximab VH variant
<400> 307
   gaagttcagc tgaccgaatc tggtggtggt ctggttcagc cgggtggttc tctgcgtctg 60
   tcttgccgtg cgtctcagtc tatcttctct aaccactgga tgaactgggt tcgtcaggcg 120
   ccgggtaaag gtctggaatg gatcggtgaa atccgttcta aatctatcta tggtgcgacc 180
   tcttacgcgg aatctgttaa aggtcgtttc atcatctctc gtgacggtaa caaaaccgac 240
   ctgtacctgg aaatgaactc tctgaaaacc gaagacaccg cggaatacta ctgcgcgcag 300
   aactactacg gttctacccc ggactactgg ggtcagggta ccctggttac cgtttct    357
<210> 308
   <211> 357
   <212> DNA
   <213> Artificial Sequence
<220>
   <223> Infliximab VH variant
<400> 308
   gaagttcagc tgaccgaatc tggtggtggt ctggttcagc cgggtggttc tctgcgtctg 60
   tcttgccgtg cgtctcagtc taccttctct aaccactgga tgaactgggt tcgtcaggcg 120
   ccgggtaaag gtctggaatg gatcggtgaa atccgttcta aatcttctta ctctgcgacc 180
   cactacgcgg aatctgttaa aggtcgtttc atcatctctc gtgacggtaa cggtaactct 240
   ctgtacctgg aaatgaactc tctgaaaacc gaagacaccg cggaatacta ctgcgcgcag 300
   aactactacg gttctacccc ggactactgg ggtcagggta ccctggttac cgtttct    357
<210> 309
   <211> 357
   <212> DNA
   <213> Artificial Sequence
<220>
   <223> Infliximab VH variant
<400> 309
   gaagttcagc tggttgaatc tggtggtggt ctggttcagc cgggtggttc tctgcgtctg 60
   tcttgcgcgg cgtctcagtt caccttctct aaccactaca tgaactgggt tcgtcaggcg 120
   ccgggtaaag gtctggaatg gatcggtgaa atccgttcta aatctatcaa ctctgcgacc 180
   cactacgcgg aatctgttaa aggtcgtttc atcatctctc gtgacgacaa cggtaacgac 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 310
   <211> 357
   <212> DNA
   <213> Artificial Sequence
<220>
   <223> Infliximab VH variant
<400> 310
   gaagttcagc tgaccgaatc tggtggtggt ctggttcagc cgggtggttc tctgcgtctg 60
   tcttgccgtg cgtctcagtt catcttctct aaccactaca tgaactgggt tcgtcaggcg 120
   ccgggtaaag gtctggaatg gatcggtgaa atccgttcta aatctatcaa ctctgcgacc 180
   tcttacgcgg aatctgttaa aggtcgtttc atcatctctc gtgacggtaa cggtaacgac 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 311
   <211> 357
   <212> DNA
   <213> Artificial Sequence
<220>
   <223> Infliximab VH variant
<400> 311
   gaagttcagc tgaccgaatc tggtggtggt ctggttcagc cgggtggttc tctgcgtctg 60
   tcttgccgtg cgtctcagtt caccttctct aaccactaca tgaactgggt tcgtcaggcg 120
   ccaggtaaag gtctggaatg gatcggtgaa atccgttcta aatcttctta cggtgcgacc 180
   tcttacgcgg aatctgttaa aggtcgtttc atcatctctc gtgacgacaa cggtaacgac 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 312
   <211> 357
   <212> DNA
   <213> Artificial Sequence
<220>
   <223> Infliximab VH variant
<400> 312
   gaagttcagc tggttgaatc tggtggtggt ctggttcagc cgggtggttc tctgcgtctg 60
   tcttgcgcgg cgtctcagtc taccttctct aaccactaca tgaactgggt tcgtcaggcg 120
   ccgggtaaag gtctggaatg gatcggtgaa atccgttcta aatcttctaa cagtgcgacc 180
   cactacgcgg aatctgttaa aggtcgtttc atcatctctc gtgacggtaa caaaaacgac 240
   ctgtacctgg aaatgaactc tctgaaaacc gaagacaccg cggaatacta ctgcgcgcgt 300
   aactactacg gttctacccc ggactactgg ggtcagggta ccctggttac cgtttct    357
<210> 313
   <211> 357
   <212> DNA
   <213> Artificial Sequence
<220>
   <223> Infliximab VH variant
<400> 313
   gaagttcagc tgaccgaatc tggtggtggt ctggttcagc cgggtggttc tctgcgtctg 60
   tcttgcgcgg cgtctcagtt caccttctct aaccactaca tgaactgggt tcgtcaggcg 120
   ccgggtaaag gtctggaatg gatcggtgaa atccgttcta aatctatcaa cggtgcgacc 180
   tcttacgcgg aatctgttaa aggtcgtttc atcatctctc gtgacgacaa cggtacctct 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 314
   <211> 357
   <212> DNA
   <213> Artificial Sequence
<220>
   <223> Infliximab VH variant
<400> 314
   gaagttcagc tggttgaatc tggtggtggt ctggttcagc cgggtggttc tctgcgtctg 60
   tcttgccgtg cgtctggttc taccttctct aaccactaca tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacggtaa caaaaactct 240
   ctgtacctgg aaatgaactc tctgaaaacc gaagacaccg cggaatacta ctgcgcgcag 300
   aactactacg gttctaccta cgactactgg ggtcagggta ccctggttac cgtttct 357
<210> 315
   <211> 357
   <212> DNA
   <213> Artificial Sequence
<220>
   <223> Infliximab VH variant
<400> 315
   gaagttcagt tgaccgaatc tggtggtggt ctggttcagc cgggtggttc tctgcgtctg 60
   tcttgcgcgg cgtctcagtt catcttctct aaccactgga tgaactgggt tcgtcaggcg 120
   ccgggtaaag gtctggaatg gatcggtgaa atccgttcta aatcttctta cggtgcgacc 180
   tcttacgcgg aatctgttaa aggtcgtttc atcatctctc gtgacgacaa cggtaactct 240
   ctgaccctgg aaatgaactc tctgaaaacc gaagacaccg cggaatacta ctgcgcgcgt 300
   aactactacg gttctacccc ggactactgg ggtcagggta ccctggttac cgtttct 357
<210> 316
   <211> 357
   <212> DNA
   <213> Artificial Sequence
<220>
   <223> Infliximab VH variant
<400> 316
   gaagttcagc tggttgaatc tggtggtggt ctggttcagc cgggtggttc tctgcgtctg 60
   tcttgccgtg cgtctcagtc taccttctct aaccactgga tgaactgggt tcgtcaggcg 120
   ccgggtaaag gtctggaatg gatcggtgaa atccgttcta aatctatcaa ctctgcgacc 180
   tcttacgcgg aatctgttaa aggtcgtttc atcatctctc gtgacgacaa cggtaacgac 240
   ctgtacctgg aaatgaactc tttgaaaacc gaagacaccg cggaatacta ctgcgcgcgt 300
   aactactacg gttctaccta cgactactgg ggtcagggta ccctggttac cgtttct 357
<210> 317
   <211> 357
   <212> DNA
   <213> Artificial Sequence
<220>
   <223> Infliximab VH variant
<400> 317
   gaagttcagc tggttgaatc tggtggtggt ctggttcagc cgggtggttc tctacgtctg 60
   tcttgcgcgg cgtctcagtc taccttctct aaccactgga tgaactgggt tcgtcaggcg 120
   ccgggtaaag gtctggaatg gatcggtgaa atccgttcta aatcttctta cggtgcgacc 180
   cactacgcgg aatctgttaa aggtcgtttc atcatctctc gtgacggtaa cggtacctct 240
   ctgaccctgg aaatgaactc tctgaaaacc gaagacaccg cggaatacta ctgcgcgcgt 300
   aactactacg gttctaccta cgactactgg ggtcagggta ccctggttac cgtttct 357
<210> 318
   <211> 357
   <212> DNA
   <213> Artificial Sequence
<220>
   <223> Infliximab VH variant
<400> 318
   gaagttcagc tggttgaatc tggtggtggt ctggttcagc cgggtggttc tctgcgtctg 60
   tcttgccgtg cgtctggttt catcttctct aaccactaca tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacgacaa cggtaactct 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 319
   <211> 357
   <212> DNA
   <213> Artificial Sequence
<220>
   <223> Infliximab VH variant
<400> 319
   gaagttcagc tgaccgaatc tggtggtggt ctggttcagc cgggtggttc tctgcgtctg 60
   tcttgcgcgg cgtctcagtt caccttctct aaccactaca tgaactgggt tcgtcaggcg 120
   ccgggtaaag gtctggaatg gatcggtgaa atccgttcta aatcttctaa ctctgcgacc 180
   tcttacgcgg aatctgttaa aggtcgtttc atcatctctc gtgacggtaa cggtacctct 240
   ctgaccctgg aaatgaactc tctgaaaacc gaagacaccg cggaatacta ctgcgcgcag 300
   aactactacg gttctaccta cgactactgg ggtcagggta ccctggttac cgtttct    357
<210> 320
   <211> 357
   <212> DNA
   <213> Artificial Sequence
<220>
   <223> Infliximab VH variant
<400> 320
   gaagttcagc tggttgaatc tggtggtggt ctggttcagc cgggtggttc tctgcgtctg 60
   tcttgcgcgg cgtctcagtc tatcttctct aaccactaca tgaactgggt tcgtcaggcg 120
   ccgggtaaag gtctggaatg gatcggtgaa atccgttcta aatctatcta cggtgcgacc 180
   tcttacgcgg aatctgttaa aggtcgtttc atcatctctc gtgacgacaa caaaaactct 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 321
   <211> 357
   <212> DNA
   <213> Artificial Sequence
<220>
   <223> Infliximab VH variant
<400> 321
   gaagttcagc tggttgaatc tggtggtggt ctggttcagc cgggtggttc tctgcgtctg 60
   tcttgcgcgg cgtctcagtc taccttctct aaccactaca tgaactgggt tcgtcaggcg 120
   ccgggtaaag gtctggaatg gatcggtgaa atccgttcta aatcttctta cggtgcgacc 180
   cactacgcgg aatctgttaa aggtcgtttc atcatctctc gtgacgacaa caaaaacgac 240
   ctgtacctgg aaatgaactc tctgaaaacc gaagacaccg cggaatacta ctgcgcgcgt 300
   aactactacg gttctacccc ggactactgg ggtcagggta ccctggttac cgtttct    357
<210> 322
   <211> 357
   <212> DNA
   <213> Artificial Sequence
<220>
   <223> Infliximab VH variant
<400> 322
   gaagttcagc tgaccgaatc tggtggtggt ctggttcagc cgggtggttc tctgcgtctg 60
   tcttgcgcgg cgtctggttc tatcttctct aaccactgga tgaactgggt tcgtcaggcg 120
   ccgggtaaag gtctggaatg gatcggtgaa atccgttcta aatctatcaa cggtgcgacc 180
   cactacgcgg aatctgttaa aggtcgtttc atcatctctc gtgacggtaa cggtacctct 240
   ctgtacctgg aaatgaactc tctgaaaacc gaagacaccg cggaatacta ctgcgcgcgt 300
   aactactacg gttctacccc ggactactgg ggtcagggta ccctggttac cgtttct    357
<210> 323
   <211> 357
   <212> DNA
   <213> Artificial Sequence
<220>
   <223> Infliximab VH variant
<400> 323
   gaagttcagc tggttgaatc tggtggtggt ctggttcagc cgggtggttc tctgcgtctg 60
   tcttgcgcgg cgtctcagtc tatcttctct aaccactgga tgaactgggt tcgtcaggcg 120
   ccgggtaaag gtctggaatg gatcggtgaa atccgttcta aatcttctaa ctctgcgacc 180
   tcttacgcgg aatctgttaa aggtcgtttc atcatctctc gtgacggtaa caaaaccgac 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 324
   <211> 357
   <212> DNA
   <213> Artificial Sequence
<220>
   <223> Infliximab VH variant
<400> 324
   gaagttcagc tgaccgaatc tggtggtggt ctggttcagc cgggtggttc tctgcgtctg 60
   tcttgcgcgg cgtctcagtc tatcttctct aaccactgga tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacgacaa cggtacctct 240
   ctgtacctag aaatgaactc tctgaaaacc gaagacaccg cggaatacta ctgcgcgcag 300
   aactactacg gttctacccc ggactactgg ggtcagggta ccctggttac cgtttct    357
<210> 325
   <211> 357
   <212> DNA
   <213> Artificial Sequence
<220>
   <223> Infliximab VH variant
<400> 325
   gaagttcagc tggttgaatc tggtggtggt ctggttcagc cgggtggttc tctgcgtctg 60
   tcttgcgcgg cgtctggttc taccttctct aaccactaca tgaactgggt tcgtcaggcg 120
   ccgggtaaag gtctggaatg gatcggtgaa atccgttcta aatcttctta ctctgcgacc 180
   tcttacgcgg aatctgttaa aggtcgtttc atcatctctc gtgacggtaa cggtaccgac 240
   ctgaccctgg aaatgaactc tctgaaaacc gaagacaccg cggaatacta ctgcgcgcag 300
   aactactacg gttctaccta cgactactgg ggtcagggta ccctggttac cgtttct    357
<210> 326
   <211> 357
   <212> DNA
   <213> Artificial Sequence
<220>
   <223> Infliximab VH variant
<400> 326
   gaagttcagc tgaccgaatc tggtggtggt ctggttcagc cgggtggttc tctgcgtctg 60
   tcttgccgtg cgtctggttt catcttctct aaccactaca tgaactgggt tcgtcaggcg 120
   ccgggtaaag gtctggaatg gatcggtgaa atccgttcta aatcttctaa ctctgcgacc 180
   tcttacgcgg aatctgttaa aggtcgtttc atcatctctc gtgacggtaa caaaaacgac 240
   ctgtacctgg aaatgaactc tctgaaaacc gaagacaccg cggaatacta ctgcgcgcgt 300
   aactactacg gttctacccc ggactactgg ggtcagggta ccctggttac cgtttct    357
<210> 327
   <211> 357
   <212> DNA
   <213> Artificial Sequence
<220>
   <223> Infliximab VH variant
<400> 327
   gaagttcagc tgaccgaatc tggtggtggt ctggttcagc cgggtggttc tctgcgtctg 60
   tcttgccgtg cgtctcagtc tatcttctct aaccactaca tgaactgggt tcgtcaggcg 120
   ccgggtaaag gtctggaatg gatcggtgaa atccgttcta aatcttctta cggtgcgacc 180
   tcttacgcgg aatctgttaa aggtcgtttc atcatctctc gtgacgacaa caaaaactct 240
   ctgaccctgg aaatgaactc tctgaaaacc gaagacaccg cggaatacta ctgcgcgcag 300
   aactactacg gttctacccc ggactactgg ggtcagggta ccctggttac cgtttct 357
<210> 328
   <211> 357
   <212> DNA
   <213> Artificial Sequence
<220>
   <223> Infliximab VH variant
<400> 328
   gaagttcagc tgaccgaatc tggtggtggt ctggttcagc cgggtggttc tctgcgtctg 60
   tcttgcgcgg cgtctggttc taccttctct aaccactaca tgaactgggt tcgtcaggcg 120
   ccgggtaaag gtctggaatg gatcggtgaa atccgttcta aatcttctaa ctctgcgacc 180
   cactacgcgg aatctgttaa aggtcgtttc atcatctctc gtgacgacaa cggtacctct 240
   ctgtacctgg aaatgaactc tctgaaaacc gaagacaccg cggaatacta ctgcgcgcgt 300
   aactactacg gttctaccta cgactactgg ggtcagggta ccctggttac cgtttct 357
<210> 329
   <211> 357
   <212> DNA
   <213> Artificial Sequence
<220>
   <223> Infliximab VH variant
<400> 329
   gaagttcagc tggttgaatc tggtggtggt ctggttcagc cgggtggttc tctgcgtctg 60
   tcttgcgcgg cgtctggttc taccttctct aaccactgga tgaactgggt tcgtcaggcg 120
   ccgggtaaag gtctggaatg gatcggtgaa atccgttcta aatctatcta cggtgcgacc 180
   tcttacgcgg aatctgttaa aggtcgtttc atcatctctc gtgacggtaa caaaaactct 240
   ctgtacctgg aaatgaactc tctgaaaacc gaagacaccg cggaatacta ctgcgcgcgt 300
   aactactacg gttctaccta cgactactgg ggtcagggta ccctggttac cgtttct 357
<210> 330
   <211> 357
   <212> DNA
   <213> Artificial Sequence
<220>
   <223> Infliximab VH variant
<400> 330
   gaagttcagc tggttgaatc tggtggtggt ctggttcagc cgggtggttc tctgcgtctg 60
   tcttgccgtg cgtctcagtt caccttctct aaccactaca tgaactgggt tcgtcaggcg 120
   ccgggtaaag gtctggaatg gatcggtgaa atccgttcta aatctatcta ctctgcgacc 180
   tcttacgcgg aatctgttaa aggtcgtttc atcatctctc gtgacgacaa cggtacctct 240
   ctgaccctgg aaatgaactc tctgaaaacc gaagacaccg cggaatacta ctgcgcgcgt 300
   aactactacg gttctaccta cgactactgg ggtcagggta ccctggttac cgtttct 357
<210> 331
   <211> 357
   <212> DNA
   <213> Artificial Sequence
<220>
   <223> Infliximab VH variant
<400> 331
   gaagttcagc tggttgaatc tggtggtggt ctggttcagc cgggtggttc tctgcgtctg 60
   tcttgcgcgg cgtctcagtc tatcttctct aaccactgga tgaactgggt tcgtcaggcg 120
   ccgggtaaag gtctggaatg gatcggtgaa atccgttcta aatctatcaa ctctgcgacc 180
   tcttacgcgg aatctgttaa aggtcgtttc atcatctctc gtgacggtaa cggtaacgac 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 332
   <211> 357
   <212> DNA
   <213> Artificial Sequence
<220>
   <223> Infliximab VH variant
<400> 332
   gaagttcagc tggttgaatc tggtggtggt ctggttcagc cgggtggttc tctgcgtctg 60
   tcttgccgtg cgtctggttc tatcttctct aaccactaca tgaactgggt tcgtcaggcg 120
   ccgggtaaag gtctggaatg gatcggtgaa atccgttcta aatcttctaa ctctgcgacc 180
   tcttacgcgg aatctgttaa aggtcgtttc atcatctctc gtgacggtaa caaaaacgac 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 333
   <211> 357
   <212> DNA
   <213> Artificial Sequence
<220>
   <223> Infliximab VH variant
<400> 333
   gaagttcagc tgaccgaatc tggtggtggt ctggttcagc cgggtggttc tctgcgtctg 60
   tcttgccgtg cgtctcagtc taccttctct aaccactaca tgaactgggt tcgtcaggcg 120
   ccgggtaaag gtctggaatg gatcggtgaa atccgttcta aatcttctaa cggtgcgacc 180
   tcttacgcgg aatctgttaa aggtcgtttc atcatctctc gtgacgacaa cggtaacgac 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 334
   <211> 357
   <212> DNA
   <213> Artificial Sequence
<220>
   <223> Infliximab VH variant
<400> 334
   gaagttcagc tggttgaatc tggtggtggt ctggttcagc cgggtggttc tctgcgtctg 60
   tcttgcgcgg cgtctggttt catcttctct aaccactgga tgaactgggt tcgtcaggcg 120
   ccgggtaaag gtctggaatg gatcggtgaa atccgttcta aatctatcaa cggtgcgacc 180
   cactacgcgg aatctgttaa aggtcgtttc atcatctctc gtgacggtaa caaaaacgac 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 335
   <211> 357
   <212> DNA
   <213> Artificial Sequence
<220>
   <223> Infliximab VH variant
<400> 335
   gaagttcagc tggttgaatc tggtggtggt ctggttcagc cgggtggttc tctgcgtctg 60
   tcttgcgcgg cgtctcagtc tatcttctct aaccactgga tgaactgggt tcgtcaggcg 120
   ccgggtaaag gtctggaatg gatcggtgaa atccgttcta aatctatcaa ctctgcgacc 180
   cactacgcgg aatctgttaa aggtcgtttc atcatctctc gtgacggtaa cggtaccgac 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 336
   <211> 357
   <212> DNA
   <213> Artificial Sequence
<220>
   <223> Infliximab VH variant
<400> 336
   gaagttcagc tggttgaatc tggtggtggt ctggttcagc cgggtggttc tctgcgtctg 60
   tcttgccgtg cgtctcagtt catcttctct aaccactaca tgaactgggt tcgtcaggcg 120
   ccgggtaaag gtctggaatg gatcggtgaa atccgttcta aatctatcta cggtgcgacc 180
   tcttacgcgg aatctgttaa aggtcgtttc atcatctctc gtgacggtaa cggtaacgac 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 337
   <211> 357
   <212> DNA
   <213> Artificial Sequence
<220>
   <223> Infliximab VH variant
<400> 337
   gaagttcagc tgaccgaatc tggtggtggt ctggttcagc cgggtggttc tctgcgtctg 60
   tcttgccgtg cgtctcagtt catcttctct aaccactaca tgaactgggt tcgtcaggcg 120
   ccgggtaaag gtctggaatg gatcggtgaa atccgttcta aatcttctta ctctgcgacc 180
   cactacgcgg aatctgttaa aggtcgtttc atcatctctc gtgacgacaa cggtaactct 240
   ctgtacctgg aaatgaactc tctgaaaacc gaagacaccg cggaatacta ctgcgcgcag 300
   aactactacg gttctaccta cgactactgg ggtcagggta ccctggttac cgtttct 357
<210> 338
   <211> 357
   <212> DNA
   <213> Artificial Sequence
<220>
   <223> Infliximab VH variant
<400> 338
   gaagttcagc tggttgaatc tggtggtggt ctggttcagc cgggtggttc tctgcgtctg 60
   tcttgcgcgg cgtctcagtt caccttctct aaccactaca tgaactgggt tcgtcaggcg 120
   ccgggtaaag gtctggaatg gatcggtgaa atccgttcta aatctatcaa cggtgcgacc 180
   tcttacgcgg aatctgttaa aggtcgtttc atcatctctc gtgacgacaa cggtaccgac 240
   ctgtacctgg aaatgaactc tctgaaaacc gaagacaccg cggaatacta ctgcgcgcgt 300
   aactactacg gttctaccta cgactactgg ggtcagggta ccctggttac cgtttct 357
<210> 339
   <211> 357
   <212> DNA
   <213> Artificial Sequence
<220>
   <223> Infliximab VH variant
<400> 339
   gaagttcagc tggttgaatc tggtggtggt ctggttcagc cgggtggttc tctgcgtctg 60
   tcttgcgcgg cgtctcagtt catcttctct aaccactaca tgaactgggt tcgtcaggcg 120
   ccgggtaaag gtctggaatg gatcggtgaa atccgttcta aatctatcta cggtgcgacc 180
   cactacgcgg aatctgttaa aggtcgtttc atcatctctc gtgacggtaa cggtaactct 240
   ctgaccctgg aaatgaactc tctgaaaacc gaagacaccg cggaatacta ctgcgcgcgt 300
   aactactacg gttctacccc ggactactgg ggtcagggta ccctggttac cgtttct 357
<210> 340
   <211> 357
   <212> DNA
   <213> Artificial Sequence
<220>
   <223> Infliximab VH variant
<400> 340
   gaagttcagc tggttgaatc tggtggtggt ctggttcagc cgggtggttc tctgcgtctg 60
   tcttgcgcgg cgtctcagtt caccttctct aaccactaca tgaactgggt tcgtcaggcg 120
   ccgggtaaag gtctggaatg gatcggtgaa atccgttcta aatctatcaa cggtgcgacc 180
   cactacgcgg aatctgttaa aggtcgtttc atcatctctc gtgacgacaa cggtacctct 240
   ctgaccctgg aaatgaactc tctgaaaacc gaagacaccg cggaatacta ctgcgcgcag 300
   aactactacg gttctaccta cgactactgg ggtcagggta ccctggttac cgtttct 357
<210> 341
   <211> 357
   <212> DNA
   <213> Artificial Sequence
<220>
   <223> Infliximab VH variant
<400> 341
   gaagttcagc tggttgaatc tggtggtggt ctggttcagc cgggtggttc tctgcgtctg 60
   tcttgccgtg cgtctcagtt caccttctct aaccactaca tgaactgggt tcgtcaggcg 120
   ccgggtaaag gtctggaatg gatcggtgaa atccgttcta aatcttctaa cggtgcgacc 180
   tcttacgcgg aatctgttaa aggtcgtttc atcatctctc gtgacggtaa cggtaacgac 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 342
   <211> 357
   <212> DNA
   <213> Artificial Sequence
<220>
   <223> Infliximab VH variant
<400> 342
   gaagttcagc tgaccgaatc tggtggtggt ctggttcagc cgggtggttc tctgcgtctg 60
   tcttgccgtg cgtctcagtc taccttctct aaccactaca tgaactgggt tcgtcaggcg 120
   ccgggtaaag gtctggaatg gatcggtgaa atccgttcta aatcttctaa cggtgcgacc 180
   tcttacgcgg aatctgttaa aggtcgtttc atcatctctc gtgacgacaa cggtaactct 240
   ctgaccctgg aaatgaactc tctgaaaacc gaagacaccg cggaatacta ctgcgcgcag 300
   aactactacg gttctaccta cgactactgg ggtcagggta ccctggttac cgtttct    357
<210> 343
   <211> 357
   <212> DNA
   <213> Artificial Sequence
<220>
   <223> Infliximab VH variant
<400> 343
   gaagttcagc tgaccgaatc tggtggtggt ctggttcagc cgggtggttc tctgcgtctg 60
   tcttgcgcgg cgtctcagtc taccttctct aaccactgga tgaactgggt tcgtcaggcg 120
   ccgggtaaag gtctggaatg gatcggtgaa atccgttcta aatcttctta ctctgcgacc 180
   tcttacgcgg aatctgttaa aggtcgtttc atcatctctc gtgacgacaa caaaaactct 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 344
   <211> 357
   <212> DNA
   <213> Artificial Sequence
<220>
   <223> Infliximab VH variant
<400> 344
   gaagttcagc tgaccgaatc tggtggtggt ctggttcagc cgggtggttc tctgcgtctg 60
   tcttgcgcgg cgtctcagtc taccttctct aaccactgga tgaactgggt tcgtcaggcg 120
   ccgggtaaag gtctggaatg gatcggtgaa atccgttcta aatctatcaa ctctgcgacc 180
   tcttacgcgg aatctgttaa aggtcgtttc atcatctctc gtgacgacaa caaaaacgac 240
   ctgaccctgg aaatgaactc tctgaaaacc gaagacaccg cggaatacta ctgcgcgcgt 300
   aactactacg gttctaccta cgactactgg ggtcagggta ccctggttac tgtttct    357
<210> 345
   <211> 357
   <212> DNA
   <213> Artificial Sequence
<220>
   <223> Infliximab VH variant
<400> 345
   gaagttcagc tgaccgaatc tggtggtggt ctggttcagc cgggtggttc tctgcgtctg 60
   tcttgccgtg cgtctggttc tatcttctct aaccactgga tgaactgggt tcgtcaggcg 120
   ccgggtaaag gtctggaatg gatcggtgaa atccgttcta aatctatcta ctctgcgacc 180
   tcttacgcgg aatctgttaa aggtcgtttc atcatctctc gtgacgacaa cggtacctct 240
   ctgaccctgg aaatgaactc tctgaaaacc gaagacaccg cggaatacta ctgcgcgcag 300
   aactactacg gttctacccc ggactactgg ggtcagggta ccctggttac cgtttct    357
<210> 346
   <211> 357
   <212> DNA
   <213> Artificial Sequence
<220>
   <223> Infliximab VH variant
<400> 346
   gaagttcagc tggttgaatc tggtggtggt ctggttcagc cgggtggttc tctgcgtctg 60
   tcttgccgtg cgtctcagtt catcttctct aaccactgga tgaactgggt tcgtcaggcg 120
   ccgggtaaag gtctggaatg gatcggtgaa atccgttcta aatctatcta cggtgcgacc 180
   cactacgcgg aatctgttaa aggtcgtttc atcatctctc gtgacgacaa caaaaacgac 240
   ctgtacctgg aaatgaactc tctgaaaacc gaagacaccg cggaatacta ctgcgcgcgt 300
   aactactacg gttctacccc ggactactgg ggtcagggta ccctggttac cgtttct    357
<210> 347
   <211> 357
   <212> DNA
   <213> Artificial Sequence
<220>
   <223> Infliximab VH variant
<400> 347
   gaagttcagc tggttgaatc tggtggtggt ctggttcagc cgggtggttc tctgcgtctg 60
   tcttgcgcgg cgtctggttt catcttctct aaccactgga tgaactgggt tcgtcaggcg 120
   ccgggtaaag gtctggaatg gatcggtgaa atccgttcta aatctatcta ctctgcgacc 180
   tcttacgcgg aatctgttaa aggtcgtttc atcatctctc gtgacggtaa cggtacctct 240
   ctgaccctgg aaatgaactc tctgaaaacc gaagacaccg cggaatacta ctgcgcgcgt 300
   aactactacg gttctaccta cgactactgg ggtcagggta ccctggttac cgtttct 357
<210> 348
   <211> 357
   <212> DNA
   <213> Artificial Sequence
<220>
   <223> Infliximab VH variant
<400> 348
   gaagttcagc tggttgaatc tggtggtggt ctggttcagc cgggtggttc tctgcgtctg 60
   tcttgccgtg cgtctcagtt catcttctct aaccactaca tgaactgggt tcgtcaggcg 120
   ccgggaaaag gtctggaatg gatcggtgaa atccgttcta aatcttctta ctctgcgacc 180
   tcttacgcgg aatctgttaa aggtcgtttc atcatctctc gtgacggtaa caaaaacgac 240
   ctgtacctgg aaatgaattc tctgaaaacc gaagacaccg cggaatacta ctgcgcgcag 300
   aactactacg gttctaccta cgactactgg ggtcagggta ccctggttac cgtttct 357
<210> 349
   <211> 357
   <212> DNA
   <213> Artificial Sequence
<220>
   <223> Infliximab VH variant
<400> 349
   gaagttcagc tgaccgaatc tggtggtggt ctggttcagc cgggtggttc tctgcgtctg 60
   tcttgccgtg cgtctggttt catcttctct aaccactgga tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacgacaa cggtaacgac 240
   ctgtacctgg aaatgaactc tttgaaaacc gaagacaccg cggaatacta ctgcgcgcag 300
   aactactacg gttctacccc ggactactgg ggtcagggta ccctggttac cgtttct 357
<210> 350
   <211> 357
   <212> DNA
   <213> Artificial Sequence
<220>
   <223> Infliximab VH variant
<400> 350
   gaagttcagc tggttgaatc tggtggtggt ctggttcagc cgggtggttc tctgcgtctg 60
   tcttgcgcgg cgtctcagtt catcttctct aaccactaca tgaactgggt tcgtcaggcg 120
   ccgggtaaag gtctggaatg gatcggtgaa atccgttcta aatcttctta ctctgcgacc 180
   cactacgcgg aatctgttaa aggtcgtttc atcatctctc gtgacggtaa caaaaacgac 240
   ctgaccctgg aaatgaactc tctgaaaacc gaagacaccg cggaatacta ctgcgcgcgt 300
   aactactacg gttctaccta cgactactgg ggtcagggta ccctggttac cgtttct 357
<210> 351
   <211> 357
   <212> DNA
   <213> Artificial Sequence
<220>
   <223> Infliximab VH variant
<400> 351
   gaagttcagc tggttgaatc tggtggtggt ctggttcagc cgggtggttc tctgcgtctg 60
   tcttgcgcgg cgtctcagtt catcttctct aaccactgga tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacgacaa caaaaccgac 240
   ctgaccctgg aaatgaactc tctgaaaacc gaagacaccg cggaatacta ctgcgcgcag 300
   aactattacg gttctacccc ggactactgg ggtcagggta ccctggttac cgtttct    357
<210> 352
   <211> 357
   <212> DNA
   <213> Artificial Sequence
<220>
   <223> Infliximab VH variant
<400> 352
   gaagttcagc tgaccgaatc tggtggtggt ctggttcagc cgggtggttc tctgcgtctg 60
   tcttgcgcgg cgtctggttc tatcttctct aaccactaca tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacggtaa cggtaccgac 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 353
   <211> 357
   <212> DNA
   <213> Artificial Sequence
<220>
   <223> Infliximab VH variant
<400> 353
   gaagttcagc tggttgaatc tggtggtggt ctggttcagc cgggtggttc tctgcgtctg 60
   tcttgcgcgg cgtctcagtt caccttctct aaccactaca tgaactgggt tcgtcaggcg 120
   ccgggtaaag gtctggaatg gatcggtgaa atccgttcta aatcttctta cggtgcgacc 180
   cactacgcgg aatctgttaa aggtcgtttc atcatctctc gtgacggtaa caaaaccgac 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 354
   <211> 357
   <212> DNA
   <213> Artificial Sequence
<220>
   <223> Infliximab VH variant
<400> 354
   gaagttcagc tggttgaatc tggtggtggt ctggttcagc cgggtggttc tctgcgtctg 60
   tcttgcgcgg cgtctcagtt catcttctct aaccactaca tgaactgggt tcgtcaggcg 120
   ccgggtaaag gtctggaatg gatcggtgaa atccgttcta aatctatcaa ctctgcgacc 180
   tcttacgcgg aatctgttaa aggtcgtttc atcatctctc gtgacggtaa caaaaccgac 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 355
   <211> 357
   <212> DNA
   <213> Artificial Sequence
<220>
   <223> Infliximab VH variant
<400> 355
   gaagttcagc tggttgaatc tggtggtggt ctggttcagc cgggtggttc tctgcgtctg 60
   tcttgcgcgg cgtctcagtt caccttctct aaccactgga tgaactgggt tcgtcaggcg 120
   ccgggtaaag gtctggaatg gatcggtgaa atccgttcta aatcttctta cggtgcgacc 180
   cactacgcgg aatctgttaa aggtcgtttc atcatctctc gtgacgacaa caaaaccgac 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 356
   <211> 357
   <212> DNA
   <213> Artificial Sequence
<220>
   <223> Infliximab VH variant
<400> 356
   gaagttcagc tgaccgaatc tggtggtggt ctggttcagc cgggtggttc tctgcgtctg 60
   tcttgcgcgg cgtctcagtc taccttctct aaccactgga tgaactgggt tcgtcaggcg 120
   ccgggtaaag gtctggaatg gatcggtgaa atccgttcta aatctatcaa cggtgcgacc 180
   tcttacgcgg aatctgttaa aggtcgtttc atcatctctc gtgacggtaa caaaacctct 240
   ctgtacctgg aaatgaactc tctgaaaacc gaagacaccg cggaatacta ctgcgcgcgt 300
   aactactacg gttctacccc ggactactgg ggtcagggta ccctggttac cgtttct    357
<210> 357
   <211> 357
   <212> DNA
   <213> Artificial Sequence
<220>
   <223> Infliximab VH variant
<400> 357
   gaagttcagc tgaccgaatc tggtggtggt ctggttcagc cgggtggttc tctgcgtctg 60
   tcttgcgcgg cgtctggttt catcttctct aaccactaca tgaactgggt tcgtcaggcg 120
   ccgggtaaag gtctggaatg gatcggtgaa atccgttcta aatctatcaa cggtgcgacc 180
   tcttacgcgg aatctgttaa aggtcgtttc atcatctctc gtgacgacaa cggtaacgac 240
   ctgaccctgg aaatgaactc tctgaaaacc gaagacaccg cggaatacta ctgcgcgcag 300
   aactactacg gttctacccc ggactactgg ggtcagggta ccctggttac cgtttct    357
<210> 358
   <211> 357
   <212> DNA
   <213> Artificial Sequence
<220>
   <223> Infliximab VH variant
<400> 358
   gaagttcagc tggttgaatc tggtggtggt ctggttcagc cgggtggttc tctgcgtctg 60
   tcttgcgcgg cgtctggttc taccttctct aaccactgga tgaactgggt tcgtcaggcg 120
   ccgggtaaag gtctggaatg gatcggtgaa atccgttcta aatctatcaa ctctgcgacc 180
   cactacgcgg aatctgttaa aggtcgtttc atcatctctc gtgacgacaa cggtacctct 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 359
   <211> 357
   <212> DNA
   <213> Artificial Sequence
<220>
   <223> Infliximab VH variant
<400> 359
   gaagttcagc tggttgaatc tggtggtggt ctggttcagc cgggtggttc tctgcgtctg 60
   tcttgcgcgg cgtctcagtc taccttctct aaccactaca tgaactgggt tcgtcaggcg 120
   ccgggtaaag gtctggaatg gatcggtgaa atccgttcta aatctatcta ctctgcgacc 180
   tcttacgcgg aatctgttaa aggtcgtttc atcatctctc gtgacgacaa cggtaactct 240
   ctgtacctgg aaatgaactc tctgaaaacc gaagacaccg cggaatacta ctgcgcgcag 300
   aactactacg gttctacccc ggactactgg ggtcagggta ccctggttac cgtttct    357
<210> 360
   <211> 357
   <212> DNA
   <213> Artificial Sequence
<220>
   <223> Infliximab VH variant
<400> 360
   gaagttcagc tgaccgaatc tggtggtggt ctggttcagc cgggtggttc tctgcgtctg 60
   tcttgcgcgg cgtctggttt caccttctct aaccactgga tgaactgggt tcgtcaggcg 120
   ccgggtaaag gtctggaatg gatcggtgaa atccgttcta aatctatcta cggtgcgacc 180
   cactacgcgg aatctgttaa aggtcgtttc atcatctctc gtgacggtaa caaaaacgac 240
   ctgaccctgg aaatgaactc tctgaaaacc gaagacaccg cggaatacta ctgcgcgcag 300
   aactactacg gttctacccc ggactactgg ggtcagggta ccctggttac cgtttct    357
<210> 361
   <211> 357
   <212> DNA
   <213> Artificial Sequence
<220>
   <223> Infliximab VH variant
<400> 361
   gaagttcagc tgaccgaatc tggtggtggt ctggttcagc cgggtggttc actgcgtctg 60
   tcttgccgtg cgtctcagtc taccttctct aaccactaca tgaactgggt tcgtcaggcg 120
   ccgggtaaag gtctggaatg gatcggtgaa atccgttcta aatcttctaa ctctgcgacc 180
   tcttacgcgg aatctgttaa aggtcgtttc atcatctctc gtgacggtaa caaaaccgac 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 362
   <211> 357
   <212> DNA
   <213> Artificial Sequence
<220>
   <223> Infliximab VH variant
<400> 362
   gaagttcagc tggttgaatc tggtggtggt ctggttcagc cgggtggttc tctgcgtctg 60
   tcttgccgtg cgtctggttc taccttctct aaccactaca tgaactgggt tcgtcaggcg 120
   ccgggtaaag gtctggaatg gatcggtgaa atccgttcta aatcttctta ctctgcgacc 180
   tcttacgcgg aatctgttaa aggtcgtttc atcatctctc gtgacgacaa caaaaccgac 240
   ctgaccctgg aaatgaactc tctgaaaacc gaagacaccg cggaatacta ctgcgcgcag 300
   aactactacg gttctacccc ggactactgg ggtcagggta ccctggttac cgtttct    357
<210> 363
   <211> 357
   <212> DNA
   <213> Artificial Sequence
<220>
   <223> Infliximab VH variant
<400> 363
   gaagttcagc tggttgaatc tggtggtggt ctggttcagc cgggtggttc tctgcgtctg 60
   tcttgccgtg cgtctggttt catcttctct aaccactgga tgaactgggt tcgtcaggcg 120
   ccgggtaaag gtctggaatg gatcggtgaa atccgttcta aatctatcaa cggtgcgacc 180
   cactacgcgg aatctgttaa aggtcgtttc atcatctctc gtgacgacaa caaaacctct 240
   ctgaccctgg aaatgaactc tctgaaaacc gaagacaccg cggaatacta ctgcgcgcag 300
   aactactacg gttctaccta cgactactgg ggtcagggta ccctggttac cgtttct    357
<210> 364
   <211> 357
   <212> DNA
   <213> Artificial Sequence
<220>
   <223> Infliximab VH variant
<400> 364
   gaagttcagc tgaccgaatc tggtggtggt ctggttcagc cgggtggttc tctgcgtctg 60
   tcttgccgtg cgtctcagtc taccttctct aaccactgga tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacggtaa cggtaacgac 240
   ctgtacctgg aaatgaactc tctgaaaacc gaagacaccg cggaatacta ctgcgcgcag 300
   aactactatg gttctacccc ggactactgg ggtcagggta ccctggttac cgtttct    357
<210> 365
   <211> 357
   <212> DNA
   <213> Artificial Sequence
<220>
   <223> Infliximab VH variant
<400> 365
   gaagttcagc tgaccgaatc tggtggtggt ctggttcagc cgggtggttc tctgcgtctg 60
   tcttgcgcgg cgtctcagtc taccttctct aaccactaca tgaactgggt tcgtcaggcg 120
   ccgggtaaag gtctggaatg gatcggtgaa atccgttcta aatcttctaa cggtgcgacc 180
   tcttacgcgg aatctgttaa aggtcgtttc atcatctctc gtgacgacaa caaaaacgac 240
   ctgaccctgg aaatgaactc tctgaaaacc gaagacaccg cggaatacta ctgcgcgcag 300
   aactactacg gttctaccta cgactactgg ggtcagggta ccctggttac ggtttct    357
<210> 366
   <211> 357
   <212> DNA
   <213> Artificial Sequence
<220>
   <223> Infliximab VH variant
<400> 366
   gaagttcagc tggttgaatc tggtggtggt ctggttcagc cgggtggttc tctgcgtctg 60
   tcttgcgcgg cgtctggttt caccttctct aaccactaca tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacggtaa cggtaactct 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 367
   <211> 357
   <212> DNA
   <213> Artificial Sequence
<220>
   <223> Infliximab VH variant
<400> 367
   gaagttcagc tgaccgaatc tggtggtggt ctggttcagc cgggtggttc tctgcgtctg 60
   tcttgcgcgg cgtctcagtt caccttctct aaccactgga tgaactgggt tcgtcaggcg 120
   ccgggtaaag gtctggaatg gatcggtgaa atccgttcta aatcttctaa cggtgcgacc 180
   cactacgcgg aatctgttaa aggtcgtttc atcatctctc gtgacgacaa cggtacctct 240
   ctgaccctgg aaatgaactc tctgaaaacc gaagacaccg cggaatacta ctgcgcgcgt 300
   aactactacg gttctacccc ggactactgg ggtcagggta ccctggttac cgtttct 357
<210> 368
   <211> 357
   <212> DNA
   <213> Artificial Sequence
<220>
   <223> Infliximab VH variant
<400> 368
   gaagttcagc tggttgaatc tggtggtggt ctggttcagc cgggtggttc tctgcgtctg 60
   tcttgccgtg cgtctggttt catcttctct aaccactaca tgaactgggt tcgtcaggcg 120
   ccgggtaaag gtctggaatg gatcggtgaa atccgttcta aatcttctaa ctctgcgacc 180
   cactacgcgg aatctgttaa aggtcgtttc atcatctctc gtgacgacaa caaaacctct 240
   ctgaccctgg aaatgaactc tctgaaaacc gaagacaccg cggaatacta ctgcgcgcag 300
   aactactacg gttctacccc ggactactgg ggtcagggta ccctggttac cgtttct 357
<210> 369
   <211> 357
   <212> DNA
   <213> Artificial Sequence
<220>
   <223> Infliximab VH variant
<400> 369
   gaagttcagc tggttgaatc tggtggtggt ctggttcagc cgggtggttc tctgcgtctg 60
   tcttgcgcgg cgtctggttt caccttctct aaccactgga tgaactgggt tcgtcaggcg 120
   ccgggtaaag gtctggaatg gatcggtgaa atccgttcta aatcttctta ctctgcgacc 180
   cactacgcgg aatctgttaa aggtcgtttc atcatctctc gtgacgacaa caaaaactct 240
   ctgaccctgg aaatgaactc tctgaaaacc gaagacaccg cggaatacta ctgcgcgcag 300
   aactactacg gttctacccc ggactactgg ggtcagggta ccctggttac cgtttct 357
<210> 370
   <211> 357
   <212> DNA
   <213> Artificial Sequence
<220>
   <223> Infliximab VH variant
<400> 370
   gaagttcagc tgaccgaatc tggtggtggt ctggttcagc cgggtggttc tctgcgtctg 60
   tcttgcgcgg cgtctggttt caccttctct aaccactaca tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacggtaa cggtaccgac 240
   ctgtacctgg aaatgaactc tctgaaaacc gaagacaccg cggaatacta ctgcgcgcag 300
   aactactacg gttctacccc ggactactgg ggtcagggta ccctggttac cgtttct 357
<210> 371
   <211> 357
   <212> DNA
   <213> Artificial Sequence
<220>
   <223> Infliximab VH variant
<400> 371
   gaagttcagc tgaccgaatc tggtggtggt ctggttcagc cgggtggttc tctgcgtctg 60
   tcttgcgcgg cgtctcagtc taccttctct aaccactaca tgaactgggt tcgtcaggcg 120
   ccgggtaaag gtctggaatg gatcggtgaa atccgttcta aatcttctta cggtgcgacc 180
   tcttacgcgg aatctgttaa aggtcgtttc atcatctctc gtgacggtaa cggtacctct 240
   ctgtacttgg aaatgaactc tctgaaaacc gaagacaccg cggaatacta ctgcgcgcgt 300
   aactactacg gttctaccta cgactactgg ggtcagggta ccctggttac cgtttct    357
<210> 372
   <211> 357
   <212> DNA
   <213> Artificial Sequence
<220>
   <223> Infliximab VH variant
<400> 372
   gaagttcagc tggttgaatc tggcggtggt ctggttcagc cgggtggttc tctgcgtctg 60
   tcttgcgcgg cgtctcagtt catcttctct aaccactaca tgaactgggt tcgtcaggcg 120
   ccgggtaaag gtctggaatg gatcggtgaa atccgttcta aatctatcaa ctctgcgacc 180
   cactacgcgg aatctgttaa aggtcgtttc atcatctctc gtgacggtaa cggtaccgac 240
   ctgtacctgg aaatgaactc tctgaaaacc gaagacaccg cggaatacta ctgcgcgcgt 300
   aactactacg gttctaccta cgactactgg ggtcagggta ccctggttac cgtttct    357
<210> 373
   <211> 357
   <212> DNA
   <213> Artificial Sequence
<220>
   <223> Infliximab VH variant
<400> 373
   gaagttcagc tgaccgaatc tggtggtggt ctggttcagc cgggtggttc tctgcgtctg 60
   tcttgccgtg cgtctcagtc taccttctct aaccactgga tgaactgggt tcgtcaggcg 120
   ccgggtaaag gtctggaatg gatcggtgaa atccgttcta aatctatcta ctctgcgacc 180
   tcttacgcgg aatctgttaa aggtcgtttc atcatctctc gtgacggtaa cggtaacgac 240
   ctgtacctgg aaatgaactc tctgaaaacc gaagacaccg cggaatacta ctgcgcgcag 300
   aactactacg gttctacccc ggactactgg ggtcagggta ccctggttac cgtttct    357
<210> 374
   <211> 357
   <212> DNA
   <213> Artificial Sequence
<220>
   <223> Infliximab VH variant
<400> 374
   gaagttcagc tgaccgaatc tggtggtggt ctggttcagc cgggtggttc tctgcgtctg 60
   tcttgcgcgg cgtctggttc taccttctct aaccactaca tgaactgggt tcgtcaggcg 120
   ccgggtaaag gtctggaatg gatcggtgaa atccgttcta aatctatcta ctctgcgacc 180
   tcttacgcgg aatctgttaa aggtcgtttc atcatctctc gtgacgacaa cggtaactct 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 375
   <211> 357
   <212> DNA
   <213> Artificial Sequence
<220>
   <223> Infliximab VH variant
<400> 375
   gaagttcagc tgaccgaatc tggtggtggt ctggttcagc cgggtggttc tctgcgtctg 60
   tcttgcgcgg cgtctggttt caccttctct aaccactaca tgaactgggt tcgtcaggcg 120
   ccgggtaaag gtctggaatg gatcggtgaa atccgttcta aatcttctta cggtgcgacc 180
   tcttacgcgg aatctgttaa aggtcgtttc atcatctctc gtgacggtaa cggtaccgac 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 376
   <211> 357
   <212> DNA
   <213> Artificial Sequence
<220>
   <223> Infliximab VH variant
<400> 376
   gaagttcagc tggttgaatc tggtggtggt ctggttcagc cgggtggttc tctgcgtctg 60
   tcttgcgcgg cgtctggttt catcttctct aaccactaca tgaactgggt tcgtcaggcg 120
   ccgggtaaag gtctggaatg gatcggtgaa atccgttcta aatctatcta cggtgcgacc 180
   tcttacgcgg aatctgttaa aggtcgtttc atcatctctc gtgacgacaa caaaaacgac 240
   ctgtacctgg aaatgaactc tctgaaaacc gaagacaccg cggaatacta ctgcgcgcag 300
   aactactacg gttctaccta cgactactgg ggtcagggta ccctggttac cgtttct    357
<210> 377
   <211> 357
   <212> DNA
   <213> Artificial Sequence
<220>
   <223> Infliximab VH variant
<400> 377
   gaagttcagc tgaccgaatc tggtggtggt ctggttcagc cgggtggttc tctgcgtctg 60
   tcttgcgcgg cgtctcagtt caccttctct aaccactgga tgaactgggt tcgtcaggcg 120
   ccgggtaaag gtctggaatg gatcggtgaa atccgttcta aatctatcta ctctgcgacc 180
   cactacgcgg aatctgttaa aggtcgtttc atcatctctc gtgacgacaa cggtacctct 240
   ctgaccctgg aaatgaactc tctgaaaacc gaagacaccg cggaatacta ctgcgcgcgt 300
   aactactacg gttctacccc ggactactgg ggtcagggta ccctggttac cgtttct    357
<210> 378
   <211> 357
   <212> DNA
   <213> Artificial Sequence
<220>
   <223> Infliximab VH variant
<400> 378
   gaagttcagc tggttgaatc tggtggtggt ctggttcagc cgggtggttc tctgcgtctg 60
   tcttgccgtg cgtctggttt catcttctct aaccactgga tgaactgggt tcgtcaggcg 120
   ccgggtaaag gtctggaatg gatcggtgaa atccgttcta aatctatcaa ttctgcgacc 180
   tcttacgcgg aatctgttaa aggtcgtttc atcatctctc gtgacggtaa caaaaccgac 240
   ctgaccctgg aaatgaactc tctgaaaacc gaagacaccg cggaatacta ctgcgcgcgt 300
   aactactacg gttctaccta cgactactgg ggtcagggta ccctggttac cgtttct    357
<210> 379
   <211> 107
   <212> PRT
   <213> Mus musculus
<400> 379
   Asp Ile Leu Leu Thr Gln Ser Pro Ala Ile Leu Ser Val Ser Pro Gly
   1               5                   10                  15
   Glu Arg Val Ser Phe Ser Cys Arg Ala Ser Gln Phe Val Gly Ser Ser
               20                  25                  30
   Ile His Trp Tyr Gln Gln Arg Thr Asn Gly Ser Pro Arg Leu Leu Ile
           35                  40                  45
   Lys Tyr Ala Ser Glu Ser Met Ser Gly Ile Pro Ser Arg Phe Ser Gly
       50                  55                  60
   Ser Gly Ser Gly Thr Asp Phe Thr Leu Ser Ile Asn Thr Val Glu Ser
   65                  70                  75                  80
   Glu Asp Ile Ala Asp Tyr Tyr Cys Gln Gln Ser His Ser Trp Pro Phe
                   85                  90                  95
   Thr Phe Gly Ser Gly Thr Asn Leu Glu Val Lys
               100                 105
<210> 380
   <211> 107
   <212> PRT
   <213> Artificial Sequence
<220>
   <223> Infliximab VL variant
<400> 380
   Glu Ile Val Leu Thr Gln Ser Pro Asp Phe Gln Ser Val Thr Pro Lys
    1               5                  10                  15
   Glu Lys Val Thr Ile Thr Cys Arg Ala Ser Gln Phe Val Gly Ser Ser
               20                  25                  30
   Ile His Trp Tyr Gln Gln Lys Pro Asp Gln Ser Pro Lys Leu Leu Ile
           35                  40                  45
   Lys Tyr Ala Ser Glu Ser Met Ser Gly Val Pro Ser Arg Phe Ser Gly
       50                  55                  60
   Ser Gly Ser Gly Thr Asp Phe Thr Phe Thr Ile Ser Ser Leu Glu Ala
   65                  70                  75                  80
   Glu Asp Ala Ala Thr Tyr Tyr Cys Gln Gln Ser His Ser Trp Pro Phe
                   85                  90                  95
   Thr Phe Gly Pro Gly Thr Lys Val Asp Ile Lys
               100                 105
<210> 381
   <211> 321
   <212> DNA
   <213> Artificial Sequence
<220>
   <223> Infliximab VL variant
<400> 381
   gaaatcgttc tgacccagtc tccggacttc cagtctgtta ccccgaaaga aaaagttacc 60
   atcacctgcc gtgcgtctca gttcgttggt tcttctatcc actggtacca gcagaaaccg 120
   gaccagtctc cgaaactgct gatcaaatac gcgtctgaat ctatgtctgg tgttccgtct 180
   cgtttctctg gttctggttc tggtaccgac ttcaccttca ccatctcttc tctggaagcg 240
   gaagacgcgg cgacctacta ctgccagcag tctcactctt ggccgttcac cttcggtccg 300
   ggtaccaaag ttgacatcaa a                                           321
<210> 382
   <211> 112
   <212> PRT
   <213> Homo sapiens
<400> 382
   Ser Ala Ser Thr Lys Gly Pro Ser Val Phe Pro Leu Ala Pro Ser Ser
    1               5                  10                  15
   Lys Ser Thr Ser Gly Gly Thr Ala Ala Leu Gly Cys Leu Val Lys Asp
               20                  25                  30
   Tyr Phe Pro Glu Pro Val Thr Val Ser Trp Asn Ser Gly Ala Leu Thr
           35                  40                  45
   Ser Gly Val His Thr Phe Pro Ala Val Leu Gln Ser Ser Gly Leu Tyr
       50                  55                  60
   Ser Leu Ser Ser Val Val Thr Val Pro Ser Ser Ser Leu Gly Thr Gln
   65                  70                  75                  80
   Thr Tyr Ile Cys Asn Val Asn His Lys Pro Ser Asn Thr Lys Val Asp
                   85                  90                  95
   Lys Lys Ala Glu Pro Lys Ser Cys Asp Lys Thr His Thr Cys Pro Pro
               100                 105                 110
<210> 383
   <211> 107
   <212> PRT
   <213> Homo sapiens
<400> 383

## Description

### Field of the Invention

The present invention relates to anti-TNFα antibodies and their use as therapeutics.

### Background of the Invention

Efficacy of a therapeutic protein can be limited by an unwanted immune reaction to the therapeutic protein. For monoclonal antibodies, a number of techniques have been developed in attempts to reduce the human anti-murine antibody (HAMA) response. In general, these approaches aim to reduce the mouse genetic information in the final antibody construct while increasing the human genetic information. Examples of such approaches are chimeric, humanized and fully human monoclonal antibodies (mAbs).

Chimeric antibodies contain regions derived from different animal species, such as a murine immunoglobulin (Ig) variable region and a human Ig constant region. Infliximab (sold under the brand name REMICADE®) is a chimeric IgG1κ monoclonal antibody that specifically binds human tumor necrosis factor-α (TNFα). Infliximab has human constant regions and murine variable regions. The heavy chain variable region (V_{H}) of infliximab has 119 amino acids; the light chain variable region (V_{L}) has 107 amino acids. *See,* U.S. Pat. Nos. 6,277,969, 6,284,471, 6,790,444, and 6,835,823.

TNFα can be produced by a wide variety of cells, but activated macrophages constitute the most abundant source of this factor (Vassalli, Ann. Rev. Immunol. 10: 411-452 (1992)). TNFα is a soluble homotrimer of 17kD protein subunits (Smith, et al., J. Biol. Chem. 262: 6951-6954 (1987)). A membrane-bound 26kD precursor form of TNFα also exists (Kriegler, et al., Cell 53: 45-53 (1988)).

TNFα causes pro-inflammatory actions which result in tissue injury, such as inducing procoagulant activity on vascular endothelial cells increasing the adherence of neutrophils and lymphocytes, and stimulating the release of platelet activating factor from macrophages, neutrophils and vascular endothelial cells (Pober, et al., J. Immunol. 136: 1680-1687 (1986); Pober, et al., J. Immunol. 138: 3319-3324 (1987); Camussi, et al., J. Exp. Med. 166: 1390-1404 (1987)).

The numerous biological effects of TNFα and the closely related cytokine, TNFβ (also known as lymphotoxin), are mediated by two TNF transmembrane receptors, the p55 and p75 receptors (Hohmann, et al., J. Biol. Chem. 264: 14927-14934 (1989); Engelmann, et al., J. Biol. Chem. 265: 1531-1536 (1990)). Extracellular domains of TNF receptors derived by proteolytic cleavage inhibit TNF functions (Kohno, et al., Proc. Natl. Acad. Sci. U.S.A. 87: 8331-8335 (1990)). TNFα is associated with infections, immune disorders, neoplastic pathologies, autoimmune pathologies and graft-versus host pathologies (Cerami, et al., Immunol. Today 9: 28-31 (1988); Oliff, et al., Cell 50: 555-563 (1987); Piguet, et al., J. Exp. Med. 166: 1280-1289 (1987)). Dysregulation and, in particular, overproduction of TNFα has been implicated in a variety of human diseases including sepsis, cerebral malaria, and autoimmune diseases such as multiple sclerosis, rheumatoid arthritis (RA), systemic lupus erythematosus, and Crohn's disease, as well as cancer (reviewed in Zhang and Tracey, The Cytokine Handbook, Thomson AW (ed). pp 517-548 (1998)). TNFα can mediate cachexia in cancer, infectious pathology, and other catabolic states (reviewed in Tracey, et al., Ann. N. Y. Acad. Sci. 587: 325-331 (1990)).

Neutralizing anti-TNFα antibodies that inhibit TNFα activities are useful in treating and/or diagnosing TNFα-mediated diseases. Infliximab has been approved in the United States for treatment of ankylosing spondylitis, Crohn's disease, psoriatic arthritis and rheumatoid arthritis. It can also effectively treat other disorders or symptoms of various immune and autoimmune pathologies as well as inflammatory diseases, such as systemic lupus erythematosus, thyroidosis, graft versus host disease, scleroderma, diabetes mellitus, Grave's disease, sarcoidosis, chronic inflammatory bowel disease, ulcerative colitis, disseminated intravascular coagulation, atherosclerosis and Kawasaki's pathology. *See*, U.S. Pat. Nos. 5,656,272, 5,698,195, and 5,919,452, all of which are incorporated herein by reference.

It has been reported that antibodies to infliximab have been observed in some treated patients (Targan et al., N. Engl. J. Med. 337: 1029-1035 (1997); Maini et al., Arthritis Rheum. 41: 1552-1563 (1998)). Accordingly, in order to potentially diminish the incidence of anti-infliximab antibodies in treated patients, it is desirable to reduce the amount of murine amino sequences present in the molecule.

WO 03/042247 describes the modification of antibodies reactive to TNF alpha to result in anti-TNF alpha antibodies that are less immunogenic than the non-modified parental antibody when used *in vitro.*

### Brief Description of the Drawings

Fig. 1 shows the alignment of V_{H} regions of the TNFα antibody C7 (SEQ ID NO: 3) and infliximab (SEQ ID NO: 1). Identical amino acids are denoted by a "-." Conservative amino acid changes are shown in bold.
Fig. 2 shows the alignment of V_{L} regions of C7 (SEQ ID NO: 380) and infliximab (SEQ ID NO: 379). Identical amino acids are denoted by a "-." Conservative amino acid changes are shown in bold.
Fig. 3 shows the ELISA titration curves for C7 and infliximab.

### Summary of the Invention

In one aspect, the invention provides an antibody capable of binding to TNF alpha, wherein the antibody comprises: (a) a polypeptide comprising a polypeptide having the sequence shown in SEQ ID NO: 2; and (b) a polypeptide comprising a polypeptide having the sequence shown in SEQ ID NO: 380, wherein said antibody binds TNF alpha with affinity higher than that of infliximab as determined using a Biacore assay, and wherein the infliximab V_{H} region has the sequence shown in SEQ ID NO: 1 and the infliximab V_{L} region has the sequence shown in SEQ ID NO: 379.

In another aspect, the invention provides a biologically active fragment of an antibody of the invention.

In another aspect, the invention provides a cell line expressing an antibody of the invention or a biologically active fragment of the invention.

In another aspect, the invention provides a pharmaceutical composition comprising an effective amount of an antibody of the invention or of at least one biologically active fragment of the invention and a pharmaceutically acceptable carrier or diluent.

One aspect of the disclosure is a polypeptide comprising a polypeptide having the sequence shown in SEQ ID NO: 2.

Another aspect of the disclosure is a polypeptide comprising a polypeptide having the sequence shown in SEQ ID NOs: 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189 or 190.

Another aspect of the disclosure is a polynucleotide comprising a polynucleotide having the sequence shown in SEQ ID NOs: 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377 or 378 or a complementary sequence.

Another aspect of the disclosure is a polynucleotide comprising a polynucleotide encoding the amino acid sequence shown in SEQ ID NOs: 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189 or 190.

### Detailed Description of the Invention

Single letter amino acid codes are used herein as understood by those skilled in the art.

The term "immunoglobulin" as used herein refers to a protein consisting of one or more polypeptides substantially encoded by immunoglobulin genes. The term "antibody" as used herein refers to a protein of the immunoglobulin family that is capable of interacting or otherwise associating with an antigen, as well as biologically active fragments, regions or derivatives thereof, provided by any known technique, such as, but not limited to enzymatic cleavage (*e.g.,* by papain or pepsin digestion), peptide synthesis or recombinant techniques. Examples of biologically active fragments regions of antibodies include, but are not limited to, Fab, Fab', F(ab')₂ and Fv. These fragments lack the Fc fragment of an intact antibody, clear more rapidly from the circulation, and can have less non-specific tissue binding than an intact antibody. The term "derivatives" includes modified antibodies that functionally resemble immunoglobulin molecules. An exemplary modification can be the addition of a cytotoxic protein such as a bacterial toxin.
The term "antigen" as used herein refers to a substance that is capable of interacting with the antibody and in the context of the present invention is meant to be TNFα. The TNFα may be soluble TNFα or membrane associated TNFα. The term "TNFα antibodies" as used herein includes antibodies capable of binding portions of TNF and inhibiting the binding of TNFα to TNF receptors.

The present invention provides TNFα antibodies that bind to TNFα and have fewer murine-derived amino acid residues than infliximab. The disclosure also provides nucleic acids encoding the TNFα antibodies, vectors containing these nucleic acids and methods of making and using the TNFα antibodies. The invention also provides host cells and compositions.

### TNFα antibody polypeptides and compositions

The present invention generally relates to a TNFα antibody as recited in claim 1 comprising a polypeptide having the sequence shown in Formula I (SEQ ID NO: 2):
EVQLX₅ESGGG LVQPGGSLRL SCX₂₃ASX₂₆X₂₇X₂₈FS NHX₃₃MNWVRQA PGKGLEWIGE IRSKSX₅₆X₅₇X₅₈AT X₆₁YAESVKGRF IISRDX₇₆NX₇₈X₇₉X₈₀ LX₈₂LEMNSLKT EDTAEYYCAX₁₀₀ NYYGSTX₁₀₇DYW GQGTLVTVS (I)
wherein,
X₅ is V or T; X₂₃ is A or R; X₂₆ is G or Q; X₂₇ is F or S; X₂₈ is I or T; X₃₃ is W or Y; X₅₆ is I or S; X₅₇ is N or Y; X₅₈ is S or G; X₆₁ is H or S; X₇₆ is D or G; X₇₈ is K or G; X₇₉ is N or T; X₈₀ is S or D; X₈₂ is Y or T; X₁₀₀ is R or Q; and X₁₀₇ is Y or P.

More particularly, the present invention relates to antibodies against TNFα that are less likely to generate anti-infliximab antibodies in humans.

The variable regions of the TNFα antibodies have significant identities with human immunoglobulin sequences or human immunoglobulin germline sequences in the framework regions. Human immunoglobulin sequences can be found in databases such as that of NCBI (http://ncbi.nlm.nih.gov/). Human immunoglobulin germline sequences can be found at, for example, http://vbase.mrc-cpe.cam.ac.uk/. Exemplary heavy chain variable region amino acid sequences are shown in SEQ ID NOs: 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189 or 190. An exemplary light chain variable region sequence is shown in SEQ ID NO: 380.

The heavy chain constant region can be derived from any of the known classes or isotypes of human heavy chains, including IgA, IgD, IgE, IgG, IgM and subtypes thereof, such as G1, G2, G3 and G4. Since the heavy chain isotype is responsible for the various effector functions of an antibody, the choice of the heavy chain constant region will be guided by the desired effector functions, such as complement fixation, or antibody-dependent cellular cytotoxicity (ADCC). An exemplary heavy chain constant region amino acid sequence is shown in SEQ ID NO: 382. The light chain constant region can be derived from either human light chain isotype kappa or lambda. An exemplary light chain constant region amino acid sequence is shown in SEQ ID NO: 383.

The TNFα antibodies of the present invention can bind TNFα with affinity higher than that of infliximab as determined using a Biacore assay. The affinity of TNFα antibodies for TNFα can also be determined experimentally using any other suitable method, *e.g*., methods using KinExA instrumentation, ELISA and competitive binding assays.

The TNFα antibodies of the present invention are capable of blocking or reducing interactions between TNFα and TNF receptors. These TNFα antibodies neutralize TNFα activity in a range of *in vitro* assays, such as cell cytotoxicity, mitogenesis, cytokine induction and induction of adhesion molecules.

The TNFα antibodies of the present invention are useful for treating disorders or symptoms of various immune and autoimmune pathologies as well as inflammatory diseases. TNF related pathologies and diseases include, but are not limited to, the following:
(A) acute and chronic immune and autoimmune pathologies, such as systemic lupus erythematosus, rheumatoid arthritis, thyroidosis, graft versus host disease, scleroderma, diabetes mellitus and Graves' disease;
(B) infections, including, but not limited to, sepsis syndrome, cachexia, circulatory collapse and shock resulting from acute or chronic bacterial infection, acute and chronic parasitic and/or bacterial, viral or fungal infectious diseases, such as AIDS (including sequelae such as cachexia, autoimmune disorders, AIDS dementia complex and infections);
(C) inflammatory diseases, such as chronic inflammatory pathologies and vascular inflammatory pathologies, including chronic inflammatory pathologies such as sarcoidosis, chronic inflammatory bowel disease, ulcerative colitis, and Crohn's pathology and vascular inflammatory pathologies, such as, but not limited to, disseminated intravascular coagulation, atherosclerosis, and Kawasaki's pathology;
(D) neurodegenerative diseases, including, but are not limited to, demyelinating diseases, such as multiple sclerosis and acute transverse myelitis; extrapyramidal and cerebellar disorders' such as lesions of the corticospinal system; disorders of the basal ganglia or cerebellar disorders; hyperkinetic movement disorders such as Huntington's Chorea and senile chorea; drug-induced movement disorders, such as those induced by drugs which block CNS dopamine receptors; hypokinetic movement disorders, such as Parkinson's disease; Progressive supranucleo palsy; Cerebellar and Spinocerebellar Disorders, such as astructural lesions of the cerebellum; spinocerebellar degenerations (spinal ataxia, Friedreich's ataxia, cerebellar cortical degenerations, multiple systems degenerations (Mencel, Dejerine-Thomas, Shi-Drager, and Machado-Joseph); and systemic disorders (Refsum's disease, abetalipoprotemia, ataxia, telangiectasia, and mitochondrial multi.system disorder); demyelinating core disorders, such as multiple sclerosis, acute transverse myelitis; disorders of the motor unit, such as neurogenic muscular atrophies (anterior horn cell degeneration, such as amyotrophic lateral sclerosis, infantile spinal muscular atrophy and juvenile spinal muscular atrophy); Alzheimer's disease; Down's Syndrome in middle age; Diffuse Lewy body disease; Senile Dementia of Lewy body type; Wernicke-Korsakoff syndrome; chronic alcoholism; Creutzfeldt-Jakob disease; subacute sclerosing panencephalitis, Hallerrorden-Spatz disease; and Dementia pugilistica, or any subset thereof;
(E) malignant pathologies involving TNF-secreting tumors or other malignancies involving TNF, such as, but not limited to leukemias (acute, chronic myelocytic, chronic lymphocytic and/or myelodyspastic syndrome); lymphomas (Hodgkin's and non-Hodgkin's lymphomas, such as malignant lymphomas (Burkitt's lymphoma or Mycosis fungoides)); and (F) alcohol-induced hepatitis.

Accordingly, another aspect of the present invention is pharmaceutical compositions as recited in claim 9 comprising at least one TNFα antibody and a pharmaceutically acceptable carrier or diluent known in the art. The carrier or diluent can be a solution, suspension, emulsion, colloid or powder.

A TNFα antibody of the invention is formulated as a pharmaceutical composition in a therapeutically or prophylactically effective amount. The term "effective amount" generally refers to the quantities of antibody necessary for effective therapy, *i.e*., the partial or complete alleviation of the symptom or disorder for which treatment was sought. Included within the definition of effective therapy are prophylactic treatments intended to reduce the likelihood of onset of the above-described symptoms or disorders.

The composition can optionally comprise at least one further compound, protein or composition useful for treating a TNF-related pathology or disease. For example, combination with anti-rheumatic drugs, anti-inflammatory agents, anti-neoplastic agents, radiotherapeutics, immunosuppressives, and cytotoxic drugs to treat various diseases and conditions are contemplated. In this regard, anti-rheumatic drugs can be at least one of auranofin, azathioprine, chloroquine, D-penicillamine, gold sodium thiomalate hydroxychloroquine, Myocrisin and sulfasalzine methotrexate. Anti-inflammatory agents can be at least one of pentasa, mesalazine, asacol, codeine phosphate, benorylate, fenbufen, naprosyn, diclofenac, etodolac and indomethacin, aspirin and ibuprofen.

### Nucleic acids, vectors and cell lines

Another aspect of the present disclosure is isolated nucleic acid molecules comprising, complementary to or having significant identity with a polynucleotide encoding at least one TNFα antibody. Other aspects of the present disclosure include recombinant vectors comprising at least one isolated TNFα antibody encoding nucleic acid molecule and cell lines and organisms that are capable of expressing proteins from the nucleic acid molecules. The nucleic acids, expression vectors and cell lines may generally be used to produce the TNFα antibodies of the invention.

In one embodiment, the nucleic acid compositions of the disclosure comprise polynucleotides encoding polypeptides having amino acid sequences shown in SEQ ID NOs: 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189 or 190. Exemplary nucleic acid sequences comprise polynucleotides shown in SEQ ID NOs: 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377 or 378.

Typically, the nucleic acids of the present dislosure are used in expression vectors for the preparation of the TNFα antibody polypeptides of the invention. Vectors within the scope of the disclosure provide necessary elements for eukaryotic expression, including viral promoter driven vectors, such as CMV promoter driven vectors, *e.g.*, pcDNA3.1, pCEP4 and their derivatives, Baculovirus expression vectors, Drosophila expression vectors and expression vectors that are driven by mammalian gene promoters, such as human immunoglobulin gene promoters. Other examples include prokaryotic expression vectors, such as T7 promoter driven vectors, *e.g*., pET41, lactose promoter driven vectors and arabinose gene promoter driven vectors.

The present invention also relates to cell lines as recited in claim 4 expressing TNFα antibodies. The host cells can be prokaryotic or eukaryotic cells. Exemplary eukaryotic cells are mammalian cells, such as but not limited to, COS-1, COS-7, HEK293, BHK21, CHO, BSC-1, HepG2, 653, SP2/0, NS0, 293, HeLa, myeloma, lymphoma cells, or any derivative thereof. Most preferably, the host cells are HEK293, NS0, SP2/0 or CHO cells. The cell lines of the present invention may stably express at least one TNFα antibody. The cell lines may be generated by stable or transient transfection procedures that are well known in the art.

The present disclosure further provides methods for expressing at least one TNFα antibody comprising culturing the cell lines under conditions wherein the TNFα antibody is expressed in detectable or recoverable amounts. The present disclosure also provides methods for generating at least one TNFα antibody comprising translating the TNFα antibody encoding nucleic acids under conditions *in vitro* or *in situ,* such that the TNFα antibody is expressed in detectable or recoverable amounts. The present invention also encompasses TNFα antibody produced by the above methods.

A TNFα antibody can be recovered and purified by well-known methods including, but not limited to, protein A purification, ammonium sulfate or ethanol precipitation, acid extraction, anion or cation exchange chromatography, phosphocellulose chromatography, hydrophobic interaction chromatography, affinity chromatography, hydroxylatpatite chromatography and lectin chromatography. High performance liquid chroatography (HPLC) can also be employed for purification.

### Uses

The TNFα antibodies are useful as, *inter alia,* research reagents and therapeutic agents. In one aspect, the present invention relates to at least one TNFα antibody for use in a method of decreasing or inhibiting the interactions between TNFα and TNF receptors in a mammal in need thereof. The TNFα antibodies neutralize the biological activities of TNFα and therefore function as antagonists of TNFα. The term "antagonist" is used in the broadest sense and includes a molecule that is capable of, directly or indirectly, partially or fully counteracting, reducing or inhibiting one or more biological activities of TNFα.

The present invention further provides a TNFα antibody for use in reducing the symptoms of, or for use in treating at least one TNF-related condition or disease comprising administering a therapeutically effective amount of at least one TNFα antibody pharmaceutical composition to a patient in need thereof. As described above, such composition comprises an effective amount of at least one TNFα antibody and a pharmaceutically acceptable carrier or diluent. The effective amount for a given therapy, whether curative or preventative, will generally depend upon many different factors, including means of administration, target site and other medicants administered. Thus, treatment doses will need to be titrated to optimize safety and efficacy.

The conditions and diseases suitable for treatment using the antibody of the present invention include but are not limited to, various immune disorders, autoimmune pathologies and inflammatory diseases as listed in detail above. These methods can optionally further comprise coadministration or combination therapies with any standard therapy used to treat the diseases.

The mode of administration can be any suitable route to deliver the pharmaceutically effective amount of TNFα antibody of the present invention to a host. For example, the TNFα antibody can be delivered via parenteral administration, such as subcutaneous, intramuscular, intradermal, intravenous or intranasal administration, or any other means known in the art.

The present invention is further described with reference to the following examples. These examples are merely to illustrate aspects of the present invention and are not intended as limitations of this invention.

### Example 1

### Generation of TNFα Antibodies

Fab libraries were designed based on a combination of infliximab sequence and structure-based criteria. The design goal was molecules with TNFα binding activity at least equal to that of infliximab with high similarity to human immunoglobulin sequences. For example, one criterion of selection for library members was sequence identity or similarity in framework and complementarity determining regions (CDRs) for both heavy and light chains with homologous human counterparts. The human immunoglobulin database at NCBI (ncbi.nlm.nih.gov) and the human germline immunoglobulin database (vbase.mrc-cpe.cam.ac.uk) were employed. Based on these criteria, V_{H} polypeptides were designed with substitutions at one or a combination of 17 sites. These V_{H} combinations were combined with one substituted V_{L} polypeptide having an amino acid sequence shown in SEQ ID NO: 380 (encoding nucleic acid sequence shown in SEQ ID NO: 381). Synthetic Fab DNA libraries were generated according to the method described in U.S. Pat. No. 6,521,427.

The Fab libraries were cloned into pTrcHis2A (Cat. No. V365-20, Invitrogen, Carlsbad, CA). Proteins were expressed in *E. coli* and solid-phase ELISA was used to assess Fab binding to human TNFα. Briefly, 96-well plates were coated overnight at 4°C with 1 µg/ml of rHuTNF-alpha (Biosource, Camarillo, CA) in Carbonate-bicarbonate buffer, pH9.4. After washing in PBS with 0.05% (w/v) Tween-20, the wells were blocked with 1%(w/v) BSA in PBS for 1 hour at room temperature. After washing, plates were incubated with transformed *E. coli* cell lysate diluted in the assay buffer (1% BSA and 0.05% Tween-20 in 10mM PBS) for 2 hours at room temperature. Various concentrations of infliximab (0.5, 1 and 2 ng/ml) were used as positive anti-Human Kappa Light Chain Biotin/anti-penta-His Biotin control. Plates were washed and incubated with ImmunoPure Goat Anti-Human Kappa Chain, Biotin Conjugated (Cat. No. 31780, Pierce Biotechnology, Rockford, IL) and Penta-His Biotin Conjugate (Cat. No. 34440, QIAGEN, Valencia, CA) diluted 1:2000 in the assay buffer for 1 hour at room temperature. Following another washing step, plates were probed for 1 hour at room temperature with 100 µL/well of Streptavidin POD (Cat. No. 1089153, Roche Applied Science, Indianapolis, IN) diluted 1:4000 in the assay buffer. Plates were further washed and then incubated with 3,3',5,5'-tetramentylbenzidine (TMB, Cat. No. 34028, Pierce Biotechnology) for 30 minutes. Substrate development was stopped by addition of 100 µL/well TMB Stop Solution (Cat. No. 50-85-05, KPL, Gaithersburg, MD) and the absorbance was measured at 450nm via an automated plate spectrophotometer.

188 clones with optical density values of at least twice the background value were further characterized. The V_{H} amino acid sequences of these clones are shown in SEQ ID NOs: 3 to 190 respectively. The V_{L} amino acid sequence is shown in SEQ ID NO: 380. Briefly, protein concentration was determined and a titration curve in TNFα ELISA was generated for each clone. A serial dilution curve was generated by eight points titration in duplicate, starting at 50ng/ml. Clones were ranked according to their EC50. The variable regions of selected Fab clones were subcloned into a mammalian construct with infliximab constant region.

### Example 2

### Sequence Analysis of a TNFα Antibody

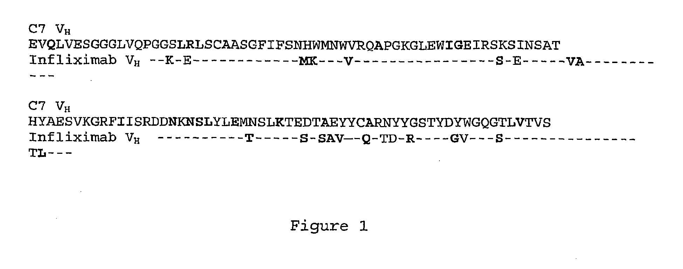

The framework portion of the V_{H} region of one of the novel TNFα antibodies selected as described in Example 1 (C7) (SEQ ID NO: 3) is identical to human germline sequence (VH3 3-72 and JH4). As shown in Fig. 1, when compared to infliximab, 61 out of the 83 amino acids in the framework regions are identical. Of the 23 positions that are different, 14 of them (shown in bold) are conservative amino acid changes.

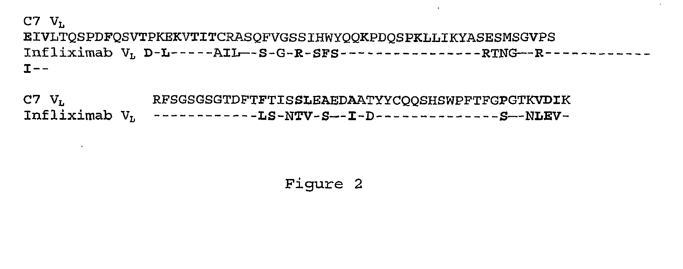

Similarly, the framework portion of C7 V_{L} region is identical to human germline sequence (Vk1 A10 and Jk3). As shown in Fig. 2, when compared to infliximab, 63 out of the 80 amino acids in the framework regions are identical. Of the 17 positions that are different, 13 of them (shown in bold) are conservative amino acid changes.

### Example 3

### Binding of Antibody C7 to TNFα

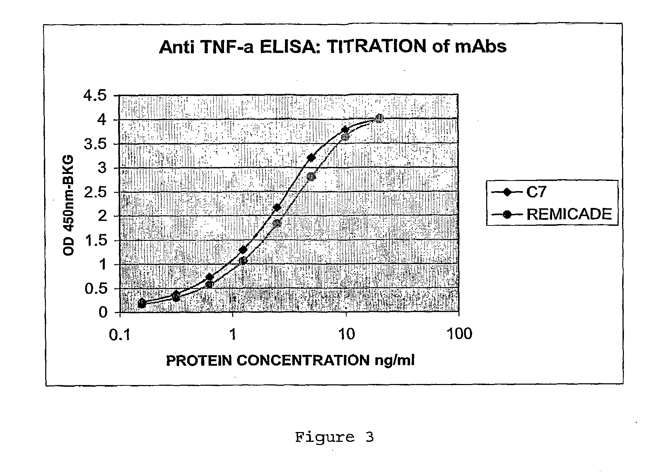

The full-length antibodies were used in a solid-phase ELISA binding assay as described in Example 1 to confirm the TNFα binding. Fig. 3 shows that the ELISA titration curve for C7 is comparable to that of infliximab.

C7 was also used in a Biacore assay to compare the binding constants of C7 and infliximab to TNFα. The results (not shown) indicated that the binding affinity of C7 to TNFα (K_{D} = 70 pM) is more than 2-fold higher than that of infliximab (K_{D} = 170 pM).

The present invention now being fully described, it will be apparent to one of ordinary skill in the art that many changes and modifications can be made thereto without departing from the scope of the appended claims.

## Claims

1. An antibody capable of binding to TNF alpha, wherein the antibody comprises:
(a) a polypeptide comprising a polypeptide having the sequence shown in SEQ ID NO: 2; and
(b) a polypeptide comprising a polypeptide having the sequence shown in SEQ ID NO: 380,
wherein said antibody binds TNF alpha with affinity higher than that of infliximab as determined using a Biacore assay, and
wherein the infliximab V_{H} region has the sequence shown in SEQ ID NO: 1 and the infliximab V_{L} region has the sequence shown in SEQ ID NO: 379.

2. An antibody of claim 1, wherein the polypeptide of claim 1(a) having the sequence shown in SEQ ID NO:2 has the sequence shown in SEQ ID NO: 3.

3. A biologically active fragment of the antibody of claim 1 or claim 2.

4. A cell line expressing the antibody of claim 1 or claim 2 or the biologically active fragment of claim 3.

5. A cell line comprising a vector comprising a polynucleotide encoding the polypeptide recited in claim 1(a) or claim 2 and a vector comprising a polynucleotide encoding the polypeptide recited in claim 1(b).

6. A cell line according to claim 5 wherein the polynucleotide encoding the polypeptide recited claim 1 or claim 2 has the sequence shown in SEQ ID NO: 191 or a complementary sequence.

7. A cell line according to claim 5 or claim 6, wherein the polynucleotide encoding the polypeptide recited in claim 1(b) has the sequence shown in SEQ ID NO: 381 or a complementary sequence.

8. The cell line of any one of claims 4 to 7 wherein the cell line is HEK293, NSO, SP2/0 or CHO cell line.

9. A pharmaceutical composition comprising an effective amount of at least one antibody of claim 1 or claim 2 or of at least one biologically active fragment of claim 3 and a pharmaceutically acceptable carrier or diluent.

## Patentansprüche

1. Antikörper, der an TNF-alpha binden kann, wobei der Antikörper Folgendes umfasst:
(a) ein Polypeptid, umfassend ein Polypeptid mit der in SEQ ID NO:2 gezeigten Sequenz; und
(b) ein Polypeptid, umfassend ein Polypeptid mit der in SEQ ID NO:380 gezeigten Sequenz,
wobei der Antikörper TNF-alpha mit einer höheren Affinität als Infliximab bindet, wie mit Hilfe eines Biacore-Assays bestimmt, und
wobei die V_{H}-Region von Infliximab die in SEQ ID NO:1 gezeigte Sequenz aufweist und die V_{L}-Region von Infliximab die in SEQ ID NO:379 gezeigte Sequenz aufweist.

2. Antikörper nach Anspruch 1, wobei das Polypeptid gemäß Anspruch 1(a) mit der in SEQ ID NO:2 gezeigten Sequenz die in SEQ ID NO:3 gezeigte Sequenz aufweist.

3. Biologisch aktives Fragment des Antikörpers gemäß Anspruch 1 oder 2.

4. Zelllinie, die den Antikörper gemäß Anspruch 1 oder 2 oder das biologisch aktive Fragment gemäß Anspruch 3 exprimiert.

5. Zelllinie, umfassend einen Vektor, umfassend ein Polynucleotid, das für das in Anspruch 1 (a) oder Anspruch 2 angegebene Polypeptid codiert, und einen Vektor, umfassend ein Polynucleotid, das für das in Anspruch 1(b) angegebene Polypeptid codiert.

6. Zelllinie nach Anspruch 5, wobei das Polynucleotid, das für das in Anspruch 1 oder Anspruch 2 angegebene Polypeptid codiert, die in SEQ ID NO:191 gezeigte Sequenz oder eine komplementäre Sequenz aufweist.

7. Zelllinie nach Anspruch 5 oder Anspruch 6, wobei das Polynucleotid, das für das in Anspruch 1(b) angegebene Polypeptid codiert, die in SEQ ID NO:381 gezeigte Sequenz oder eine komplementäre Sequenz aufweist.

8. Zelllinie nach einem der Ansprüche 4 bis 7, wobei es sich bei der Zelllinie um die HEK293-, NSO-, SP2/0- oder CHO-Zelllinie handelt.

9. Pharmazeutische Zusammensetzung, umfassend eine wirksame Menge mindestens eines Antikörpers gemäß Anspruch 1 oder Anspruch 2 oder mindestens eines biologisch aktiven Fragments gemäß Anspruch 3 und einen pharmazeutisch unbedenklichen Träger oder ein pharmazeutisch unbedenkliches Verdünnungsmittel.

## Revendications

1. Anticorps capable de se lier au TNF alpha, l'anticorps comprenant :
(a) un polypeptide comprenant un polypeptide ayant la séquence présentée dans SEQ ID NO:2 ; et
(b) un polypeptide comprenant un polypeptide ayant la séquence présentée dans SEQ ID NO:380,
ledit anticorps se liant au TNF alpha avec une affinité plus grande que celle de l'infliximab, telle que déterminée par utilisation d'un essai Biacore ; et
la région V_{H} de l'infliximab ayant la séquence présentée dans SEQ ID NO:1, et la région V_{L} de l'infliximab ayant la séquence présentée dans SEQ ID NO:379.

2. Anticorps selon la revendication 1, dans lequel le peptide de la revendication 1(a) ayant la séquence présentée dans SEQ ID NO:2 a la séquence présentée dans SEQ ID NO:3.

3. Fragment biologiquement actif de l'anticorps selon la revendication 1 ou 2.

4. Lignée cellulaire exprimant l'anticorps de la revendication 1 ou 2 ou le fragment biologiquement actif de la revendication 3.

5. Lignée cellulaire comprenant un vecteur comprenant un polynucléotide codant pour le polypeptide mentionné dans la revendication 1(a) ou dans la revendication 2 et un vecteur comprenant un polynucléotide codant pour le polypeptide mentionné dans la revendication 1(b).

6. Lignée cellulaire selon la revendication 5, dans laquelle le polynucléotide codant pour le polypeptide mentionné dans la revendication 1 ou 2 a la séquence présentée dans SEQ ID NO:191 ou une séquence complémentaire.

7. Lignée cellulaire selon la revendication 5 ou 6, dans laquelle le polynucléotide codant pour le polypeptide mentionné dans la revendication 1(b) a la séquence présentée dans SEQ ID NO:381 ou une séquence complémentaire.

8. Lignée cellulaire selon l'une quelconque des revendications 4 à 7, la lignée cellulaire étant une lignée cellulaire HEK293, NSO, SP2/0 ou CHO.

9. Composition pharmaceutique comprenant une quantité efficace d'au moins un anticorps de la revendication 1 ou 2 ou au moins un fragment biologiquement actif de la revendication 3 et un support ou un diluant pharmaceutiquement acceptable.
